# FASCICULE DE BREVET EUROPEEN

(11) **EP 1 430 134 B1**
(45) Date de publication et mention de la délivrance du brevet: **04.08.2010**
(21) Numéro de dépôt: 02781380.7
(22) Date de dépôt: 23.09.2002
(51) Int. Cl.: C12N 15/51, C07K 14/08, A61K 39/29, G01N 33/576, C12Q 1/70

(54) **MOLECULES D'ACIDES NUCLEIQUES D'HDV, LEURS FRAGMENTS ET LEURS APPLICATIONS**
NUKLEINSÄUREN VON HDV, DEREN BRUCHSTÜCKEN UND DEREN VERWENDUNGEN
HDV NUCLEIC ACID MOLECULES, FRAGMENTS AND APPLICATIONS THEREOF

(30) Priorité: 24.09.2001 FR 0112285
(43) Date de publication de la demande: 23.06.2004
(60) Demande divisionnaire: 10005982.3
(73) Titulaire: ASSISTANCE PUBLIQUE, HOPITAUX DE PARIS, 75004 Paris (FR)
(72) Inventeur: DENY, Paul, F-78420 Carrieres sur Seine (FR); RADJEF, Nadjia, F-94000 Creteil (FR); HUC-ANAIS, Patricia, F-97150 Saint-Martin (FR)
(74) Mandataire: Vialle-Presles, Marie José
(86) Numéro de dépôt international: PCT/FR2002/003239
(87) Numéro de publication internationale: WO 2003/027291

(56) Documents cités:
- US-A- 5 932 219
- IMAZEKI ET AL.: "Heterogeneity and evolution rates of delta virus RNA sequences" J. VIROL., vol. 64, no. 11, 1990, pages 5594-5599, XP001073432
- DATABASE GENBAN [en ligne] P. DENY: "Hepatitis delta virus genotypes I and II cocirculate in an endemic area of Yakutis, Russia" Database accession no. AJ309874 XP002196404 & V. IVANIUSHINA ET AL.: "Hepatitis delata virus genotypes I and II cocirculate in an endemic area of Yakutia, Russia" J. GEN. VIROL., vol. 82, 2001, pages 2709-2718,
- J.C. WU ET AL.: "Characterisation and phylogenetic analysis of a novel hepatitis D virus strain discovered by restriction fragment length polymorphism" J. GEN VIROL., vol. 79, 1998, pages 1105-1113, XP002196403

## Description

La présente invention est relative à des molécules d'acides nucléiques issues de nouvelles souches ou isolats du virus de l'hépatite D constituant des génotypes différents des génotypes connus I, II et III, à leurs fragments, aux protéines correspondantes, ainsi qu'à leurs applications, en tant que réactifs de diagnostic.

La présente invention est également relative à une méthode de diagnostic sensible du virus de l'hépatite D (ou virus de l'hépatite delta) ainsi qu'à une méthode de suivi épidémiologique des infections à HDV.

Le virus de l'hépatite D (VHD ou HDV, pour *hepatitis D virus*) *ou* delta est un virus satellite de l'hépatite B. Ce virus a une structure particulière : structure chimère associant aux composants spécifiques de l'HDV (ARN viral et protéines HD), une enveloppe comportant les trois glycoprotéines de l'HBV : grande (préS1-préS2-S), moyenne (préS2-S) et petite (S). Le diamètre moyen des particules HDV se situe entre celui des particules HBV matures (particules de DANE : 42 nm) et celui des enveloppes vides de l'HBV (formes sphériques ou filamenteuses : 22 nm) et la densité de flottaison est de 1,24-1,25 g/cm³.

Au sein des virions, l'ARN HDV est circulaire et de polarité négative. Plus petit génome connu des virus infectant les mammifères, ce brin monocaténaire circulaire fermé, a un pourcentage élevé de GC (60%).

L'ARN HDV se réplique indépendamment de l'HBV, dont le rôle se limite à fournir l'enveloppe de l'HDV. Les seules protéines retrouvées (sHD et LHD) sont codées par l'ARN antigénomique qui, dans la cellule infectée, est complet, circulaire et pseudo double-brin, sert d'intermédiaire de réplication et supporte l'édition.

L'ARN HDV appartient à un type de ribozyme spécifique. La réaction d'auto-clivage nécessite l'ARN et un cation divalent (Mg⁺⁺). Le clivage crée une extrémité 2',3' phosphate cyclique et une extrémité 5' hydroxyle.

Les ribozymes delta (génomique et anti-génomique) ont une configuration secondaire voisine en pseudo-noeud. Les séquences impliquées incluent principalement ou exclusivement des séquences situées en 3' du site d'auto-clivage (environ 84 nucléotides).

Au cours du cycle viral, l'ARNm de l'HDV code pour une protéine dont il existe deux formes : une protéine de 194-195 acides aminés (forme 's' pour small) de 24 kilodaltons (kDa) et une protéine de 214 acides aminés (forme 'L' pour Large) de 27 kDa, qui existent en proportions variables. Ces protéines portent l'antigénicité 'delta' et sont détectées dans le foie ou le sérum de patients ou d'animaux infectés (chimpanzé, marmotte). Ces deux protéines virales sHD et LHD s'initient au premier ATG du cadre de lecture ouvert situé à la position 1598 (selon la numérotation de Wang et al., 1986 ou 1987) de l'ARN antigénomique. Au cours de la réplication, une mutation, dépendante d'une enzyme cellulaire 'l'adénosine déaminase dépendante des ARN double brin' apparaît à la position 1012, transformant le codon stop ambre (UAG) en codon tryptophane (UGG), rallongeant en 3' le cadre de lecture de 19 ou 20 codons, et conférant aux deux formes sHD et LHD des propriétés différentes.

L'ARNm est terminé par une queue de poly(A), 15 nucléotides après le signal consensus de polyadénylation, AAUAAA (positions 954-959).

Dans le cycle de réplication, les fonctions des protéines de 24 et 27 Kd s'opposent : sHD active la réplication virale alors que LHD la réprime et joue un rôle dans l'assemblage des particules virales. Ces protéines sont phosphorylées sur les résidus sérine mais non glycosylées (Tableau I). Elles sont constituées de domaines fonctionnels communs et d'un domaine spécifique à la grande protéine LHD.

**Tableau I : Résumé et comparaison des fonctions des deux formes p24 et p27**

| Activités biochimiques et biologiques | p24 (S) | p27 (L) |
|---|---|---|
| Acides aminés | 195 | 214 |
| Transactivation de la réplication | + | - |
| Trans-inhibition de la réplication | - | + |
| Dimérisation et polymérisation | + | + |
| Fixation à l'ARN | + | + |
| Stabilisation de l'ARN | + | + |
| Localisation nucléaire | + | + |
| Assemblage | - | + |
| Phosphorylation | + | +(x6) |
| 19 aa carboxy-terminaux spécifiques | - | + |
| Farnésylation | - | + |

Brièvement, les différents domaines de ces deux protéines sont les suivants :
* domaines communs
   - Le domaine de polymérisation, qui comprend la séquence entre les résidus d'acides aminés 13 et 48 formée d'un agencement de leucine ou isoleucine, organisé en hélice α de type "leucine zipper" (crémaillère à leucine), impliquée dans la polymérisation des protéines, indispensable à (i) la trans activation de la réplication virale par l'Ag-sHD, (ii) l'inhibition de la réplication par l'Ag-LHD et (iii) l'assemblage des complexes sHD-LHD dans les enveloppes de l'HBV.
   - Le signal de localisation nucléaire (SLN) qui implique deux séquences de localisation nucléaire identifiées dans la région 67-88, essentielles pour transloquer l'Ag-sHD synthétisé dans le cytoplasme et, peut-être la ribonucléoprotéine après son entrée dans la cellule, vers le noyau.
   - Le site de fixation à l'ARN qui repose sur deux séquences riches en arginine localisées entre les résidus 97 et 163 qui permettent l'attachement des protéines sHD sur l'ARN génomique ou antigénomique. Cette fixation est indispensable pour que l'Ag-sHD active la réplication.
* domaines spécifiques

Les 19-20 acides aminés situés à l'extrémité COOH de la grande protéine ont un rôle important dans le cycle de l'HDV. En effet, ces acides aminés (aa 195-214), interviennent dans l'assemblage des particules virales (Chang et. al., 1991). Cette activité pourrait être en partie liée à la présence d'une cystéine à la position 211 (Glenn et al., 1992), conservée pour tous les génomes viraux caractérisés à ce jour. Cette cystéine, située 4 acides aminés avant l'extrémité COOH de la protéine, forme une boîte "CXXX" et fixe un groupement farnésyle (Glenn et al., 1992) chaîne de 15 carbones dérivée de l'acide mévalonique, par action d'une farnésyltransférase. Cette maturation post-traductionelle oriente les protéines vers les membranes cellulaires.

La petite et la grande protéine ont été par ailleurs différenciées par des anticorps monoclonaux (clone 9E4) (Hwang et Lai, 1993a). Ces anticorps ne reconnaissaient que sHD (Lai et. al., 1993). La séquence en acides aminés de la petite protéine étant incluse dans la grande, ces résultats suggèrent une conformation différente entre sHD et LHD au sein des 30 acides aminés carboxyterminaux de la petite protéine sHD, suggérant que l'épitope reconnu sur sHD soit masqué dans LHD en conditions non-dénaturantes.

L'HDV se transmet surtout par le biais des aiguilles contaminées et le sang, donc via les porteurs du HDV ou du HBV.

En Amérique du Nord et en Europe de l'Ouest, l'hépatite D se rencontre donc surtout chez les utilisateurs de drogues intraveineuses, les hémophiles et ceux qui ont reçu de multiples transfusions.

L'épidémiologie et les modes de contamination se superposent en partie. On estime globalement à 5 % la proportion de porteurs d'Ag-HBs infectés par l'HDV. Cependant, des disparités de prévalence géographique et épidémiologique sont constatées.

Une haute prévalence de cette maladie, chez les porteurs du virus de l'hépatite B, existe dans certaines régions du globe incluant le bassin amazonien de l'Amérique du Sud, l'Afrique centrale, le sud de l'Italie et dans les pays du Moyen-Orient.

Sur le pourtour méditerranéen, tout particulièrement en Italie du Sud, en Grèce et au Moyen-Orient où la fréquence du portage chronique de l'HBV est intermédiaire (1 % à 5 %), l'infection par l'HDV est élevée. Dans ces régions, la transmission intra-familiale a été suggérée, argumentée par des études phylogénétiques de virus infectant les membres d'une même famille (Niro et al., 1999). En Italie du Sud, la prévalence chez le sujet Ag-HBs positif diminue, passant de 23 % en 1987 à 8 % en 2000 (Gaeta et al., 2000).

En Afrique et en Asie, où le portage chronique de l'HBV est de fréquence élevée (10 % à 20 %), ainsi qu'en Amérique du Sud et dans les îles du Pacifique, où elle est intermédiaire (1 % à 5 %), la répartition de l'HDV est paradoxalement disparate. En Afrique, les études de séroprévalence montrent une distribution très hétérogène des patients ayant des anticorps anti-HD, alors que la prévalence globale d'infection HBV, estimée par la détection de l'Ag-HBs, se stabilise entre 12 et 14 % (Roingeard et al., 1992). Ainsi, des taux variables de 4 % (zone Nord du Sénégal) à 44 % (banlieue de Dakar) font émerger de probables facteurs socioéconomiques impliqués dans la transmission.

Les études de prévalence de l'HDV sont à interpréter prudemment. En effet, dans les populations étudiées, il y a une inclusion préférentielle des patients atteints d'hépatopathies. Chez les patients atteints d'hépatite aiguë ou chronique, la prévalence de l'infection par l'HDV est plus importante que chez les porteurs chroniques asymptomatiques de l'HBV. De plus, l'enquête sérologique d'une infection HDV repose sur la détection de l'Ag-HD et des Ac anti-HD totaux dans le sérum. De ce fait, des infections aiguës bénignes, au cours desquelles une production transitoire isolée d'IgM anti-HD se développerait, ne seraient pas recensées.

L'HDV est responsable d'hépatites aiguës et chroniques. Ces infections sont particulièrement sévères et évoluent plus rapidement vers la cirrhose que les hépatites B seules. C'est l'une des raisons pour lesquelles le diagnostic fiable d'HDV associé à HBV est crucial.

L'infection par un HDV est dépendante de l'HBV. Les isolats HDV de régions géographiques différentes montrent une variabilité génétique. Actuellement trois génotypes sont identifiés et désignés génotype-I, -II et -III.

Le génotype sert à l'épidémiologie des transmissions virales, permet d'étudier la répartition géographique et pourrait être corrélé au pouvoir pathogène.

L'HDV ne se développe que chez les patients également infectés par l'HBV. Cette double infection découle soit d'une co-infection ou d'une surinfection :
- la co-infection est à l'origine d'une hépatite aiguë. Le diagnostic, évoqué au cours d'une cytolyse hépatique, repose sur la détection des marqueurs de l'HDV associée à la présence d'IgM anti-HBc. L'Ag-HBs, généralement présent serait exceptionnellement négatif, justifiant de répéter les prélèvements pour suivre la cinétique d'évolution des marqueurs. Il est classique de constater une inhibition de réplication de l'HBV par l'HDV. Les IgM anti-HBc témoignent de l'infection récente par l'HBV. L'Ag-HD, très précoce, est rarement détecté, compte tenu de sa fugacité. Les anticorps apparaissent 2 à 3 semaines après le début des symptômes : les IgM anti-HD prédominent, mais gardent un titre modéré (<1:1000). Deux pics d'élévation des transaminases, séparés de deux à cinq semaines s'observent dans 10 à 20 % des co-infections, reflétant probablement différentes cinétiques de réplication des virus. La co-infection est donc caractérisée par une sévérité de l'hépatite aiguë souvent supérieure à celle occasionnée par l'HBV isolé. Ainsi, des hépatites fulminantes sont décrites en Amérique du sud et en Afrique Sub-saharienne ou dans certaines populations. L'évolution est généralement marquée par une résolution de l'hépatite après la phase aiguë et à l'image de l'histoire naturelle de l'HBV, seuls 5 % des patients co-infectés évoluent vers la chronicité.
- La surinfection est caractérisée par l'apparition d'une séroconversion HDV chez un patient porteur chronique de l'Ag-HBs. La virémie HDV précède l'apparition d'anticorps anti-HDV en l'absence de détection des IgM anti-HBc. La détection de ces marqueurs peut précéder une élévation des transaminases de plusieurs mois. En phase aiguë, la surinfection se traduit par une hépatite fulminante dans plus de 10 % des cas. De plus, passée la phase aiguë, la surinfection entraîne fréquemment (60 à 70 %) une hépatite chronique active avec évolution rapide vers la cirrhose. A la phase aiguë de la surinfection, la détection de l'Ag-HD est rapidement suivie de l'apparition des anticorps qui persistent à des taux élevés. Contrairement aux modèles classiques d'infection virale, IgG anti-HD et IgM anti-HD sont simultanément détectés dans les hépatites B-delta chroniques.

**Le Tableau II ci-après résume l'évolution des marqueurs B et delta au cours des co-infections et des surinfections**

| Co-infection par l'HDV | Phase aiguë | Évolution | |
|---|---|---|---|
| | | Chronique | Guérison |
| Ag-HBs | + | + | - |
| IgM anti-HBc | + | - | - |
| Ag-HD | +/- | - | - |
| IgM anti-HD | +/- | + | - |
| IgG anti-HD | +/- | + | + |
| ARN HDV | + | + | - |
| Ag-HD intrahépatique | + | + | - |
| | | | |

| Surinfection par l'HDV | | Évolution | |
|---|---|---|---|
| | | Chronique | Guérison |
| Ag-HBs | + | + | - |
| IgM anti-HB c | - | - | - |
| Ag-HD | +/- | - | - |
| IgM anti-HD | + | + | - |
| IgG anti-HD | + | + | + |
| ARN HDV | + | + | - |
| Ag-HD intra-hépatique | + | + | - |

Co-infection et surinfection sont cliniquement indistinctes. Le diagnostic virologique repose habituellement sur les différents marqueurs sériques. Plus rarement, l'Ag-HD peut être détecté sur les coupes anatomopathologique de la biopsie de foie.

Les marqueurs permettent de suivre l'évolution de la maladie vers la guérison ou la chronicité, de décider la mise sous traitement d'un malade et d'en évaluer l'efficacité.

L'HDV ne s'isole pas en culture cellulaire et le diagnostic repose donc essentiellement sur la recherche de l'Ag-HD (ELISA, IF) ou du génome viral (hybridation, PCR, PCR temps réel) pour les techniques directes et sur la détection des anticorps IgM anti-HD et IgG anti-HD pour les méthodes indirectes (ELISA).
- La recherche de l'Ag-HD intrahépatique peut être discutée dans les hépatites fulminantes compte tenu de la cinétique d'apparition des marqueurs sériques. Cet examen a valeur de référence pour étudier la réplication de l'HDV, mais ne peut pas être utilisée en routine.
- L'Ag-HD sérique est recherché dans le sérum en présence d'un agent dissociant qui expose l'Ag-HD, inclus dans l'enveloppe virale portant l'Ag-HBs. La présence d'anticorps (Ac) anti-HD à taux élevé (hépatites chroniques) fixant les antigènes sériques gêne la détection. Les techniques de *Western blot* sont développées à visée de recherche. La présence du virus dans le sang est transitoire et limitée à la phase précoce de l'infection et la possibilité de détecter l'Ag-HD diminue dans les jours suivant l'apparition des symptômes.
- L'immunocapture est utilisée pour détecter les IgM anti-HD et la compétition pour les IgG anti-HD. Les techniques ELISA ont d'abord utilisé comme antigène l'Ag-HD de sérum ou de foie de patients ou d'animaux infectés. Les nouveaux tests reposent sur des Ag-HD recombinants ou des peptides de synthèse.
- Les techniques d'hybridation ou de RT-PCR permettent la détection de l'ARN génomique après extraction des acides nucléiques et dénaturation des structures secondaires. Plusieurs systèmes d'amorces ont été décrits : leur choix est déterminant car la variabilité génétique dans des régions dites conservées peut conduire à des faux négatifs si les amorces choisies ne sont pas appropriées aux souches virales circulantes. Le choix des amorces de PCR doit tenir compte de l'épidémiologie locale des génotypes décrits et il est primordial de bien connaître la répartition de ces génotypes dans le monde.

Toutefois, aussi bien en cas de co-infection qu'en cas de surinfection, l'Ag-HD est en fait difficile à détecter, alors que la virémie précède l'apparition des anticorps.

Dans ce contexte, et notamment en raison de la mise en évidence de nouveaux génotypes, des réactifs nucléiques et protéiques permettant le diagnostic du HDV, quel que soit le génotype, sont nécessaires.

En effet l'étude des séquences nucléotidiques de l'HDV par différentes équipes dans le monde n'a permis de différencier, jusqu'à présent, que trois génotypes distincts :
- le génotype-I, qui est le plus fréquent et le plus largement répandu dans le monde. Depuis sa description initiale (chimpanzé infecté expérimentalement) par Wang (Wang et al., 1986; Wang et al., 1987), plusieurs groupes ont séquencé le génome de l'HDV d'isolats géographiques différents. La première séquence d'un HDV chez l'homme a été décrite en 1987, aux Etats-Unis par S. Makino et al., chez un patient toxicomane (Makino et al., 1987). Le génotype-I est très largement répandu en Italie, aux Etats-Unis, Taiwan, Nauru, France, Liban, Chine (Makino et al., 1987; Chao et al., 1991b ; Imazeki et al., 1991; Lee et al., 1992 ; Niro et al., 1997 ; et Shakil et al., 1997). A l'intérieur du génotype-I un pourcentage de similarité nucléotidique de plus de 85 % est décrit.
- Un isolat japonais (Imazeki et al., 1990 ; Imazeki et al., 1991) est le prototype d'un 2^{ème} sous-groupe d'HDV. Ce génotype-II qui n'avait été décrit dans un premier temps qu'au Japon et à Taiwan (Imazeki et al., précités ; Lee et al., 1996b) semble avoir une répartition géographique beaucoup plus large. En particulier, des séquences de génotype-I et de génotype-II provenant de Yakoutie (Russie) ont également été caractérisées. Enfin, certains auteurs s'appuient sur une diversité intra-génotypique permettant la division en sous-types IIA (Imazeki et al., 1990 ; Imazeki et al., 1991 ; Lee et al., 1996b), IIB (Wu et al., 1998 ; Sakugawa et al., 1999) et IIC. Dans certains pays, l'infection par des virus de génotype-II s'associerait à des hépatites moins sévères que celles causées par les HDV de génotype-I ou -III (Wu et al., 1995b).
- En 1993 un 3^{ème} groupe a été décrit pour des génomes de virus péruviens et colombiens (Casey et al., 1993a). Le génotype-III n'a été décrit qu'en Amérique du Sud, et plus particulièrement dans le Bassin Amazonien associé à des hépatites sévères, voire à des épidémies d'hépatites fulminantes avec stéatose microvésiculaire (Casey et al., 1993a ; Casey et al., 1996b) et à une morbidité et à une mortalité élevées. Dans cette région géographique, on observe que l'HDV de génotype-III est associé de manière privilégiée avec l'HBV de génotype F. D'autres isolats de ce groupe ont récemment été isolés au Vénézuela (Nakano et al., 2001).

En comparant l'ensemble des génomes, deux à quatre régions conservées sont décrites (Chao et al., 1991b). Deux sont constamment retrouvées et sont centrées autour des sites d'autoclivage des génomes et antigénomes impliqués dans les activités auto-catalytiques. Les deux autres régions conservées sont situées dans le cadre de lecture codant pour la protéine HD (Chao et al., 1991b).

Toutefois, les techniques de détection sont tributaires de la variabilité génétique du virus recherché ; les réactifs connus, notamment à partir des séquences spécifiques de génotype-I, -II ou -III, ne permettent pas de détecter les infections à HDV variant et notamment les HDV de génotype différent de ceux précités.

En conséquence, les techniques de détection précisées ci-dessus risquent d'aboutir à des résultats négatifs tant au niveau nucléique qu'en ce qui concerne la réponse en anticorps.

La mise en évidence et la prise en compte de nouveaux variants sont importantes pour mettre au point des réactifs de détection et de diagnostic des hépatites D (sérodiagnostic, PCR, hybridation), suffisamment sensibles et spécifiques, c'est-à-dire ne conduisant pas à des résultats faussement négatifs ou faussement positifs : en effet, une dissociation IgM anti-HD positif / ARN-HDV négatif peut, à l'heure actuelle, être observée dans le contexte d'une hépatopathie sévère.

Dans le cadre de leurs travaux, les Inventeurs ont maintenant caractérisé, de manière surprenante, que la diversité génétique de l'HDV était significativement plus importante qu'antérieurement décrit, ce qui a des conséquences sur la fiabilité du diagnostic.

Ils ont en particulier mis en évidence neuf nouvelles séquences complètes d'HDV (trois originaires de Yakoutie et six originaires d'Afrique), circulant également en Île de France et qui n'appartiennent à aucun des génotypes connus.

L'analyse de ces nouveaux isolats:
- confirme l'existence d'une variabilité beaucoup plus importante de l'HDV que celle qui était décrite jusqu'à présent,
- remet en question la classification des HDV en seulement trois génotypes
- a conduit les Inventeurs à proposer un algorithme de PCR-RFLP, à partir d'une région partielle du génome pour le génotypage HDV et
- a conduit les Inventeurs à mettre au point des réactifs adaptés à un diagnostic fiable des infections à HDV, et ce quel que soit le génotype, alors que précédemment, on observait de nombreux résultats faussement négatifs (existence de nouveaux génotypes).

Les Inventeurs se sont donc donné pour but de pourvoir à des molécules d'acides nucléiques d'HDV, aptes à permettre la détection d'un HDV variant par rapport aux trois génotypes antérieurement décrits.

Sont décrites des molécules d'acide nucléique isolées, **caractérisées en ce qu**'elles sont sélectionnées dans le groupe constitué par :
- le génome d'un HDV, qui moléculairement présente, sur l'ensemble de son génome, une divergence ou distance génétique ≥ 20 % (moins de 80 % de similitude) par rapport aux séquences d'un HDV de génotype I, d'un HDV de génotype II ou d'un HDV de génotype III,
- le génome d'un HDV, qui moléculairement présente une divergence ou distance génétique ≥ 25 % (moins de 75 % de similitude) sur une région dénommée R0, délimitée par les positions 889 à 1289 du génome HDV, par rapport aux séquences R0 correspondantes d'un HDV de génotype I, d'un HDV de génotype II ou d'un HDV de génotype III,
- les génomes complets des isolats ou variants d'HDV dénommés dFr45, dFr47, dFr73, dFr910, dFr48 et dFr644 qui présentent respectivement les séquences SEQ ID NO: 1, 6, 11, 16, 21 et 26, et
- le génome d'un HDV qui présente une divergence ou distance génétique ≤ 15 % avec au moins l'une des séquences SEQ ID NO: 1, 6, 11, 16, 21 et 26.

Selon un mode de réalisation avantageux desdites molécules, la région R0 est de préférence obtenue par amplification de l'ARN HDV avec les amorces 900S (SEQ ID NO:33) et 1280AS (SEQ ID NO:34).

Au sens de la présente invention, on entend par molécule d'acide nucléique, une molécule d'ADNc ou d'ARN présentant l'une des séquences génomiques d'HDV telles que définies ci-dessus et leurs séquences complémentaires sens et anti-sens.

Sont également décrites des molécules d'acide nucléique qui comprennent au moins l'un des fragments des séquences d'un HDV variant telles que définies ci-dessus, sélectionnés dans le groupe constitué par :
a) les fragments R0 des HDV variants isolés suivants : dFr45, dFr47, dFr48, dFr69, dFr73, dFr644, dFr910, dFr1843, dFr1953, dFr2020 et dFr2066, qui présentent respectivement les séquences suivantes : SEQ ID NO:48 à SEQ ID NO:58,
b) le fragment R1 qui s'étend des positions 307 à 1289 du génome d'HDV,
c) le fragment R2 qui s'étend des positions 889 à 328 du génome d'HDV,
d) le fragment R3 qui s'étend des positions 1486 à 452 du génome d'HDV,
e) le fragment R'1 qui s'étend des positions 305 à 1161 du génome d'HDV,
f) le fragment R'2 qui s'étend des positions 984 à 331 du génome d'HDV,
g) le fragment R644 qui s'étend des positions 889 à 446 du génome d'HDV,
h) le fragment G910 qui s'étend des positions 1206 à 929 du génome d'HDV,
i) le fragment p910 qui s'étend des positions 553 à 1550 du génome d'HDV,
j) les ADNc codant pour la protéine sHD, de séquences SEQ ID NO:4, 9, 14, 19, 24 et 29,
k) les ADNc codant pour la protéine LHD, de séquences SEQ ID NO:2, 7, 12, 17, 22 et 27 et
l) les amorces de séquence SEQ ID NO:33 à SEQ ID NO:47.

Au sens de la présente invention, les positions des fragments dans le génome d'HDV sont indiquées sur le génome circulaire en orientation génomique, selon la numérotation de Wang et al., 1986 ou 1987.

Sont également décrits des fragments nucléotidiques complémentaires des précédents ainsi que des fragments modifiés, par rapport aux précédents, par enlèvement, ou addition de nucléotides dans une proportion d'environ 15 %, par rapport à la longueur des fragments ci-dessus et/ou modifiés au niveau de la nature des nucléotides dès lors que les fragments nucléotidiques modifiés conservent une capacité d'hybridation avec les séquences d'ARN génomiques ou antigénomiques des isolats tels que définis ci-dessus.

En effet, ces différentes souches virales, chez un même patient, à un moment donné, montre une population hétérogène de molécules d'ARN HDV ; en outre, au cours d'une infection chronique, en plus des hétérogénéités observées au niveau du site d'édition (position 1012), des mutations sont susceptibles d'apparaître. Les séquences virales semblent évoluer au sein de populations virales avec un taux de substitution variable de 3.10-2 à 3.10⁻³ par nucléotide et par an.

Certains de ces fragments sont spécifiques et sont utilisés comme sonde ou comme amorces ; ils s'hybrident spécifiquement à une souche d'HDV variante telle que définie ci-dessus ou à une souche apparentée ; on entend par HDV apparenté à un variant tel que défini ci-dessus, un HDV présentant une divergence génétique ≤ à 15%.

De tels fragments sont utilisés pour la détection et le suivi épidémiologique des infections à HDV. Par exemple, le fragment R0 est utilisé pour la détection (RT-PCR) et le génotypage (PCR-RFLP) d'HDV. Les autres fragments qui couvrent la totalité du génome d'HDV sont utilisés pour la caractérisation moléculaire des HDV variants ; l'analyse phylogénétique de la séquence complète du génome ou de fragments de celui-ci correspondant en particulier à R0 ou à R2 permettent de rattacher les profils observés en PCR-RFLP à un génotype donné ou de caractériser de nouveaux génotypes.

En conséquence, est également décrite une méthode de détection d'un HDV variant selon l'invention, par hybridation et/ou amplification, réalisée à partir d'un échantillon biologique, laquelle méthode est **caractérisée en ce qu**'elle comprend :
(1) une étape d'extraction de l'acide nucléique à détecter, appartenant au génome du virus éventuellement présent dans l'échantillon biologique,
(2) la réalisation d'au moins une amplification génique à l'aide d'une paire d'amorces sélectionnée dans le groupe constitué par les amorces aptes à amplifier l'une des régions suivantes de l'ARN génomique d'HDV: R0, R1, R2, R3, R644, G910, p910, R'1 et R'2 et
(3) l'analyse du produit amplifié par comparaison avec l'une des molécules de séquences SEQ ID NO:1, 6, 11, 16, 21 et 26, correspondant respectivement aux génomes complets des isolats ou variants dénommés dFr45, dFr47, dFr73, dFr910, dFr48 et dFr644.

De manière avantageuse, l'étape (3) d'analyse peut être mise en oeuvre par restriction, séquençage ou hybridation ; dans ce dernier cas, la sonde utilisée (notamment dans des puces à ADN) sera avantageusement un fragment de 15 à 20 nucléotides, spécifique desdits fragments amplifiés.

Selon un mode de mise en oeuvre avantageux de ladite méthode, les amorces spécifiques pour l'amplification des régions R0, R1, R2, R3, R644, G910, p910, R'1 et R'2, mises en oeuvre à l'étape (2) sont sélectionnées dans le groupe constitué par :
- les amorces 900S (SEQ ID NO:33) et 1280AS (SEQ ID NO:34), pour l'amplification de R0 (environ 400 pb),
- les amorces 320S (SEQ ID NO:39) et 1280AS (SEQ ID NO:34), pour l'amplification du fragment R1 (environ 960 pb),
- les amorces 900S (SEQ ID NO:33) et 320AS (SEQ ID NO:45), pour l'amplification de R2 (environ 1100 pb), qui contient le gène sHD correspondant aux positions 1598-950,
- les amorces 1480S (SEQ ID NO:46 ) et 440AS (SEQ ID NO:47),, pour l'amplification de R3 (environ 650 pb),
- les amorces 900S (SEQ ID NO:33) et 420AS (SEQ ID NO:40), pour l'amplification de la région R644 (environ 1250 pb) de l'isolat dFr644,
- les amorces 318S (SEQ ID NO:35) et 1150AS (SEQ ID NO:36), pour l'amplification de R'1 (environ 850 pb),
- les amorces 960S (SEQ ID NO:37) et 345AS (SEQ ID NO:38), pour l'amplification de R'2 (environ 1050 pb),
- les amorces R910S (SEQ ID NO:41) et R910As (SEQ ID NO:42) pour l'amplification de la région G910 (environ 1400 pb) de l'isolat dFr910,
- les amorces S910R (SEQ ID NO:43) et As1910R (SEQ ID NO:44) pour l'amplification de la région p910 (environ 650 pb) de l'isolat dFr910.

Est également décrite une méthode de détection et de génotypage d'HDV à partir d'un échantillon biologique, laquelle méthode est **caractérisée en ce qu**'elle comprend :
(a) une étape d'extraction de l'acide nucléique appartenant au génome du virus HDV,
(b) une étape d'amplification de la région R0 délimitée par les positions 889 à 1289 du génome HDV,
(c) un premier traitement des molécules d'acides nucléiques amplifiées par les enzymes de restriction *Sma*I et *Xho*I, pour produire un premier ensemble de fragments de restriction, et
(d) un deuxième traitement des molécules d'acides nucléiques par l'enzyme de restriction *Sac*II, pour produire un deuxième ensemble de fragments de restriction
(e) l'analyse combinée des deux ensembles de fragments de restriction produits par RFLP (*Restriction Fragment Length Polymorphism*), de manière à détecter la présence et/ou déterminer le type d'HDV présent dans ledit échantillon biologique.

Selon un mode de mise en oeuvre avantageux de ladite méthode, l'étape (b) d'amplification est avantageusement mise en oeuvre avec les amorces 900S (SEQ ID NO: 33) et 1280AS (SEQ ID NO:34).

La méthode selon l'invention permet de définir de nouveaux profils de restriction et de classer les HDV en sept génotypes distincts.

Selon un autre mode de mise en oeuvre avantageux de ladite méthode, elle comprend en outre :
(f) l'amplification des molécules d'acide nucléique dudit échantillon par RT-PCR avec les amorces 900S (SEQ ID NO:33) et 320AS (SEQ ID NO: 45) de manière à amplifier la région R2 et
(g) le séquençage direct de la région R2 amplifiée et la comparaison avec l'une des molécules d'ARN de séquences SEQ ID NO: 1, 6, 11, 16, 21 et 26, correspondant respectivement aux génomes complets des isolats ou variants dénommés respectivement dFr45, dFr47, dFr73, dFr910, dFr48 et dFr644, par exemple par analyse phylogénétique.

Lorsque des profils inhabituels sont observés, cette étape supplémentaire permet de caractériser de nouveaux génotypes. En effet, ces analyses complémentaires de la PCR-RFLP permettent de rattacher le nouveau profil observé à un génotype donné, ou de caractériser un nouveau génotype, par analyse Phylogénétique.

Est également décrit un vecteur recombinant, notamment un plasmide comprenant un insert constitué par une molécule d'acide nucléique telle que définie ci-dessus.

Est également décrite une cellule transformée par une molécule d'acide nucléique telle que définie ci-dessus.

Sont également décrits des produits de traduction codés par l'une des molécules d'ARN de séquences SEQ ID NO:1, 6, 11, 16, 21 et 26 correspondant respectivement aux ARN génomiques complets des isolats ou variants dénommés respectivement dFr45, dFr47, dFr73, dFr910, dFr48 et dFr644 ou par leurs séquences complémentaires sens ou anti-sens.

Sont également décrites les protéines codées par le génome d'un HDV variant tel que défini ci-dessus.

Selon un mode de réalisation avantageux de l'invention, ladite protéine est sélectionnée dans le groupe constitué par :
- la proteine LHD de dFr45, dFr47, dFr73, dFr910, dFr48 et dFr644 qui présente respectivement les séquences SEQ ID NO: 3, 8, 13, 18, 23 et 28, et
- la proteine sHD de dFr45, dFr47, dFr73, dFr910, dFr48 et dFr644 qui présente respectivement les séquences SEQ ID NO: 5, 10, 15, 20, 25 et 30.

La présente invention a également pour objet un peptide, **caractérisé en ce qu**'il est constitué par un fragment d'une protéine telle que définie ci-dessus, sélectionné dans le groupe constitué par:
- le peptide A constitué par les 19 acides aminés de l'extrémité carboxy-terminale des séquences SEQ ID NO: 3, 8, 13, 18, 23 et 28,
- le peptide B de séquence (code à une lettre) RLPLLECTPQ (SEQ ID NO:59) constitué par les 10 acides aminés de l'extréminé carboxy-terminale des séquences SEQ ID NO: 3, 8, 13, 18, 23 et 28, et
- le peptide C constitué par les 9 acides aminés précédents la séquence SEQ ID NO:59 (SEQ ID NO:60 à SEQ ID NO:65).

De tels peptides sont utiles pour le diagnostic indirect (sérologie) d'une infection par HDV, notamment par une méthode immunoenzymatique (ELISA) :
- le peptide B, qui est conservé, permet la détection de l'ensemble des variants selon l'invention et du génotype II d'HDV, et
- le peptide C est spécifique des différents variants d'HDV selon l'invention.

Est décrite l'utilisation d'une molécule d'acide nucléique telle que définie ci-dessus ou d'une protéine telle que définie ci-dessus pour la préparation d'un kit de détection et de génotypage d'un HDV.

Outre les dispositions qui précèdent, sont encore décrites d'autres dispositions, qui ressortiront de la description qui va suivre, qui se réfère à des exemples de mise en oeuvre de la présente invention ainsi qu'aux dessins annexés dans lesquels :

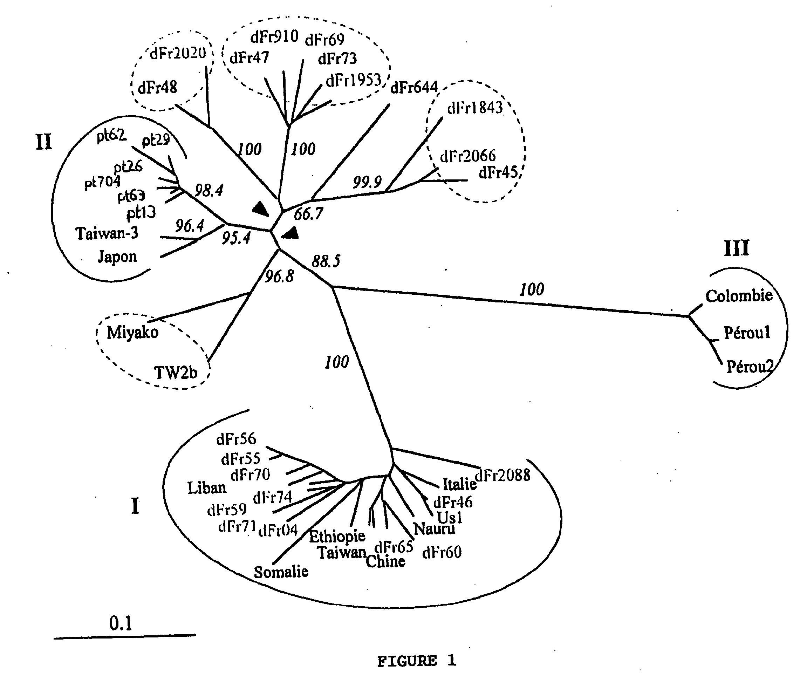

- la figure 1 représente l'arbre phylogénétique de la région R0 obtenu par la méthode du plus proche voisin "*neighbor-joining*". Les nombres en italique indiquent les valeurs de bootstraping (BV) sur 10⁴ rééchantillonnages et le signe π indique les BV < 50 %. L'échelle représente le nombre de substitution de nucléotides par site.

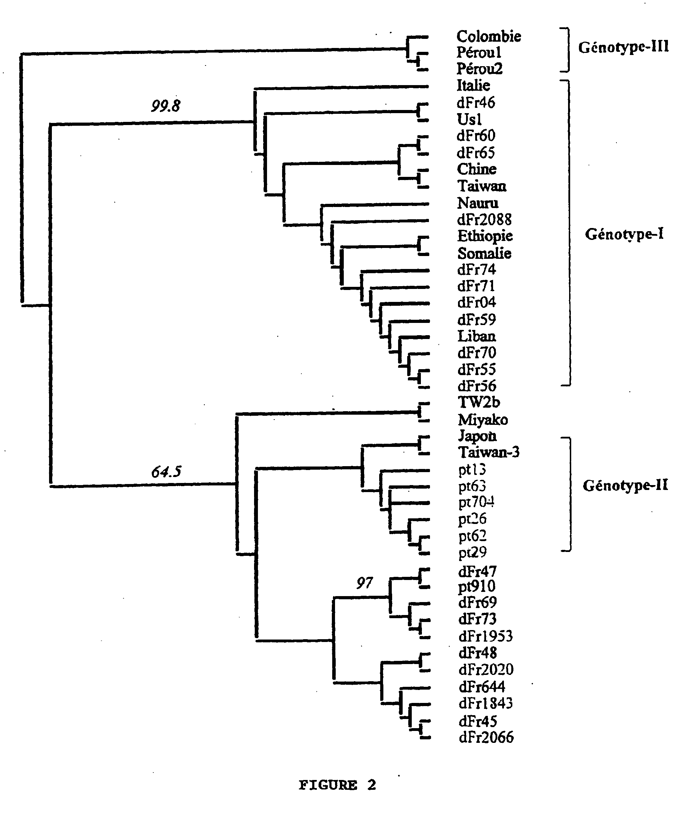

- la figure 2 représente l'arbre phylogénétique des régions R0 d'HDV obtenu par la méthode de maximum de parcimonie. Les isolats Pérou1, Pérou2 et Colombie sont choisis comme "outgroup". Les chiffres en italique indiquent les valeurs de bootstraping (BV) sur 10⁴ rééchantillonnages,

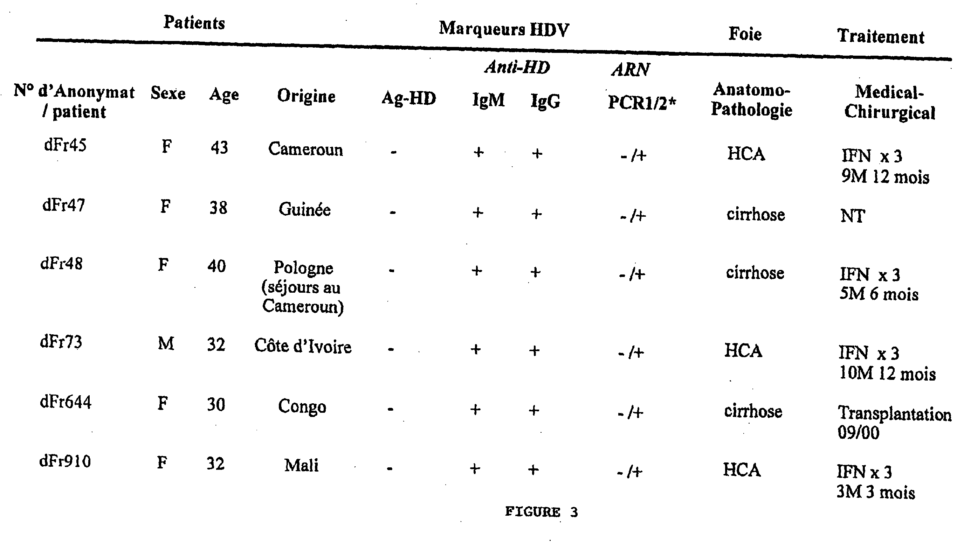

- la figure 3 illustre les données cliniques de chacun des six patients infectés par les isolats d'HDV d'origine africaine. * indique respectivement les PCR 6S/6As et PCR R0,

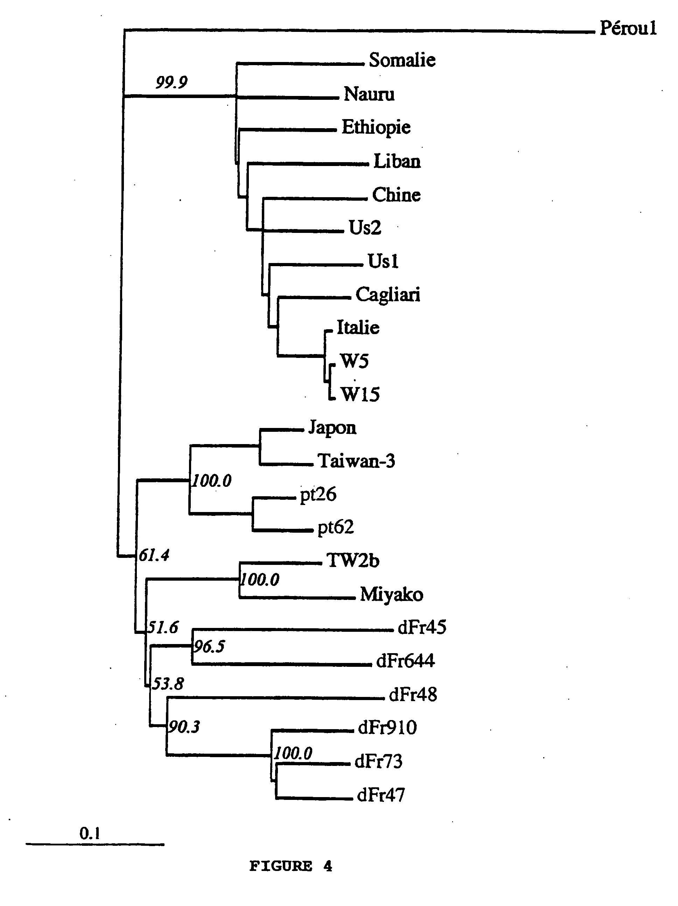

- la figure 4 représente l'arbre phylogénétique des génomes complets d'HDV, obtenu par la méthode du plus proche voisin "*neighbor-joining*". Les nombres en italique, au niveau de chaque noeud, indiquent les valeurs de bootstraping (BV) sur 10⁴ rééchantillonnages. L'échelle représente le nombre de substitution de nucléotides par site.

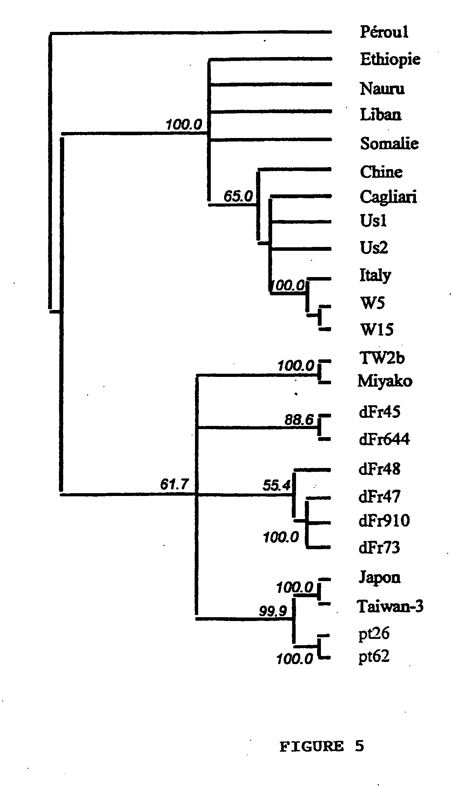

- la figure 5 représente l'arbre phylogénétique des génomes complets d'HDV, obtenu par la méthode de maximum de parcimonie. Les nombres en italique, au niveau de chaque noeud, indiquent les valeurs de bootstraping (BV) sur 10⁴ rééchantillonnages,

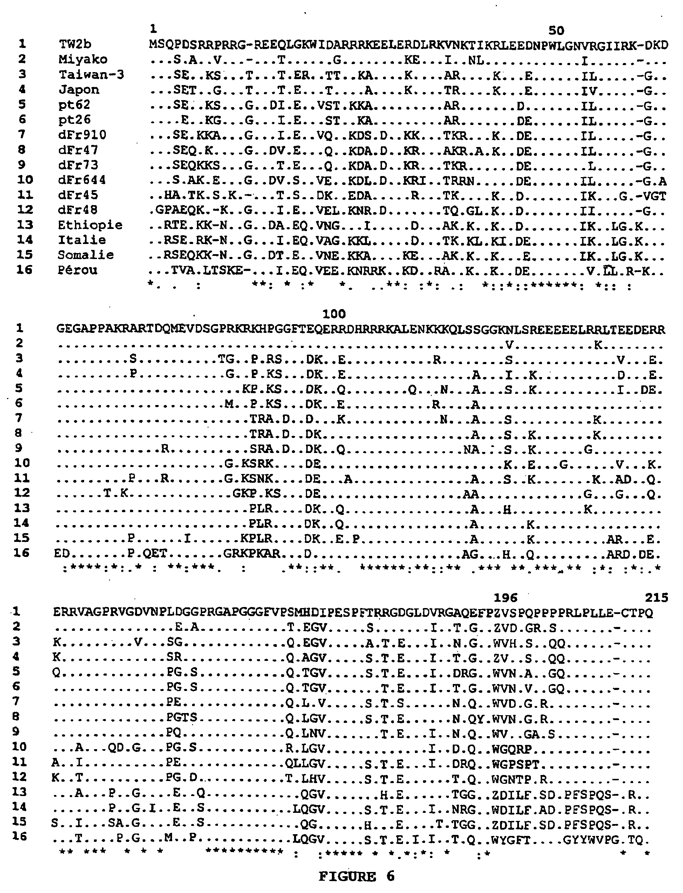

- la figure 6 représente l'alignement des séquences en acides aminés des protéines delta des six isolats d'origine africaine (lignes 7, 8, 9, 10, 11 et 12) avec les séquences connues de génotype I (lignes 13, 14 et 15), génotype II (lignes 3, 4, 5 et 6), génotype III (ligne 16) et TW2b/Miyako (lignes 1 et 2), à l'aide du logiciel Clustal (version 1.8). L'acide aminé en position 196 de la protéine p27 correspond au codon de terminaison de la protéine p24 (Z) ou au codon tryptophane (W) qui conduit à la synthèse de la protéine p27 qui s'étend des acides aminés 1 à 215.

Il doit être bien entendu, toutefois, que ces exemples sont donnés uniquement à titre d'illustration de l'objet de l'invention, dont ils ne constituent en aucune manière une limitation.

### EXEMPLE 1: MATERIELS ET METHODES

### 1- Patients et échantillons.

22 sérums provenant de sujets suivis dans les différents centres hospitaliers de la région parisienne ont été analysés. Les patients étaient porteurs chroniques de l'Ag-HBs. Le diagnostic de l'infection delta a été effectué par la recherche des marqueurs sérologiques (Ag-HD, anti-AgHD de type IgM et IgG) et la détection du génome viral de l'HDV par RT-PCR. L'Ag-HD n'a été détecté dans aucun des sérums analysés. Les anticorps anti-AgHD de type IgM témoignant de la chronicité de l'infection delta, et de type IgG ont été retrouvés chez tous les patients. La totalité du génome de l'HDV a été caractérisée sur six patients. Tous les sérums étaient conservés à -80°C jusqu'à l'extraction de l'ARN viral.

### 2- Extraction de l'ARN HDV.

Pour extraire l'ARN HDV, un volume de 250 µl de sérum est ajouté à 75 µl de TRIzol LS Reagent (Gibco BRL, Life Technologies). Après 30 secondes d'homogénéisation, le mélange est incubé pendant 5 min à température ambiante. L'extraction des lipides s'effectue en ajoutant 200 µl de chloroforme refroidi à +4°C. Après une nouvelle homogénéisation au vortex, les tubes sont incubés puis centrifugés à 14 000 rotations par minute (rpm) pendant 10 min, à +4°C. La phase aqueuse est transférée dans des tubes d'extraction et les ARNs sont précipités par 500 µl d'isopropanol froid, en présence d'1 µg de glycogène. Après 15 min d'homogénéisation, les échantillons sont centrifugés à 14 000 rpm pendant 10 min, à +4°C. Après rinçage à l'éthanol à 70 %, les tubes sont à nouveau centrifugés à 14 000 rpm pendant 10 min, à + 4°C. Les culots sont séchés sous une hotte à température ambiante, puis repris dans 100 µl d'eau stérile comprenant un inhibiteur de ribonucléases (RNasin, Promega). A cette étape, des précautions sont prises pour éviter d'éventuelles contaminations des tampons et des échantillons par les ribonucléases.

### 3- Synthèse d'un ADN complémentaire (ADNc).

Cette étape consiste à synthétiser un brin d'ADN complémentaire à l'ARN HDV par transcription inverse.

Pour éliminer la structure secondaire de l'ARN HDV, 5 µl d'ARN extrait précédemment sont ajoutés à un mélange réactionnel contenant 5 µl (ou 0,5 pmoles) de désoxynucléotides triphosphates (dNTP) et 1 µl (0,4 pmoles) d'hexanucléotides aléatoires. Les ARNs sont ensuite dénaturés pendant 3 min, à 95°C. Pour fixer les ARNs dénaturés, les tubes sont congelés immédiatement dans l'éthanol refroidi à -20°C. Dix µl d'un mélange réactionnel, contenant 2,5 µl de dithiothréitol (DTT), 100 unités (U) de transcriptase réverse Superscript II, (Gibco BRL, Life Technologies) et son tampon réactionnel, ainsi que 20 U d'inhibiteur de ribonucléases (RNasin, Promega) sont ajoutés à l'ARN dénaturé. La réaction de transcription inverse est effectuée à 42°C pendant 45 min, puis arrêtée par incubation à 94°C pendant 5 min. Les ADNc sont ensuite conservés à -80°C.

### 4- Amplification génique.

L'amplification des ADNc est effectuée, de manière exponentielle, par PCR (*Polymerase Chain Reaction*). Deux types de polymérases ont été utilisés : la polymérase AmpliTaq Gold (*Thermophilus aquaticus*) (PE Applied Biosystems) et la polymérase Pwo (*Pyrococcus woesi*) ou Expand ^{™} High Fidelity PCR System (Roche).

L'amplification a été effectuée à partir de 5 µl d'ADNc qui sont ajoutés à un mélange réactionnel de PCR contenant : 0,25 pmoles/µl d'amorce sens et antisens (Tableau III), 200 µmoles de chaque dNTP, 1,5 mM de MgCl₂, 1 U d'AmpliTaq Gold ou 2,6 U de polymérase Expand^{™} en présence des tampons PCR correspondants. La réaction de PCR a été réalisée dans un thermocycleur (PCR Sprint, Hybaid, Coger), selon le protocole suivant : dénaturation des hybrides ADNc - ARN à 94°C pendant 9 min, suivie de 40 cycles successifs, chacun comprenant une dénaturation des ADNs à 94°C pendant 45 sec, une hybridation des amorces (900S/1280As ou 6S/6As) à 58°C pendant 30 sec, synthèse du brin complémentaire grâce à la polymérase par élongation à 72°C pendant 45 sec. Enfin, une élongation finale à 72°C pendant 4 min 30 sec à 72°C.

**Tableau III : Séquences des amorces utilisées pour les réactions de PCR et leur position sur le génome HDV.**

| **Amorces** | **Séquence 5' -> 3 '** | **numéro d'identification** | **positions*** |
|---|---|---|---|
| **6S** | gaggaaagaaggacgcgagacgcaa | SEQ ID NO:31 | 904-929 |
| **6As** | accccctcgaaggtggatcga | SEQ ID NO: 32 | 1141-1121 |
| **900S** | catgccgacccgaagaggaaag | SEQ ID NO: 33 | 889-911 |
| **1280As** | gaaggaaggccctcgagaacaaga | SEQ ID NO:34 | 1289-1265 |
| **318S** | ctccagaggaccccttcagcgaac | SEQ ID NO:35 | 305-328 |
| **1150As** | cccgcgggttggggatgtgaaccc | SEQ ID NO:36 | 1161-1138 |
| **960S** | gtacactcgaggagtggaaggcg | SEQ ID NO:37 | 962-984 |
| **345As** | tctgttcgctgaaggggtcct | SEQ ID NO:38 | 331-311 |
| **320S** | ccagaggaccccttcagcgaac | SEQ ID NO 39 | 307-328 |
| **420As** | aacaccctcctgctagcccc | SEQ ID NO: 40 | 446-427 |
| **R910S** | ccggagttcctcttcctcctcc | SEQ ID NO:41 | 1206-1227 |
| **R910AS** | gttcgcgtcegagtccttettte | SEQ ID NO:42 | 929-907 |
| **S1910R** | gagctttcttcgattcggac | SEQ ID NO:43 | 1531-1550 |
| **AS1910R** | gactggtcccctcatgttcc | SEQ ID NO:44 | 572-553 |

| | | | |
|---|---|---|---|
| * Selon la numérotation de Wang et al. (Nature, 1986, 323, 508-514 ; Nature, 1987, 328, 456) | | | |

### 4.1 - Stratégie d'amplification du génome viral HDV

Le couple d'amorces 6S et 6As permet d'amplifier un fragment d'ADN correspondant à l'extrémité carboxy-terminale du gène codant pour l'antigène delta.

La région R0 comprenant l'extrémité carboxy-terminale du gène codant pour l'Ag-HD et une partie de la région non codante a été amplifiée pour tous les échantillons à l'aide des amorces 900S (SEQ ID NO:33) et 1280As (SEQ ID NO:34). L'amorce 900S utilisée a été délétée de 7 nucléotides à l'extrémité 5', par rapport à celle utilisée par Casey et al., 1993a, précité, pour la classification des génotypes d'HDV.

La sélection de ces amorces permet, de manière surprenante, l'amplification d'un fragment apte à permettre de distinguer les génotypes connus (I, II et III) de nouveaux génotypes.

Les séquences complètes du génome viral HDV de quatre échantillons (*dFr45, dFr47, dFr48 et dFr73*) ont été obtenues par amplification de deux régions chevauchantes R'1 (850 bases) et R'2 (1050 bases), respectivement, à l'aide des couples d'amorces 318S/1150AS et 960S/345AS.

Pour l'échantillon *dFr644*, la variabilité observée dans la région correspondant aux amorces précédemment décrites a conduit à amplifier la région 644 (R644) à l'aide d'un couple d'amorces spécifique : 900S et 480AS.

Pour l'échantillon *dFr910*, la séquence nucléotidique R0, a permis de définir des amorces nouvelles et spécifiques de l'échantillon pour amplifier le génome complet. Deux couples d'amorces ont été choisis : les amorces R910S et R910AS, amplifiant un fragment de 1400 bases correspondant à la région G910. Un autre couple d'amorces S 1910R et AS 1910R amplifiant un fragment de 650 bases (région p910) a été indispensable pour couvrir la totalité du génome.

L'amplification des différentes régions (R1, R2, R3, R644, R'1, R'2, G910 et p910) est effectuée comme décrite précédemment. Les températures d'hybridation et d'élongation ainsi que le temps d'élongation utilisés pour chacune des PCR, sont indiqués dans le Tableau IV.

**Tableau IV :Amplification des différents fragments du génome**

| Régions amplifiées | Taille des fragments (bases) | Température d'hybridation (°C) | Température d'élongation (°C) | Temps d'élongation |
|---|---|---|---|---|
| RI | 960 | 62 | 72 | 1 min 15 s |
| R2 | 1100 | 56 | 72 | 1 min 30 s |
| R3 | 650 | 50 | 72 | 1 min |
| R644 | 1250 | 58 | 72 | 40 s |
| G910 | 1400 | 58 | 72 | 1 min 40 s |
| p910 | 650 | 58 | 72 | 1 min |
| R'I | 850 | 63 | 72 | 1 min |
| R'2 | 1050 | 60 | 72 | 1 min 20 s |

### 5-Analyse des produits d'amplification.

Un volume de 8 µl du produit PCR est déposé en présence de 2 µl de solution de dépôt dans un gel à 1,3 % d'agarose préparé dans du tampon Tris-Borate/EDTA 0,5X et contenant 0,5 µg/ml de bromure d'éthidium (BET). L'électrophorèse est effectuée en tampon TBE 0,5X. La migration est effectuée, en présence d'un marqueur de taille (Raoul^{™}, Appligène). Le fragment amplifié est visualisée sous rayons ultraviolets à 312 nm et photographié.

### 6- Clonage et séquençage des génomes HDV.

Avant l'étape de clonage et de séquençage, les produits d'amplification ont été purifiés afin d'éliminer toutes traces de sels et d'enzymes.

### 6.1- Élongation par la Taq-polymérase standard.

Cette étape est effectuée dans le cas où l'amplification du produit a été réalisée avec la polymérase Pwo. Elle permet d'ajouter les résidus de désoxyadénosines (A) aux extrémités 3' des produits de PCR, du fait que la polymérase Pwo, dotée d'une activité exonucléasique 5' ----> 3', diminue l'incorporation des désoxyadénosines.

Un volume de 10 µl d'ADN purifié est ajouté à un mélange réactionnel de 70 µl contenant : 0,2 mM de dNTP, 1,5 mM de MgCl₂, du tampon 1X et 2,5 U de Taq Polymérase (Perkin Elmer). L'élongation est effectuée à 72°C, pendant 30 minutes. Les produits de PCR subissent alors une nouvelle purification au phénol-chloroforme et une précipitation à l'éthanol, puis sont repris dans 10 µl d'eau stérile.

### 6.2- Clonage dans le vecteur pCRII-TA-cloning (Invitrogen).

Le clonage est utilisé pour confirmer la séquence nucléotidique de l'ADN amplifié. Il est réalisé en utilisant le vecteur pCRII (Invitrogen).

Le vecteur pCRII se présente sous forme linéaire. Il possède des résidus de désoxythymidine (T) permettant le clonage du produit amplifié grâce aux résidus complémentaires de désoxyadenosine (A) ajoutés par la Taq polymérase. Il possède également les séquences promoteurs Sp6 et T7, deux sites de restriction *EcoR*I qui encadrent le site d'insertion du produit de PCR et les gènes de résistance à l'ampicilline et à la kanamycine. Une fraction du gène *lacZ*α, codant pour la β-galactosidase, facilite la sélection des recombinants par la couleur des colonies. En effet, les plasmides ayant intégré l'insert n'expriment pas le gène *lacZ*α. Les colonies bactériennes sont alors blanches en présence de substrat de β-galactosidase (X-Gal ou 5-bromo 4-chloro 3-indoyl β-galactoside, Roche) et d'un inducteur du gène (IPTG ou isopropyl-thio β-D-galactoside, Roche). Ainsi, les bactéries recombinantes sont sélectionnées grâce à leur résistance à l'ampicilline et à un crible bleu-blanc.

Le rapport moléculaire choisi insert/vecteur est de 3/1 et le volume de produit PCR utilisé est variable, selon la quantité d'ADN estimée par électrophorèse en gel d'agarose comme décrit précédemment. Le mélange réactionnel de 10 µl contient 50 ng de vecteur pCRII, la quantité d'insert correspondante, 4 U de T4 DNA Ligase, et le tampon Ligase 1X. La réaction de ligature est conduite pendant 18 heures à 14°C. Les tubes sont ensuite conservés à +4°C.

Les bactéries *Escherichia coli* TOP10F' (Invitrogen), rendues compétentes par traitement au chlorure de calcium, sont conservées à -80°C, prêtes à l'utilisation. Un volume de 50 µl de bactéries compétentes est mis au contact de 3 µl de la solution de ligature pendant 30 minutes, dans de la glace. Un choc thermique (30 sec à 42°C) fait pénétrer l'ADN plasmidique dans les bactéries, qui sont remises immédiatement sur glace quelques minutes, avant d'être incubées 1 heure à 37°C, dans 250 µl de milieu SOC (Tryptone 2 % ; NaCl 10 mM ; KC1 2,5 mM ; MgCl₂ 10 mM; glucose 20 mM, extraits de levure 5 g/l). On isole ensuite les colonies sur boîtes de Pétri contenant du LB agar (milieu de Luria-Bertani), supplémenté en ampicilline (50 µl /ml), 40 µl de X-Gal (40 mg/ml) et 40 µl d'IPTG (100 mM) sont répartis.

### 6.3-Extraction plasmidique et analyse de l'insert.

Les colonies blanches sont ensemencées en bouillon LB-ampicilline (50 µl/ml) et incubées 18 heures, à 37°C, sous agitation. Une colonie bleue, c'est-à-dire n'ayant pas inséré de fragment est retenue comme témoin négatif de ligation.

L'extraction plasmidique est réalisée à l'aide d'un kit commercial QIAprep® Spin Miniprep (Qiagen). Brièvement, après centrifugation (3000 tours par min à +4°C) et élimination du surnageant, le culot bactérien est suspendu dans 250 µl de tampon (Tris-HCl 50 mM pH 8, EDTA 10 mM, RNase A 100 µl/ml) et lysé par ajout de 250 µl de tampon alcalin (NaOH 200 mM, SDS 1 %). Après 5 min d'homogénéisation, on ajoute 350 µl de tampon (acétate de potassium 3M, pH 5,5). Le surnageant contenant l'ADN plasmidique est ensuite transféré dans une colonne QIAprep. Une centrifugation élimine l'éluat dans le tube collecteur.

La colonne est lavée par un tampon éthanol, séchée, puis l'ADN est élué dans 50 µl d'eau stérile.

Pour vérifier l'insertion du fragment d'intérêt, le plasmide est alors digéré par l'enzyme de restriction EcoRI. La digestion est effectuée dans un mélange réactionnel de 30 µl contenant : 2 µl de la solution plasmidique, 10 U d'enzyme EcoRI (Appligène) et du tampon réactionnel 1X. La digestion dure 2 heures à 37°C et le résultat est visualisé par électrophorèse en gel d'agarose.

### 6.4- Séquencage par la méthode BigDye Terminator

Le séquençage est réalisé sur les produits PCR préalablement purifiés sur colonnes Microcon 50 (Amicon) ou sur l'ADN plasmidique. Les fragments sont, soit séquencés directement avec les amorces de PCR (fragment R0 séquencé avec les amorces 900S et 1280As) soit après clonage dans le vecteur pCRII en utilisant les amorces universelles (Sp6 et T7).

Deux clones différents ont été retenus pour chacune des régions amplifiées, afin de lever d'éventuelles ambiguïtés lors de la lecture des séquences nucléotidiques.

Le séquençage est effectué à l'aide du réactif BigDye Terminator (PE, Applied Biosystems). Le principe de séquençage consiste en une électrophorèse verticale en gel de polyacrylamide de l'ADN marqué par quatre fluochromes différents. Les matrices d'ADN sont déposées sur le gel et séparées selon leur taille, avant de soumettre le gel à un faisceau laser en continu. Le laser excite les fluochromes qui émettent chacun à une longueur d'onde différente, détectée par un spectrographe. Un logiciel informatique, couplé au séquenceur, permet l'analyse automatique et la conversion des données en séquences nucléotidiques.

Le mélange réactionnel de 10 µl comprend : 4 µl de la solution de marquage (désoxynucléosides triphosphates (dATP, dCTP, dGTP, dUTP), AmpliTaq DNA polymérase, MgCl₂, Tampon Tris-HCI pH 9), 20 pmoles d'amorce (sens ou anti-sens) et 500 ng de plasmide purifié sur colonnes de type Centricon. Les réactions de séquences (sens et antisens) sont effectuées dans un thermocycleur de type Perkin 9600, avec 25 cycles (96°C pendant 10 sec ; 50°C pendant 5 sec ; 60°C pendant 4 min). Les produits sont ensuite précipités dans 40 µl d'éthanol à 70 %, déposés sur gel et analysés grâce à un séquenceur automatique de type ABI PRISM 377.

Les séquences brutes obtenues se présentent sous forme d'électrophorégrammes. La validation et l'exploitation des séquences se fait à l'aide du Logiciel Séquence Navigator (PE, Applied Biosystems). Elles font l'objet d'au moins une double lecture, par deux amorces de séquençage différentes (Sens et Antisens), afin de minimiser les erreurs.

Ces séquences sont ensuite directement saisies sur un ordinateur en utilisant le logiciel *DNA Strider 1.3* permettant une analyse rapide des séquences.

### 7- Analyse informatique des séquences nucléotidiques et protéiques

Les séquences lues et corrigées sont comparées et soumises aux différents algorithmes phylogénétiques.

Les séquences obtenues (22 séquences) ont été comparées à 21 séquences génomiques complètes d'HDV disponibles dans GenBank (Tableau V).

**Tableau V : Numéros d'accession des différents isolats**

| **Numéros d'accès (GenBank)** | **Nom d'isolat** | **Origine géographique** |
|---|---|---|
| 1 X04451 | Italy 1 (A20) | Italie |
| 2 M84917 | Lebanon 1 | Liban |
| 3 X85253 | PatientA. | Cagliari (Italie) |
| 4 X60193 | 7/18/83 (patient S) | (patient S) Japon |
| 5 M92448 | Taiwan | Taiwan |
| 6 L22061 | Columbia | Colombie |
| 7 X77627 | Chinese human serum | Chine centrale |
| 8 L22064 | Peru-2 | Pérou |
| 9 L22063 | Peru-1 | Pérou |
| 10 L22066 | US-2 | États-Unis |
| 11 M58629 | Nauru | Île de Nauru |
| 12 U81988 | Somalia | Somalie |
| 13 U81989 | Ethiopia 1 | Ethiopie |
| 14 AF098261 | Canada | Canada(Quebec) |
| 15 U19598 | Taiwan 3 | Taiwan |
| 16 AF018077 | TW2b | Taiwan |
| 17 L22062 | Japan 3 | Japon |
| 18 AF309420 | Miyako | Ile de Miyako (Okinawa, Japon) |
| 19 D01075 | Us- 1 | États-Unis |
| 20 M21012 | W15 | Transmission expérimentale (Marmotte) |
| 21 AJ307077 | W5 | Transmission expérimentale (Marmotte) |
| 22 AJ309868 à | Isolats de Yakoutie | Yakoutie (Russie) |
| AJ309881 | | |

La première étape consiste globalement à aligner les séquences d'intérêt avec les séquences HDV de référence décrites et répertoriées dans la banque de données (Genbank), en utilisant le programme CLUSTAL W1.8 (Thompson et al., N.A.R, 1994, 22, 4673-4680). Des corrections manuelles mineures ont parfois été nécessaires à l'aide du logiciel *SeqPup* afin d'optimiser l'alignement.

Deux approches ont été suivies : l'utilisation de l'alignement des protéines pour le gène HD et l'étude de la stabilité des positions alignées à l'aide d'un logiciel d'alignement adapté.

A partir de cet alignement de séquences nucléotidiques, des arbres phylogénétiques sont construits par différents algorithmes. Les analyses sont basées sur des matrices de distances (approche phénétique), des calculs de maximum de parcimonie (MP; approche cladistique) et de maximum de vraisemblance (ML ; approche statistique).

### - approche phénétique (distance génétique)

Le principe de cette méthode est de trouver des paires de séquences voisines, en minimisant la longueur totale des branches de l'arbre. Cette approche permet de reconstruire une phylogénie sur la base du calcul de la ressemblance globale entre les séquences comparées deux à deux qui est exprimée par une distance. C'est une méthode qui permet de convertir les données de séquences en valeurs numériques de distances, arrangées en matrice. La topologie de l'arbre est construite de manière à regrouper les séquences qui possèdent le plus de caractères en commun, en utilisant une des méthodes de regroupement comme la méthode du plus proche voisin ou "*neighbor-joining"* (Saitou et al., 1987).

### - approche cladistique (maximum de parcimonie)

Le principe de cette méthode consiste à établir des relations de parenté entre les séquences par la recherche de bases nucléotidiques partagées, en minimisant les évènements génétiques. L'algorithme de maximum de parcimonie construit un arbre phylogénétique de telle manière qu'il implique le minimum de mutations. L'arbre retenu est celui qui nécessite le moins de changement. Cette méthode est sensible aux différences de taux de mutations le long des branches. Les "clades" ou "groupes monophylétiques" sont constitués par les groupes de séquences partageant un ancêtre commun, à l'exclusion de toute autre séquence.

### - approche statistique

La méthode de maximum de vraisemblance est considérée comme une approche statistique. Le programme calcule la probabilité pour qu'une séquence évolue vers une autre dans le temps. En d'autres termes, il consiste à considérer les changements au niveau de chaque site ou caractère comme des événements probabilistes indépendants. Cet algorithme de vraisemblance est cumulé sur tous les sites, et la somme est maximisée pour estimer la longueur de branche de l'arbre. Cette méthode nécessite un temps de calcul important pour rechercher l'arbre phylogénétique, le plus vraisemblable, correspondant aux séquences observées du fait qu'elle prend en compte la probabilité de changement de chaque caractère.

Toutes les analyses phylogénétiques ont été conduites en utilisant les logiciels informatiques *Phylip* 3.75 (*PHY Logenetic Inference Package*) (Felsenstein et al., 1989) et *Paup * version 4. Obêta6* (Phylogenetic Analysis Using Parsimony) (Swofford et al., 1998).

L'analyse de distance a été calculée par la méthode de Kimura à deux paramètres, qui considère les taux de transition (mutations T <-> C et G <-> A) à chaque site, et de transversion (mutations « A ou G » <---> « T ou C ») à chaque site comme différents.

La fiabilité et la robustesse des groupements de séquences (ou des topologies) sont évaluées statistiquement par l'approche de rééchantillonnage (ou bootstrap) sur 10³ et 10⁴ rééchantillonnages.

Les résultats obtenus se présentent sous la forme d'un arbre phylogénétique visualisé par le programme *Treeview* (version 1.6.5), proposant différentes présentations de l'arbre (cladogramme, radial et phylogramme). Il permet également de visualiser les valeurs de bootstrap, à chaque noeud et de déterminer un taxon comme outgroup (séquences du génotype-III).

La traduction en acides aminés du gène delta est effectuée à l'aide du programme *DNAStrider version* 1.3. L'alignement des séquences protéiques est réalisé comme décrit précédemment.

### 8- Analyse génotypique de l'HDV par polymorphisme de restriction (RFLP)

Le génotypage de l'HDV est effectué par PCR-RFLP de la région R0, selon les étapes suivantes :
- Etape 1 : Les produits PCR sont digérés par les deux enzymes de restriction *SmaI* et *XhoI* (New England Biolabs) : 10 µl de produit amplifié sont digérés séparément dans deux tubes par 5 U d'enzymes *Sma*I ou *Xho*I, respectivement à 30°C et 37°C pendant 3 heures dans un volume final de 50 µl en présence du tampon approprié et d'eau stérile. Les produits de digestion sont visualisés sous rayons ultraviolets comme décrit précédemment et les tailles des fragments sont déterminées par comparaison avec un marqueur de taille (50 pb échelle d'ADN, ou les marqueurs V et VI, Life Technologies GibcoBRL).
- Etape 2 : Les échantillons présentant un profil autre que le profil de génotype I sont digérés par une autre enzyme *Sac*II (New England Biolabs) pendant 3 heures à 37°C et les produits de digestion sont visualisés comme à l'étape 1.
- Etape 3 : le génotype du virus est déterminé à partir de l'analyse de la combinaison des profils de restriction *Sma*I*, Xho*I et *Sac*I.

### 9- Algorithme de génotypage de l'HDV par PCR-RFLP

L'algorithme de génotypage de l'HDV par PCR-RFLP comprend au moins deux étapes :
- la première, consiste en la coupure par deux enzymes de restriction, *SmaI* et *XhoI* du fragment R0 amplifié par RT-PCR à partir des ARN extraits des sérums des patients ;
- la seconde pour les patients de « profil non-I », consiste en une coupure de R0 par l'enzyme *SacII* ;
- le séquençage de la région R0 ou de la région codant pour la p24 (ou si nécessaire de la totalité du génome), suivi d'analyses phylogéniques ne seront réalisés qu'en deuxième intention si des profils de restriction inhabituels sont obtenus.

### EXEMPLE 2: MISE EN EVIDENCE DE NOUVEAUX GENOTYPES D'HDV

### 1- Analyse phylogénétique de la région R0

22 échantillons de patients infectés par HBV et HDV ont été analysés. La région R0 a été amplifiée par PCR puis le fragment obtenu a été séquencé en utilisant les amorces 900S et 1280As.

L'étude phylogénétique a été effectuée à partir d'un alignement de séquences de 336 bases de R0 (les régions ambiguës sont éliminées), en y incluant, en plus des 22 séquences étudiées, 15 séquences de référence et 6 séquences R0 d'HDV de Yakoutie (Pt13, 26 (SEQ ID NO:66), 29, 62 (SEQ ID NO:67), 63 et 704). Le nom donné aux séquences correspond à dFr (pour "delta France") suivi du numéro de sérum du patient.

### a) analyse de la distance génétique

L'arbre phylogénétique, obtenu par reconstruction à partir de distances génétiques de la région R0 est présenté à la figure 1.

La topologie de l'arbre individualise les génotypes-I et -III, représentés respectivement par sept et trois séquences nucléotidiques de référence. Les autres séquences de référence sont représentées par les séquences de type II (Japon, Taiwan-3 et les séquences de Yakoutie), et un groupe de deux séquences (TW2b, Miyako) décrites respectivement chacune comme prototype de « sous-types IIB et IIC ».

Cet arbre montre que les séquences virales provenant des 22 échantillons analysés correspondent à deux situations :
- 11 séquences sont affiliées aux séquences de génotype I, à l'exception de la séquence dFr46, qui semble être apparentée à la séquence Us-1 décrite par Makino (Makino et al., 1987) ; toutes ces séquences sont réparties de façon hétérogène au sein du génotype-L
- les 11 séquences restantes sont très éloignées du génotype-I et du génotype-III. De plus, aucune de ces séquences ne se regroupe directement avec les séquences de type-II (Japon, Taiwan-3, Yakoutie 13, 26, 29, 62, 63, 704) ou au groupe de séquences (TW2b, Miyako) ; ces séquences de référence forment par elles-mêmes deux groupes distincts.

La topologie de l'arbre, obtenu par reconstruction à partir des distances génétiques de la région R0, montre que les molécules nucléiques isolées à partir des divers HDV variants se répartissent au sein de quatre sous-groupes (figure 1):
la molécule dFr644 qui apparaît isolée ; elle possède toutefois, avec un groupe de trois molécules (dFr45, dFr2066 et dFr1843), un noeud qui est soutenu pour une valeur de rééchantillonnage de seulement 66,7 %.
. en revanche, la branche unissant les molécules dFr45, dFr2066, dFr1843 est robuste, puisqu'elle est soutenue par une valeur de bootstrap (BV) de 99,9 %.
. un ensemble de cinq molécules : dFr47, dFr910, dFr69, dFr73 et dFr1953 est soutenue par une BV de 100 % et
. une paire de molécules dFr48 et dFr2020, qui est également soutenue par une BV de 100 %.

### b) analyse du maximum de parcimonie

L'arbre phylogénétique, obtenu par reconstruction à partir du maximum de parcimonie de la région R0, est présenté à la figure 2.

L'analyse en maximum de parcimonie soutient la même topologie que l'analyse de la distance génétique. La reconstruction met en évidence l'existence au sein des 11 séquences variantes, des mêmes trois groupes monophylétiques ; par exemple, avec cette approche, le groupe de cinq molécules *dFr47, dFr9l0, dFr69, dFr73* et *dFrl953* est également soutenu par une BV de 97 % (Figure 2).

Les 11 molécules variantes, les molécules de génotype II et l'ensemble [TW2b, Miyako] semblent dériver d'une branche commune qui pourrait, par comparaison aux séquences de génotype I et de génotype III, individualiser l'ensemble des séquences du génotype II. Cependant, les valeurs de bootstrap soutenant ce branchement sont relativement modestes : 88,5 % en NJ et 64,5 % en MP (rééchantillonnage effectué sur 10⁴ échantillons) comparées à celles du génotype-I (BV = 99.8 %) et du génotype-III (BV= 100 %). De plus, la distance moyenne entre les différents sous-groupes définis au sein des 11 HDV variants ou entre ces variants et les séquences de génotype-II, apparaît plus élevée qu'entre l'ensemble des isolats du génotype-I ou au sein des trois molécules définissant le génotype-III.

L'ensemble de ces résultats mettent en valeur la caractérisation de nouveaux génotypes d'HDV.

### 2 - Analyse phylogénétique de la totalité des génomes

### a) Reconstruction du génome complet à partir de fragments amplifiés

Afin d'étudier le génome complet de ces variants et, dans le but de préciser leur affiliation, plusieurs régions du génome HDV ont été amplifiées (Tableau II) à partir de 6 échantillons incluant au moins un membre de chacun des 4 sous-groupes et trois représentants du groupe majoritaire ont été sélectionnés : *dFr45, dFr47, dFr48, dFr73, dFr644 et dFr9l0.*

De manière plus précise, les fragments suivants ont été amplifiés par PCR (Tableau IV):
- des fragments de 850 pb (R'l) et 1050 pb (R'2) chevauchants à leurs extrémités pour *dFr45, dFr47, dFr48 et dFr73,*
- deux fragments chevauchants de 960 pb et de 1250 pb pour *dFr644,* et
- deux fragments de 1400 pb et 650 pb pour *dFr9l0.*

Toutes ces régions génomiques amplifiées ont été clonées dans un vecteur pCRII™ (Tableau VI). Deux clones correspondant à chacun des fragments amplifiés ont été séquencés. La reconstruction de séquences complètes consensus d'ADNc HDV a été effectuée après alignement des régions chevauchantes et alignement avec les séquences de référence.

**Tableau VI : Clones de pcRII contenant les différents inserts**

| | R0 | R'1 | R'2 | G910 | R1 |
|---|---|---|---|---|---|
| dFr45 | - | dFr45R'1 clone 2 | dFr45R2 clone 8 | - | - |
| | | dFr45R'1 clone 4 | dFr45R2 clone 10 | | |
| dFr47 | - | dFr47R'1 clone 13 | dFr47R2 clone 19 | - | - |
| | | dFr47R'1 clone 16 | dFr47R2 clone 22 | | |
| dFr48 | - | dFr48R'1 clone 23 | dFr48R2 clone 19 | - | - |
| | | dFr48R'1 clone 28 | dFr48R2 clone 22 | | |
| dFr73 | - | dFr73R'1 clone 36 | DFr73R2 clone 29 | - | - |
| | | dFr73R'1 clone 39 | dFr48R2 clone 33 | | |
| dFr644 | - | - | - | - | 644R1 clone 4 |
| | | | | | 644R1 clone 8 |
| dFr910 | 910R0-clone 4 | - | - | R910 clone 29 | 910R1 clone 4 |
| | 910R0-clone 4 | | | R910 clone 31 | 910R1 clone 5 |

### b) Analyse de six nouvelles séquences génomiques complètes d'HDV, d'origine africaine.

### b₁) Caractéristiques cliniques des 6 patients

Cinq patients sont originaires d'Afrique de l'Ouest, et un patient a séjourné au Cameroun. Au moment du prélèvement, ces patients résidaient en région parisienne depuis au moins deux ans. Tous ces patients étaient atteints d'hépatite sévère et les données cliniques sont résumées dans la figure 3.

### b₂) l'organisation génomique des nouvelles séquences d'HDV

L'analyse comparative des régions R0 de 22 patients infectés par HDV et HBV avec celles disponibles dans les bases de données a mis en évidence la grande diversité génétique du génome viral de l'HDV.

La taille des génomes complets est différente pour les six séquences des six isolats d'HDV d'origine africaine, ce qui confirme la variabilité de l'HDV :
- le génome viral des isolats *dFr9l0, dFr47* et *dFr73* qui comprend 1697 nucléotides est le plus long jamais décrit pour HDV.
- le génome de l'isolat *dFr45* apparaît parmi les plus petits (1672 nt), et
- les séquences génomiques des virus *dFr644* et *dFr48* sont respectivement de 1680 nt et de 1687 nt.

L'analyse après l'alignement des différentes séquences étudiées, révèle un degré élevé de conservation dans les régions du génome HDV correspondant aux ribozymes responsables des clivages des ARN génomiques et antigénomiques. De même, le cadre de lecture codant pour l'antigène delta est retrouvé sur le brin anti-génomique. Un codon tryptophane (UGG) est le seul à être caractérisé pour deux séquences (*dFr47*, *dFr9l0*) et une ambiguïté (G/A) retrouvée pour les quatre autres séquences indique que la petite et la grande protéine delta sont très probablement synthétisées. Les régions variables comprennent la portion non codante ainsi que les extrémités 5' et 3' du gène *LHD.* De façon remarquable, une insertion de 7 nucléotides existe dans la séquence *dFr48.* Cette insertion est présente dans une boucle correspondant à l'une des extrémités du génome dans sa forme pseudo double brin (à la position 797 de la séquence de référence Italie (Wang et al., 1987).

### c) Comparaison des six séquences HDV d'origine africaine avec les séquences représentatives des différents génotypes.

La comparaison des six nouvelles molécules avec les molécules connues, représentatives des, trois génotypes connus, indique une similarité en nucléotides se situant entre 71,7 % (*dFr45 versus* Liban) et 80,0 % (*dFr73 versus* Yakoutie p26) au regard des molécules de génotypes I, II, et des molécules TW2b et Miyako. En effet, pour chacun des six isolats, la moyenne de similarité en nucléotides est de l'ordre de 73,3 % à 74,6 % avec les molécules du génotype I, de 74,5 % à 78,8 % avec celles du génotype II et de l'ordre de 74,6 % à 77,8 % avec les molécules TW2b/Miyako. En revanche, la similarité nucléotidique avec l'isolat du Pérou (génotype-III), n'est que de 63,9 % à 66,0 % confirmant l'éloignement particulier de cette molécule (Tableau VII). De plus, lorsque l'on compare, entre elles les six molécules correspondant à ces génomes complets et définissant les six variants *dFr45, dFr47, dFr48, dFr73, dFr644* et *dFr9l0*, seul le groupe de molécules *dFr73*, *dFr9l0* et *dFr47* présente une similarité de séquence de l'ordre de 90 %. Les molécules *dFr45, dFr48,* et *dFr644* sont aussi éloignées l'une de l'autre que des génotypes I, II, des séquences TW2b / Miyako et du groupe de molécules *dFr73*, *dFr9l0* et *dFr47* (de l'ordre de 73,2 % à 78 %) (Tableau VIII).

**Tableau VII : Pourcentage de similarité des séquences complètes HDV Africaines avec les différents génotypes connus (Calcul de la moyenne).**

| Isolats HDV* | Type I | Type II | TW2b/Miyako | Type III |
|---|---|---|---|---|
| *dFr45* | **73,3** | **74,5** | **74,6** | **66** |
| | *71,7 - 74,6* | *73,2 - 75,5* | | |
| *dFr47* | **74,2** | **78,6** | **77,4** | **65,5** |
| | *73,0 - 75,0* | *78,2 - 79,9* | | |
| *dFr48* | **73,3** | **77,1** | **75,5** | **65,4** |
| | *72,0 - 74,0* | *76,6 - 77,7* | *74,4 - 76, 6* | |
| *dFr73* | **74,1** | **78,8** | **77,8** | **65,9** |
| | *73,0 - 75,0* | *77,7 - 80,0* | *77,5 - 78,0* | |
| *dFr644* | **73,6** | **76,8** | **77,0** | **63,9** |
| | *72,2 - 74,6* | *76,2 - 77,2* | *76,9 - 77,2* | |
| *dFr910* | **74,6** | **77,9** | **77,2** | **64,6** |
| | *73,0 - 75,8* | *77,0 - 78,6* | *77,0 - 77,5* | |

| | | | | |
|---|---|---|---|---|
| * Les isolats HDV de référence correspondent aux génomes complets étudiés à l'exemple 2.1. | | | | |

**Tableau VIII : Pourcentage de similarité des nouvelles molécules HDV entre elles.**

| | *dFr47* | *dFr48* | *dFr73* | *dFr644* | *dFr9*/*0* |
|---|---|---|---|---|---|
| *dFr45* | 74,8 | 73,2 | 75 | 78 | 74,7 |
| *dFr47* | | 77,1 | **90** | 76,3 | **89** |
| *dFr48* | | | 77,7 | 75,5 | 76,1 |
| *dFr73* | | | | 76,3 | **89** |
| *dFr644* | | | | | 76,1 |

### d) Analyse phylogénétique des six molécules HDV d'origine africaine et des molécules représentatives des différents génotypes

L'analyse phylogénétique a été menée sur les six séquences complètes d'origine africaine, seize séquences de référence et 2 séquences de Yakoutie (Pt26 et Pt62). La Figure 4 illustre les résultats obtenus par analyse de distance. L'arbre phylogénétique reconstruit par "*Neighbor-Joining"* (NJ) montre qu'aucune des six séquences étudiées (*dFr45, dFr47, dFr48, dFr73, dFr644* et *dFr9l0*) ne se regroupe avec les séquences de référence du génotype I ou du génotype III. L'affiliation de ces séquences africaines aux séquences du génotypes II (aux séquences TW2b et Miyako décrites respectivement comme les sous-types IIB et IIC) n'est pas soutenue, par des valeurs de bootstrap élevées (<70 %) (Wu et al., 1998). De plus, les séquences TW2b et Miyako apparaissent former un groupe distinct et monophylétique avec une BV de 100 %. Ces deux séquences semblent constituer par elles-mêmes un "clade" représentant un génotype différent du type II.

Dans les analyses de distance, les six séquences africaines sont subdivisées en 3 sous-groupes distincts (supportés par des BV supérieures à 90,3 % pour 10⁴ rééchantillonnages). Les séquences *dFr47*, *dFr73* et *dFr910* constituent un groupe dont la branche repose sur une valeur de bootstrap de 100 %. Pour appuyer ces résultats, l'étude du maximum de parcimonie a été conduite sur le même jeu de séquences (Figure 5). En enracinant, l'arbre de façon artificielle grâce à la séquence 'Peru-1', on individualise comme dans toutes les analyses menées précédemment l'ensemble des séquences du génotype-I (BV = 100 %). La topologie des autres séquences soutient la répartition des isolats africains et asiatiques en plusieurs groupes ; ceci montre l'intérêt de l'utilisation de la région R0. Le génotype-II regroupe les séquences de Yakoutie, Taiwan-3 et Japan avec une BV de 99,9 % sur 10⁴ rééchantillonnages. De même, l'individualisation de TW2b et Miyako se confirme (BV = 100 %). Enfin, les séquences africaines indiquent l'existence d'au moins 3 sous-groupes. La monophylie des séquences *dFr47*, *dFr73*, *dFr9l0* (BV =100 %) soutient l'affiliation de ces séquences dans un sous-groupe. En revanche, la séquence *dFr48,* qui possède avec les isolats du groupe précédant (*dFr9l0*, *dFr47*, *dFr73*) une similarité nucléotidique respective de 76,1, 77,1 et 77,7 % se regroupe avec ces séquences seulement dans 55,4 % des rééchantillonages, suggérant son individualisation possible. Bien qu'apparaissant distantes l'une de l'autre, le groupement *dFr45* et *dFr644,* s'observe avec une BV élevée (NJ = 96.5 / MP = 88,6) dans le contexte étudié.

Par conséquent, à la fois les analyses phylogénétiques des régions R0 et des séquences complètes des séquences africaines indiquent que les groupes différent les uns des autres et pourraient constituer trois (voir quatre) génotypes distincts ; ces résultats mettent ainsi en évidence l'existence d'au moins sept génotypes d'HDV.

### 3- Analyse de la séquence en acides aminés (aa) de l'antigène delta (Ag-HD).

L'Ag-HD est représenté par les deux formes p24 (sHD) et p27 (LHD) de la protéine delta. La séquence protéique de 1 à 194-195 acides aminés correspond à la petite protéine delta (sHD) ou forme p24. La grande protéine delta (LHD) ou forme p27 possède la même extrémité amino-terminale et une extension de 19 à 20 acides aminés à son extrémité carboxy-terminale.

L'alignement de la séquence de l'antigène HD des six séquences africaines avec les séquences des protéines HD connues est présenté à la figure 6.

L'analyse des séquences montre que les six isolats d'origine africaine ont une identité en amino-acides de l'ordre de 69 à 77 % avec les séquences du génotype I, de 71 à 79 % avec les isolats du génotype II, de 72 à 78 % avec les séquences TW2b / Miyako et de 63 % avec l'isolat du Pérou (génotype III).

La taille des protéines correspondant aux nouveaux isolats varie entre 213 et 214 acides aminés. Toutes ces protéines ont le même profil d'hydrophobicité. La forme p24 possède 2 petites régions hydrophobes, l'une située aux alentours des acides aminés 50-60 (entre le site de polymérisation et le SLN) et l'autre entre les positions 160 et 172 (en regard d'un motif extrêmement conservé). Deux autres domaines sont bien conservés entre les différents génotypes : il s'agit du domaine de fixation à l'ARN et du domaine de localisation nucléaire. A l'image de ce qui a été décrit dans la littérature, l'extrémité carboxy-terminale de la protéine delta (entre les acides aminés 195 et 215) constitue une région hypervariable. Deux acides aminés seulement sur les 19-20 sont conservés. Il s'agit de la cystéine (C) correspondant au site de farnésylation de la forme large de la protéine HD et de la glycine (G) carboxy-terminale. De plus, on retrouve les séquences signatures spécifiques des isolats du même génotype, par exemple, les 19 acides aminés spécifiques de la grande protéine du génotype 1 ou les 20 acides aminés du génotype-III.

En revanche, pour les séquences protéiques des isolats d'origine africaine, des isolats du génotype Il et de TW2b/Miyako, l'extrémité carboxy-terminale semble subdivisée en deux domaines. Le domaine variable est représenté par les acides aminés 197 à 205 et le domaine conservé allant des acides aminés 206 à 215 (RLPLLECTPQ) (Figure 5).

### 4- Définition de 7 clades d'HDV

L'analyse des séquences complètes des six isolats d'Afrique permet de définir sept clades d'HDV correspondants aux génotypes suivants (Tableau IX) :

**Tableau IX : Correspondance Clade/Génotype**

| Clade | Génotype | Isolats |
|---|---|---|
| 1 | I | Italie, W5, W15, Us 1, Us2, Liban, Ethiopie Somalie, De de Nauru, Chine, Cagliari, Canada... |
| 2 | IIA | Japon, Taiwan3, Yakutie26, Yakutie62 |
| 3 | III | Pérou 1 |
| 4 | IIB, IIC | TW2b, Miyako |
| 5 | ? | dFr9l 0, dFr73, dFr47 |
| 6 | ? | dFr 48 |
| 7 | ? | dFr45, dFr644 |

### EXEMPLE 3: METHODE DE GENOTYPAGE DE HDV-1 à HDV-7 par PCR-RFLP

Le génotypage est effectué selon le protocole décrit à l'exemple 1.8.

### 1- Manque de sensibilité de la PCR 6A/6S

Initialement, trois patients Ag-HBs positifs, ont posé un problème diagnostique d'infection delta. En effet chez ces patients on observe une hépatite sévère associée à la présence d'IgM anti-HDV mais une absence de réplication d'HDV par RT-PCR à l'aide des amorces 6A-6S décrites dans Deny et al. (1991, 1993, 1994, précités) pour le diagnostic en routine de l'infection par HDV. La PCR 6A/6S amplifie un fragment d'ADNc de 234 pb correspondant à la partie de l'extrémité carboxyterminale du gène LHD (positions 904 à 1141 sur le génome viral).

Les ARN extraits du sérum de ces mêmes patients ont été réamplifiés à l'aide du couple d'amorces 900S et 1280AS définissant la région R0.

Les résultats obtenus à partir des échantillons de ces trois patients ont mis en évidence la présence reproductible d'une bande de 400 pb (R0) avec les amorces 900S et 1280AS, alors que la PCR 6A-6S restait négative.

Ces résultats ont été confirmés sur une série d'échantillons de sérum de patients qui ont été analysés en parallèle avec les couples d'amorces 6A-6S et 900S-1280AS. Sur 286 échantillons, 14 sont positifs uniquement avec la PCR R0.

Ces résultats démontrent une plus grande spécificité et une meilleure sensibilité des amorces 900S et 1280AS, en comparaison avec les amorces 6S et 6A, pour la détection de l'ARN d'HDV dans le sérum des patients infectés.

### 2- Profils de restriction attendus pour HDV-1 à HDV-7

Les méthodes classiquement utilisées de PCR-*RFLP* (Wu et. al., 1995a ; Wu et al., 1995b ; Casey et al., 1996) permettent la distinction de trois différents génotypes delta. L'utilisation de l'enzyme de restriction *SmaI* ne différencie pas tous les génotype-I, -IIA, -IIB reconnus à ce jour et l'enzyme *XhoI* a été utilisée pour différencier le «sous-type IIA» du «sous-type IIB» (Wu et al., 1995b)

L'association des deux enzymes *SmaI* et *XhoI* dans une première étape révèle sept profils distincts (de P1 à P7) (Tableau X). Ces sept profils ne se superposent pas exactement aux sept clades (HDV-1 à HDV-7). En conséquence, les échantillons de profil «non-P1» sont coupés dans une deuxième étape par l'enzyme *SacII*, aboutissant ainsi à l'obtention, de façon combinée, de dix profils delta distincts (de D1 à D10) (Tableau XI) pouvant être rattachés spécifiquement aux différents clades décrits, grâce aux analyses de phylogénie.

**Tableau X : Profils de restriction coupure de la Région R0, par les enzymes SmaI et XhoI.**

| **ETAPE 1** Génotypes décrits | Fragments *Sma*I Taille (pb) | Profil *Sma*I | Fragments *Xho*I Taille (pb) | Profil *XhoI* | Profil Combiné *SmaI- XhoI* | |
|---|---|---|---|---|---|---|
| I | 220,179 | *S1* | 383,16 | *X1* | *S1 X1* | **P1** |
| IIA | 397 | *S2* | 303,78,16 | *X2* | *S2 X2* | **P2** |
| IIB | 397 | *S2* | 319,79 | *X3* | *S2 X3* | **P3** |
| IIC (Miyako) | 397 | *S2* | 157,162,79 | *X4* | *S2 X4* | **P4** |
| III | 298,107 | *S3* | 405 | *X5* | *S3 X5* | **P5** |
| | | | | | | |
| II Yakutie | 178,117,110 | *S4* | 303,78,16 | *X2* | *S4 X2* | **P6** |
| *dFr45* | 217,179 | *S1* | 303,78,16 | *X2* | *S1 X2* | **P7** |
| *dFr644* | 217,179 | *S1* | 303,78,16 | *X2* | *S1 X2* | **P7** |
| *dFr47, 73,* | 179,111,107 | *S4* | 303,78,16 | *X2* | *S4 X2* | **P6** |
| *910* | | | | | | |
| *dFr48* | 397 | *S2* | 303,78,16 | *X2* | *S2 X2* | **P2** |

**Tableau XI : Profils de restriction attendus après coupure de la Région R0, par les enzymes SmaI, XhoI et SacII.**

| **ETAPE 2** Génotypes décrits | Fragments *Sac*II Taille (pb) | Profil *Sac*II | Profil Combiné *SmaI- XhoI*/ *SacII* | |
|---|---|---|---|---|
| I | 362,38 | *sc1* | *S1 X1 Sc1* | **D1** |
| IIA | 266,92,38 | *Sc2* | *S2 X2 Sc2* | **D2** |
| IIB | 268,130 | *Sc3* | *S2 X3 Sc3* | **D3** |
| IIC (Miyako) | 268,130 | *Sc3* | *S2 X4 Sc3* | **D4** |
| III | 405 | *Sc4* | *S3 X5 Sc4* | **D5** |
| | | | | |
| II Yakutie | 266,92,38 | *Sc2* | *S4 X2 Sc2* | **D6** |
| *dFr45* | 268,130 | *Sc3* | *S1 X2 Sc3* | **D7** |
| *dFr644* | 397 | *Sc4* | *S1 X2 Sc4* | **D8** |
| *dFr47, 73,* | 268,130 | *Sc3* | *S4X2 Sc3* | **D9** |
| *910* | | | | |
| *dFr48* | 268,130 | *Sc3* | *S2 X2 Sc3* | **D10** |

### 3- Génotypage des échantillons de patients par PCR-RFLP

A partir de l'analyse par PCR-RFLP d'échantillons (plus de 50) :
- aucun génotype-II ou -III n'a été retrouvé.
- 89,7 % des patients présentaient un profil D1 (génotype-I) et 10,3% un profil « non-I »
- deux nouveaux profils *XhoI* (X6 et X7) entraînant trois combinaisons nouvelles (D11, D12 et D13) supplémentaires ont été détectés (Tableaux XII et XIII).

**Tableau XII : Nouveaux profils de restriction XhoI obtenus chez cinq patients originaires d'Afrique de l'Ouest**

| **ETAPE 1** | Fragments *Sma*I | Profil | Fragments *XhoI* | Profil | Profil Combiné | |
|---|---|---|---|---|---|---|
| PATIENTS | Taille (pb) | *SmaI* | Taille (pb) | *XhoI* | *SmaI-XhoI* | |
| dFr1843 | 218, 179 | *S1* | 303, 78, 16 | *X2* | *S1 X2* | **P7** |
| dFr1953 | 218, 179 | *S1* | 303, 78, 16 | *X2* | *S1 X2* | **P7** |
| dFr2020 | 392 | *S1* | 303, 73, 16 | *X2* | *S2 X2* | **P2** |
| dFr2088 | 220, 179 | *S1* | 242, 171, 16 | ***X6*** | *S1 X6* | **P8** |
| dFr2066 | 217, 179 | *S1* | 237, 66, 16 | ***X7*** | *S1 X7* | **P9** |

**Tableau XIII : Nouveaux profils de restriction XhoI, SmaI, SacII, obtenus chez cinq patients originaires d'Afrique de l'Ouest**

| **ETAPE 2** | Fragments *Sac*II | Profil | Profil Combiné | |
|---|---|---|---|---|
| PATIENTS | Taille (pb) | *SacII* | *SmaI-XhoIlSacII* | |
| dFr1843 | 267, 130 | *Sc3* | *S1 X2 Sc3* | **D7** |
| dFr1953 | 267, 92, 38 | *Sc2* | *S1 X2 Sc2* | **D11** |
| dFr2020 | 262, 130 | *Sc3* | *S2 X2 Sc3* | **D10** |
| dFr2088 | 396 | *Sc4* | *S1 X6 Sc4* | **D12** |
| dFr2066 | 396 | *Sc4* | *S1 X7 Sc4* | **D13** |

La correspondance entre les profils combinés et les génotypes identifiés par l'anlyse phylogénétique est présentée dans le Tableau XIV.

**Tableau XIV : Résumé des différents résultats basés sur les analyses phylogénétiques et les différents profils correspondants**

| **Clades** | **Génotypes** | **Isolats** | **Profils combinés** (*Sma*I*-Xho*I / *Sac*II) |
|---|---|---|---|
| **HDV-1** | I | Italie | **D1A** |
| | | dFr2088 | **D1B** |
| **HDV-2** | IIA | Japon | **D2A** |
| | | isolats de Yakoutie | **D2B** |
| **HDV-3** | III | Pérou1 | **D3** |
| **HDV-4** | IIB | TW2b | **D4A** |
| | IIC | Miyako | **D4B** |
| **HDV-5** | V | dFr47, dFr73 et dFr910 | **D5A** |
| | | dFr1953 | **D5B** |
| **HDV-6** | VI | dFr48, dFr2020 | **D6** |
| **HDV-7** | VII | dFr45, dFr1843 | **D7A** |
| | | dFr2066 | **D7B** |
| | | dFr644 | **D7C** |

### RÉFÉRENCES BIBLIOGRAPHIQUES

- Casey J.L. et al., Proc. Natl. Acad. Sci. USA, 1993a, 90, 9016-20.
- Casey J.L. et al., J. Infect. Dis., 1996b, 174, 920-6.
- Chang F.L. et al., Proc. Natl. Acad. Sci. USA, 1991, 88, 8490-8494.
- Chao Y.C. et al., Hepatology, 1991b, 13, 345-52.
- Deny P. et al., Res. Virol., 1994, 145, 287-95.
- Deny P. et al., J. Med. Virol., 1993, 39, 214-8.
- Deny P. et al., J. Gen. Virol., 1991, 72, 735-9.
- Felsenstein J. et al., Cladistics, 1989, 5, 164-166.
- Gaeta G.B. et al., Hepatology, 2000, 32, 824-7.
- Glenn J.S. et al., Science, 1992, 256, 1331-3.
- Hwang S. et al., Virology, 1993a,193, 924-931.
- Imazeki F. et al., J. Virol., 1990, 64, 5594-5599.
- Imazeki F. et al., Nucl. Acid Res., 1991, 19, 5439-5440.
- Lai M.M.C. et al., S. Hadziyannis, J. Taylor et F. Bonino (ed.), Hepatitis delta virus. Molecular biology, Pathogenis, and Clinical aspects, 1993, 382, 21-27. Wiley-Liss, New York.
- Lee C.M. et al., Virology, 1992, 188, 265-273.
- Lee C.M. et al., J. Med. Virol., 1996b, 49, 145-54.
- Makino S. et al., Nature, 1987, 329, 343-6.
- Nakano T. et al., J. Gen. Virol., 2001, 82, 2183-2189.
- Niro G.A. et al., J. Hepatol., 1999, 30, 564-9.
- Niro G.A. et al., Hepatology, 1997, 25, 728-34.
- Roingeard P. et al., Clin. Infect. Dis., 1992, 14, 510-14.
- Saitou N. et al., Mol. Biol. Evol., 1987, 4, 406-25.
- Sakugawa H. et al., J. Med. Virol., 1999, 58, 366-72.
- Shakil A.O. et al., Virology, 1997, 234, 160-7.
- Swofford D. et al., PAUP*: Phylogenetic Analysis Using Parsimony (and other methods), version 4.0d64.
- Thompson J.D. et al., Nuc. Acid. Res., 1994, 22, 4673-80.
- Wang K.S. et al., Nature, 1986, 323, 508-514.
- Wang K.S. et al., Nature, 1987, 328, 456.
- Wu J.C. et al., Hepatology, 1995a, 22, 1656-60.
- Wu J.C. et al., J. Gen. Virol., 1998, 79, 1105-13.
- Wu J.C. et al., Lancet, 1995b, 346, 939-41.

### LISTE DE SEQUENCES

<110> ASSISTANCE PUBLIQUE-HOPITAUX DE PARIS
   DENY Paul
   RADJEF Nadjia
   HUC-ANAIS Patricia
<120> .
<130> S1020FR17
<140>
   <141>
<160> 67
   <170> PatentIn Ver. 2.1
<210> 1
   <211> 1672
   <212> ADN
   <213> virus de l'hépatite D
<220>
   <221> source
   <222> (1)..(1672)
   <223> séquence génomique complète de dFr45
<400> 1
   atgagccaag tccgacgaag agtccggaga tgggagagga gagagacccc cgaggaggca 60
   aagtcgagag ggcggagaca ctctgaggag aggactccca agaggacaag aaggactcaa 120
   gatccgggaa cgaatcccca aaacgccggt aaagctcttg gaatgaggaa aacgggcggg 180
   atggtagaaa agagcgagcc tcccgattac gagattcccc tgaacttatt ggatctggag 240
   actccgacct taaggggtca aaggtaggac agggaggcaa tccaccagga gatttcggac 300
   aactcacctc cagaggaccc cttcagcgaa cagaagagcc tgtcccggtc gaattgtccc 360
   gtagcgatag gaggagatgc taggagtagg gagagaccgg tgcgagagag gcaagacaag 420
   agagcagcgg ggctagcgag tggatgttcc gccccccgtt ggctccgagt gaggcttatc 480
   ccggggaatt tggctawctt ccccaactag ccggtcccgg atccccttcc aratgaacgg 540
   ggggactccg gcctctcggg gaatcggcgg ctccatggtg aacccccgca gccccccact 600
   ccacactcct tccccccgcg gggcccccca tccaaagatg gaactccacc cctcagggtt 660
   cgcyatccta cccttttctt acctttggcc ggcatggtcc cagcctcctc gctggcgccg 720
   gctgggcaac attccgaggg gaccgtccct cggcaatggc gaatgggacc caagacctcc 780
   aagattccca gagagaatct ggaggtgact gggcactccc tttgccatcc gagtggacgt 840
   tcgtcctcct tcggatgccc aggtcggacc acggggaggt ggagatgcca tgccgacccg 900
   aagaggaaag aaggattcgg acgcaaacct gtgagtggaa gttcgctctt tattgggggg 960
   tacactcgag gagtggaagg cggggagggg gggtcggact aggtccctat ggaaactgtc 1020
   ggtctcctcg gatgtcgagt ccctctcccg ttctggagaa gggggactcc gggacaccta 1080
   gcagttgagg aacgaagccg cccccgggcg ctcccctcgg tggtccctcg ggagggttca 1140
   catccccaac ccgcgggccg gctattcttc ttgctctttg ctcgtcatcg gcggtcaact 1200
   tcctgagttc ctcttcttcc tccttgctga gggactttcc tcctgcggac agctgcttct 1260
   tcttgttctc gagggccttc cttcgtcggt gagcccgtct ctcctcgtcg gtgaagcctc 1320
   ccttgttact ctttttccct ggtccggagt cgacctccat ccgatctgtt cggggtctct 1380
   tcgccggggg agctccctcc ccagtcccga cctttccaat tattcctttg atgtttccca 1440
   gccagggatt gtcgtcctca agtttcttga tggtcttctt tgtcttccgg agcctcctct 1500
   cgagttcttc cgcgtcttcc cttgctttgw cccacttcga aagggtctcc tcccttccct 1560
   tccgggatct cttcgtatcg gcgtggctca tctcggcaga ggcgggcgat cctcagttct 1620
   cttactcttt tcttgaaaga ggagactgct ggacgctccg ccgtgtccga gc         1672
<210> 2
   <211> 642
   <212> ADN
   <213> virus de l'hépatite D
<220>
   <221> CDS
   <222> (1)..(692)
   <223> ADNc de LHD
<400> 2
   atg agc cac gcc gat acg aag aga tcc cgg aag gga agg gag gag acc    48
   Met Ser His Ala Asp Thr Lys Arg Ser Arg Lys Gly Arg Glu Glu Thr
    1               5                  10                  15
   ctt tcg aag tgg gwc aaa gca agg gaa gac gcg gaa gaa ctc gag agg    96
   Leu Ser Lys Trp Xaa Lys Ala Arg Glu Asp Ala Glu Glu Leu Glu Arg
               20                  25                  30
   agg ctc cgg aag aca aag aag acc atc aag aaa ctt gag gac gac aat    144
   Arg Leu Arg Lys Thr Lys Lys Thr Ile Lys Lys Leu Glu Asp Asp Asn
           35                  40                  45
   ccc tgg ctg gga aac atc aaa gga ata att gga aag gtc ggg act ggg    192
   Pro Trp Leu Gly Asn Ile Lys Gly Ile Ile Gly Lys Val Gly Thr Gly
       50                  55                  60
   gag gga gct ccc ccg gcg aag aga ccc cga aca gat cgg atg gag gtc    240
   Glu Gly Ala Pro Pro Ala Lys Arg Pro Arg Thr Asp Arg Met Glu Val
   65                  70                  75                  80
   gac tcc gga cca ggg aaa aag agt aac aag gga ggc ttc acc gac gag    288
   Asp Ser Gly Pro Gly Lys Lys Ser Asn Lys Gly Gly Phe Thr Asp Glu
                   85                  90                  95
   gag aga cgg gct cac cga cga agg aag gcc ctc gag aac aag aag aag    336
   Glu Arg Arg Ala His Arg Arg Arg Lys Ala Leu Glu Asn Lys Lys Lys
               100                 105                 110
   cag ctg tcc gca gga gga aag tcc ctc agc aag gag gaa gaa gag gaa    384
   Gln Leu Ser Ala Gly Gly Lys Ser Leu Ser Lys Glu Glu Glu Glu Glu
           115                 120                 125
   ctc agg aag ttg acc gcc gat gac gag caa aga gca aga aga ata gcc    432
   Leu Arg Lys Leu Thr Ala Asp Asp Glu Gln Arg Ala Arg Arg Ile Ala
       130                 135                 140
   ggc ccg cgg gtt ggg gat gtg aac cct ccc gag gga cca ccg agg gga    480
   Gly Pro Arg Val Gly Asp Val Asn Pro Pro Glu Gly Pro Pro Arg Gly
   145                 150                 155                 160
   gcg ccc ggg ggc ggc ttc gtt cct caa ctg cta ggt gtc ccg gag tcc    528
   Ala Pro Gly Gly Gly Phe Val Pro Gln Leu Leu Gly Val Pro Glu Ser
                   165                 170                 175
   ccc ttc tcc aga acg gga gag gga ctc gac atc cga gga gac cga cag    576
   Pro Phe Ser Arg Thr Gly Glu Gly Leu Asp Ile Arg Gly Asp Arg Gln
               180                 185                 190
   ttt cca tgg gga cct agt ccg acc ccc cct ccc cgc ctt cca ctc ctc    624
   Phe Pro Trp Gly Pro Ser Pro Thr Pro Pro Pro Arg Leu Pro Leu Leu
           195                 200                 205
   gag tgt acc ccc caa taa                                            642
   Glu Cys Thr Pro Gln
       210
<210> 3
   <211> 213
   <212> PRT
   <213> virus de l'hépatite D
<400> 3
   Met Ser His Ala Asp Thr Lys Arg Ser Arg Lys Gly Arg Glu Glu Thr
   1               5                   10                  15
   Leu Ser Lys Trp Xaa Lys Ala Arg Glu Asp Ala Glu Glu Leu Glu Arg
               20                  25                  30
   Arg Leu Arg Lys Thr Lys Lys Thr Ile Lys Lys Leu Glu Asp Asp Asn
           35                  40                  45
   Pro Trp Leu Gly Asn Ile Lys Gly Ile Ile Gly Lys Val Gly Thr Gly
       50                  55                  60
   Glu Gly Ala Pro Pro Ala Lys Arg Pro Arg Thr Asp Arg Met Glu Val
   65                  70                  75                  80
   Asp Ser Gly Pro Gly Lys Lys Ser Asn Lys Gly Gly Phe Thr Asp Glu
                   85                  90                  95
   Glu Arg Arg Ala His Arg Arg Arg Lys Ala Leu Glu Asn Lys Lys Lys
               100                 105                 110
   Gln Leu Ser Ala Gly Gly Lys Ser Leu Ser Lys Glu Glu Glu Glu Glu
           115                 120                 125
   Leu Arg Lys Leu Thr Ala Asp Asp Glu Gln Arg Ala Arg Arg Ile Ala
       130                 135                 140
   Gly Pro Arg Val Gly Asp Val Asn Pro Pro Glu Gly Pro Pro Arg Gly
   145                 150                 155                 160
   Ala Pro Gly Gly Gly Phe Val Pro Gln Leu Leu Gly Val Pro Glu Ser
                   165                 170                 175
   Pro Phe Ser Arg Thr Gly Glu Gly Leu Asp Ile Arg Gly Asp Arg Gln
               180                 185                 190
   Phe Pro Trp Gly Pro Ser Pro Thr Pro Pro Pro Arg Leu Pro Leu Leu
           195                 200                 205
   Glu Cys Thr Pro Gln
       210
<210> 4
   <211> 585
   <212> ADN
   <213> virus de l'hépatite D
<220>
   <221> CDS
   <222> (1)..(585)
   <223> ADNc de sHD
<400> 4
   atg agc cac gcc gat acg aag aga tcc cgg aag gga agg gag gag acc      48
   Met Ser His Ala Asp Thr Lys Arg Ser Arg Lys Gly Arg Glu Glu Thr
    1               5                  10                  15
   ctt tcg aag tgg gwc aaa gca agg gaa gac gcg gaa gaa ctc gag agg      96
   Leu Ser Lys Trp Xaa Lys Ala Arg Glu Asp Ala Glu Glu Leu Glu Arg
               20                  25                  30
   agg ctc cgg aag aca aag aag acc atc aag aaa ctt gag gac gac aat     144
   Arg Leu Arg Lys Thr Lys Lys Thr Ile Lys Lys Leu Glu Asp Asp Asn
           35                  40                  45
   ccc tgg ctg gga aac atc aaa gga ata att gga aag gtc ggg act ggg     192
   Pro Trp Leu Gly Asn Ile Lys Gly Ile Ile Gly Lys Val Gly Thr Gly
       50                  55                  60
   gag gga gct ccc ccg gcg aag aga ccc cga aca gat cgg atg gag gtc     240
   Glu Gly Ala Pro Pro Ala Lys Arg Pro Arg Thr Asp Arg Met Glu Val
   65                  70                  75                  80
   gac tcc gga cca ggg aaa aag agt aac aag gga ggc ttc acc gac gag     288
   Asp Ser Gly Pro Gly Lys Lys Ser Asn Lys Gly Gly Phe Thr Asp Glu
                   85                  90                  95
   gag aga cgg gct cac cga cga agg aag gcc ctc gag aac aag aag aag     336
   Glu Arg Arg Ala His Arg Arg Arg Lys Ala Leu Glu Asn Lys Lys Lys
               100                 105                 110
   cag ctg tcc gca gga gga aag tcc ctc agc aag gag gaa gaa gag gaa     384
   Gln Leu Ser Ala Gly Gly Lys Ser Leu Ser Lys Glu Glu Glu Glu Glu
           115                 120                 125
   ctc agg aag ttg acc gcc gat gac gag caa aga gca aga aga ata gcc     432
   Leu Arg Lys Leu Thr Ala Asp Asp Glu Gln Arg Ala Arg Arg Ile Ala
       130                 135                 140
   ggc ccg cgg gtt ggg gat gtg aac cct ccc gag gga cca ccg agg gga     480
   Gly Pro Arg Val Gly Asp Val Asn Pro Pro Glu Gly Pro Pro Arg Gly
   145                 150                 155                 160
   gcg ccc ggg ggc ggc ttc gtt cct caa ctg cta ggt gtc ccg gag tcc     528
   Ala Pro Gly Gly Gly Phe Val Pro Gln Leu Leu Gly Val Pro Glu Ser
                   165                 170                 175
   ccc ttc tcc aga acg gga gag gga ctc gac atc cga gga gac cga cag     576
   Pro Phe Ser Arg Thr Gly Glu Gly Leu Asp Ile Arg Gly Asp Arg Gln
               180                 185                 190
   ttt cca tag                                                         585
   Phe Pro
           195
<210> 5
   <211> 194
   <212> PRT
   <213> virus de l'hépatite D
<400> 5
   Met Ser His Ala Asp Thr Lys Arg Ser Arg Lys Gly Arg Glu Glu Thr
   1               5                   10                  15
   Leu Ser Lys Trp Xaa Lys Ala Arg Glu Asp Ala Glu Glu Leu Glu Arg
               20                  25                  30
   Arg Leu Arg Lys Thr Lys Lys Thr Ile Lys Lys Leu Glu Asp Asp Asn
           35                  40                  45
   Pro Trp Leu Gly Asn Ile Lys Gly Ile Ile Gly Lys Val Gly Thr Gly
       50                  55                  60
   Glu Gly Ala Pro Pro Ala Lys Arg Pro Arg Thr Asp Arg Met Glu Val
   65                  70                  75                  80
   Asp Ser Gly Pro Gly Lys Lys Ser Asn Lys Gly Gly Phe Thr Asp Glu
                   85                  90                  95
   Glu Arg Arg Ala His Arg Arg Arg Lys Ala Leu Glu Asn Lys Lys Lys
               100                 105                 110
   Gln Leu Ser Ala Gly Gly Lys Ser Leu Ser Lys Glu Glu Glu Glu Glu
           115                 120                 125
   Leu Arg Lys Leu Thr Ala Asp Asp Glu Gln Arg Ala Arg Arg Ile Ala
       130                 135                 140
   Gly Pro Arg Val Gly Asp Val Asn Pro Pro Glu Gly Pro Pro Arg Gly
   145                 150                 155                 160
   Ala Pro Gly Gly Gly Phe Val Pro Gln Leu Leu Gly Val Pro Glu Ser
                   165                 170                 175
   Pro Phe Ser Arg Thr Gly Glu Gly Leu Asp Ile Arg Gly Asp Arg Gln
               180                 185                 190
   Phe Pro
<210> 6
   <211> 1697
   <212> ADN
   <213> virus de l'hépatite D
<220>
   <221> source
   <222> (1)..(1697)
   <223> séquence génomique complète de dFr47
<400> 6
   atgggccacg yagccgacaa ggagccggag gaggaggggt tggaggtccc gaggaggact 60
   cyaagcaaag aaggggagga atctcgaaaa agaactccca agaagaaccg agaacaaccy 120
   caagaagagg aggtttccca aacgcyggtg gagcacccgg gaacaaggaa acaggagaag 180
   gatcggtaga aaagagcgag cctcccgatc cgaggggccc tggacacctc agctctggag 240
   tcctccgacc cgtgaggttg aggttctccg cgcagggnag gcagccaccg ggagaaaacg 300
   gagaaaccac ctccagagga ccccttcagc gaacagagaa gcgcactggg acgtcaggat 360
   gagtccatag cgataggagg ggatgctagg agtcggcagc gaccgaagcg aggaaagaaa 420
   gtaaagarag caacggggct agcgagtgga tgttccgcct ccccggagtc ccgagtgagg 480
   cttatcccgg ggaacccggc caagccccca aaatgtctgg ttcccacggg ctccacttcc 540
   aaaaggagga gcgggggnac ttggaacgtg aggggaccag ttcagtccgt ggganttrcc 600
   ccggacctcc ggttctccac actccttccc ccctgcgggc ttcccagtaa acggtaagaa 660
   cccaccctcg gggtccgacg tcccaccctt tcttaccttg tggccggcat ggtcccagcc 720
   tcctcgctgg cgccggctgg gcaacattcc grggggaccg tccctcggta atggcgaatg 780
   ggrcccagaa ctctctctag gttcccagag agaaccgaga gaaaactggc tctcccttag 840
   ccatccgagt ggacgttctg tcctccttcg gatgcccagg tcggaccgcg gggaggtgga 900
   gatgccatgc cgacccgaag aggaaagaag gactcggacg cgaactcgtg agtggaaact 960
   tattccttta ctggggagta cactcgagga gtggaaggcg gggaggccgg ggtcccgggy 1020
   ytacccacgg gtactgctgg ttccccctca cgtccagtcc ctcccccgtc ctggagaagg 1080
   gagactcrgg aacacctagc atttgaggga caaagccgcc cccgggcgct cccctcggag 1140
   atgttccggg tgggttcaca tccccaaccc gcgggccggc tactcttctt tctcttctct 1200
   cgtcttcctc ggtcaacttc cggagttcct cttcctcctc cttgctragt gactttcctc 1260
   ccgcgctcag ttgtttcttc ttgttctcga gggccttcct tcttcggtgg tcccgtctct 1320
   ccttgtcggt gaagtctccc gcgcgagtcc tcttcctagg tccggagtcg acctccatcy 1380
   gatccgtccg ggccctcttc gccgggggag ctccctcccc gtccttccct tttcttatta 1440
   ttccgaggat gttccccagc cagggattct catcctcgag tytcttggcg gttctcttgg 1500
   ccttccggag ccttttctcg aggtcctccg cgtcttttct tgcttggatc catttctcga 1560
   ggacatcctc ccttcctccc ctccggggtt tcctctgttc ggactggctc atcctcggag 1620
   agggggcgac ggcgtctgtt ctctattctt ttttctctaa aagaggagac agcttgccgc 1680
   ccgcccccag ccccggg                                                1697
<210> 7
   <211> 645
   <212> ADN
   <213> virus de l'hépatite D
<220>
   <221> CDS
   <222> (1)..(645)
   <223> ADNc de LHD
<400> 7
   atg agc cag tcc gaa cag agg aaa ccc cgg agg gga gga agg gag gat     48
   Met Ser Gln Ser Glu Gln Arg Lys Pro Arg Arg Gly Gly Arg Glu Asp
   1               5                   10                  15
   gtc ctc gag aaa tgg atc caa gca aga aaa gac gcg gag gac ctc gag     96
   Val Leu Glu Lys Trp Ile Gln Ala Arg Lys Asp Ala Glu Asp Leu Glu
               20                  25                  30
   aaa agg ctc cgg aag gcc aag aga acc gcc aag ara ctc gag gat gag     144
   Lys Arg Leu Arg Lys Ala Lys Arg Thr Ala Lys Xaa Leu Glu Asp Glu
           35                  40                  45
   aat ccc tgg ctg ggg aac atc ctc gga ata ata aga aaa ggg aag gac     192
   Asn Pro Trp Leu Gly Asn Ile Leu Gly Ile Ile Arg Lys Gly Lys Asp
       50                  55                  60
   ggg gag gga gct ccc ccg gcg aag agg gcc cgg acg gat crg atg gag     240
   Gly Glu Gly Ala Pro Pro Ala Lys Arg Ala Arg Thr Asp Xaa Met Glu
   65                  70                  75                  80
   gtc gac tcc gga cct agg aag agg act cgc gcg gga gac ttc acc gac     288
   Val Asp Ser Gly Pro Arg Lys Arg Thr Arg Ala Gly Asp Phe Thr Asp
                   85                  90                  95
   aag gag aga cgg gac cac cga aga agg aag gcc ctc gag aac aag aag     336
   Lys Glu Arg Arg Asp His Arg Arg Arg Lys Ala Leu Glu Asn Lys Lys
               100                 105                 110
   aaa caa ctg agc gcg gga gga aag tca cty agc aag gag gag gaa gag     384
   Lys Gln Leu Ser Ala Gly Gly Lys Ser Xaa Ser Lys Glu Glu Glu Glu
           115                 120                 125
   gaa ctc cgg aag ttg acc gag gaa gac gag aga aga gaa aga aga gta     432
   Glu Leu Arg Lys Leu Thr Glu Glu Asp Glu Arg Arg Glu Arg Arg Val
       130                 135                 140
   gcc ggc ccg cgg gtt ggg gat gtg aac cca ccc gga aca tct ccg agg     480
   Ala Gly Pro Arg Val Gly Asp Val Asn Pro Pro Gly Thr Ser Pro Arg
   145                 150                 155                 160
   gga gcg ccc ggg ggc ggc ttt gtc cct caa atg cta ggt gtt ccy gag     528
   Gly Ala Pro Gly Gly Gly Phe Val Pro Gln Met Leu Gly Val Xaa Glu
                   165                 170                 175
   tct ccc ttc tcc agg acg ggg gag gga ctg gac gtg agg ggg aac cag     576
   Ser Pro Phe Ser Arg Thr Gly Glu Gly Leu Asp Val Arg Gly Asn Gln
               180                 185                 190
   cag tac ccg tgg gtg grc ccg gga ccc cgg cct ccc cgc ctt cca ctc     624
   Gln Tyr Pro Trp Val Xaa Pro Gly Pro Arg Pro Pro Arg Leu Pro Leu
           195                 200                 205
   ctc gag tgt act ccc cag taa                                         645
   Leu Glu Cys Thr Pro Gln
       210                 215
<210> 8
   <211> 214
   <212> PRT
   <213> virus de l'hépatite D
<400> 8
   Met Ser Gln Ser Glu Gln Arg Lys Pro Arg Arg Gly Gly Arg Glu Asp
   1               5                   10                  15
   Val Leu Glu Lys Trp Ile Gln Ala Arg Lys Asp Ala Glu Asp Leu Glu
               20                  25                  30
   Lys Arg Leu Arg Lys Ala Lys Arg Thr Ala Lys Xaa Leu Glu Asp Glu
           35                  40                  45
   Asn Pro Trp Leu Gly Asn Ile Leu Gly Ile Ile Arg Lys Gly Lys Asp
       50                  55                  60
   Gly Glu Gly Ala Pro Pro Ala Lys Arg Ala Arg Thr Asp Xaa Met Glu
   65                  70                  75                  80
   Val Asp Ser Gly Pro Arg Lys Arg Thr Arg Ala Gly Asp Phe Thr Asp
                   85                  90                  95
   Lys Glu Arg Arg Asp His Arg Arg Arg Lys Ala Leu Glu Asn Lys Lys
               100                 105                 110
   Lys Gln Leu Ser Ala Gly Gly Lys Ser Xaa Ser Lys Glu Glu Glu Glu
           115                 120                 125
   Glu Leu Arg Lys Leu Thr Glu Glu Asp Glu Arg Arg Glu Arg Arg Val
       130                 135                 140
   Ala Gly Pro Arg Val Gly Asp Val Asn Pro Pro Gly Thr Ser Pro Arg
   145                 150                 155                 160
   Gly Ala Pro Gly Gly Gly Phe Val Pro Gln Met Leu Gly Val Xaa Glu
                   165                 170                 175
   Ser Pro Phe Ser Arg Thr Gly Glu Gly Leu Asp Val Arg Gly Asn Gln
               180                 185                 190
   Gln Tyr Pro Trp Val Xaa Pro Gly Pro Arg Pro Pro Arg Leu Pro Leu
           195                 200                 205
   Leu Glu Cys Thr Pro Gln
       210
<210> 9
   <211> 588
   <212> ADN
   <213> virus de l'hépatite D
<220>
   <221> CDS
   <222> (1)..(588)
   <223> ADNc de sHD
<400> 9
   atg agc cag tcc gaa cag agg aaa ccc cgg agg gga gga agg gag gat      48
   Met Ser Gln Ser Glu Gln Arg Lys Pro Arg Arg Gly Gly Arg Glu Asp
    1               5                  10                  15
   gtc ctc gag aaa tgg atc caa gca aga aaa gac gcg gag gac ctc gag      96
   Val Leu Glu Lys Trp Ile Gln Ala Arg Lys Asp Ala Glu Asp Leu Glu
               20                  25                  30
   aaa agg ctc cgg aag gcc aag aga acc gcc aag ara ctc gag gat gag     144
   Lys Arg Leu Arg Lys Ala Lys Arg Thr Ala Lys Xaa Leu Glu Asp Glu
           35                  40                  45
   aat ccc tgg ctg ggg aac atc ctc gga ata ata aga aaa ggg aag gac     192
   Asn Pro Trp Leu Gly Asn Ile Leu Gly Ile Ile Arg Lys Gly Lys Asp
       50                  55                  60
   ggg gag gga gct ccc ccg gcg aag agg gcc cgg acg gat crg atg gag     240
   Gly Glu Gly Ala Pro Pro Ala Lys Arg Ala Arg Thr Asp Xaa Met Glu
   65                  70                  75                  80
   gtc gac tcc gga cct agg aag agg act cgc gcg gga gac ttc acc gac     288
   Val Asp Ser Gly Pro Arg Lys Arg Thr Arg Ala Gly Asp Phe Thr Asp
                   85                  90                  95
   aag gag aga cgg gac cac cga aga agg aag gcc ctc gag aac aag aag     336
   Lys Glu Arg Arg Asp His Arg Arg Arg Lys Ala Leu Glu Asn Lys Lys
               100                 105                 110
   aaa caa ctg agc gcg gga gga aag tca cty agc aag gag gag gaa gag     384
   Lys Gln Leu Ser Ala Gly Gly Lys Ser Xaa Ser Lys Glu Glu Glu Glu
           115                 120                 125
   gaa ctc cgg aag ttg acc gag gaa gac gag aga aga gaa aga aga gta     432
   Glu Leu Arg Lys Leu Thr Glu Glu Asp Glu Arg Arg Glu Arg Arg Val
       130                 135                 140
   gcc ggc ccg cgg gtt ggg gat gtg aac cca ccc gga aca tct ccg agg     480
   Ala Gly Pro Arg Val Gly Asp Val Asn Pro Pro Gly Thr Ser Pro Arg
   145                 150                 155                 160
   gga gcg ccc ggg ggc ggc ttt gtc cct caa atg cta ggt gtt ccy gag     528
   Gly Ala Pro Gly Gly Gly Phe Val Pro Gln Met Leu Gly Val Xaa Glu
                   165                 170                 175
   tct ccc ttc tcc agg acg ggg gag gga ctg gac gtg agg ggg aac cag     576
   Ser Pro Phe Ser Arg Thr Gly Glu Gly Leu Asp Val Arg Gly Asn Gln
               180                 185                 190
   cag tac ccg tga                                                     588
   Gln Tyr Pro
           195
<210> 10
   <211> 195
   <212> PRT
   <213> virus de l'hépatite D
<400> 10
   Met Ser Gln Ser Glu Gln Arg Lys Pro Arg Arg Gly Gly Arg Glu Asp
   1               5                   10                  15
   Val Leu Glu Lys Trp Ile Gln Ala Arg Lys Asp Ala Glu Asp Leu Glu
               20                  25                  30
   Lys Arg Leu Arg Lys Ala Lys Arg Thr Ala Lys Xaa Leu Glu Asp Glu
           35                  40                  45
   Asn Pro Trp Leu Gly Asn Ile Leu Gly Ile Ile Arg Lys Gly Lys Asp
       50                  55                  60
   Gly Glu Gly Ala Pro Pro Ala Lys Arg Ala Arg Thr Asp Xaa Met Glu
   65                  70                  75                  80
   Val Asp Ser Gly Pro Arg Lys Arg Thr Arg Ala Gly Asp Phe Thr Asp
                   85                  90                  95
   Lys Glu Arg Arg Asp His Arg Arg Arg Lys Ala Leu Glu Asn Lys Lys
               100                 105                 110
   Lys Gln Leu Ser Ala Gly Gly Lys Ser Xaa Ser Lys Glu Glu Glu Glu
           115                 120                 125
   Glu Leu Arg Lys Leu Thr Glu Glu Asp Glu Arg Arg Glu Arg Arg Val
       130                 135                 140
   Ala Gly Pro Arg Val Gly Asp Val Asn Pro Pro Gly Thr Ser Pro Arg
   145                 150                 155                 160
   Gly Ala Pro Gly Gly Gly Phe Val Pro Gln Met Leu Gly Val Xaa Glu
                   165                 170                 175
   Ser Pro Phe Ser Arg Thr Gly Glu Gly Leu Asp Val Arg Gly Asn Gln
               180                 185                 190
   Gln Tyr Pro
           195
<210> 11
   <211> 1697
   <212> ADN
   <213> virus de l'hépatite D
<220>
   <221> source
   <222> (1)..(1697)
   <223> séquence génomique complète de dFr73
<400> 11
   atgggcctcg agccgacgaa gagccggaga gggaggggtc ggggaycccg aggaggraac 60
   caagcaaaga aggggagact cccgaggraa grtatctcct aagaagagtc gagaarattc 120
   tcaagaggag gaggwatccc ctcaaagctg acggaactcc gggaacaagg aaayaggaga 180
   aggaacggta gaaaagagcg agcctcccga tccgaggggc ccggacacct cggctctgga 240
   gtcctccgac ctgtgaggtc gagaacctcc gcgcagggag acagccacca ggagaaggca 300
   garaaaccac ctccagagga ccccttcagc gaacagagaa gcactctgag gcgtcrggag 360
   agnntccata gcgataggag gagatgctag gagtgggcgg cgaccraagc gaggatcgaa 420
   agtaaagaaa gcaacggggc tagcaggtgg atgttccgcc tccccagggt cccgagtgag 480
   gcttatcccg gggaacccgg ctaagccccg aaatgtctgg ttccgccggc tccacttcca 540
   aaaggaggag cggggggact tggarcgyaa ggggccagtt cagtccgtgg ggwttacccc 600
   ggagctcccg ttctccacac tccttccccc cgcgggaccc ccaataatnt ggtaagaacc 660
   cacccactgg ggtccgacgy tcctaccctt tcttacctgt ggccggcatg gtcccagcct 720
   cctcgctggc gccggctggg caacattccg aggggaccgc ctcctcggta atggcgaatg 780
   ggacccagaw ctctctctag gttcccagag agaaccgaga gaaaactggc tctcccttag 840
   ccatccgagt ggacgtctgt cctccttcgg atgcccaggt cggaccgcgg ggaggtggag 900
   atgccatgcc gacccgaaga ggaaagaagg actcggacgc gaacccgtga gtggaacctt 960
   attcctttat tggggagtac actcgaggag tggaaggcgg ggagacgggg ctcccgggct 1020
   taccyacggg aactgctggt tcccccttat gtccagtccc tctcccgtcc tggtgaaggr 1080
   agactcggga acgttcagca tttgagggac aaagccgccc ccgggcgctc ccctcggagg 1140
   tccctgtggt gggttcacat ccccaacccg cgggccggct actcttcttt cccttctctc 1200
   gtcttcytcg gtcaacctcc cgagttcctc ttcttcctcc ttgctgagtg actttccycc 1260
   cgcgttcagc tgtttcttct tgttctcgag ggccttcctt cttcggtggt cctgtctctc 1320
   cttgtcggtg aagtctcccg cgcgagatct cttcctaggt ccggaatcga cctccatccg 1380
   atccgtccgg gccctcttcg ccgggggagc tccctccccg tccttccctt ttcttattat 1440
   tccgaggacg ttccccagcc agggattttc gtcctcgagt ctcttgatgg ttctcttggt 1500
   cttccggagc cttytctcga ggtcstccgc rtcttttctt gcttggaycc atttctcgag 1560
   grtctcctcc cttcctcccc tccgggattt cttctgttcg gactggctca tcctcggata 1620
   gggggcgacg gcgtctgttc tctattttct tcgttctaag aagaggagay agcgtgccgc 1680
   ccgcccccag ccccggg                                                1697
<210> 12
   <211> 645
   <212> ADN
   <213> virus de l'hépatite D
<220>
   <221> CDS
   <222> (1)..(695)
   <223> ADNc de LHD
<400> 12
   atg agc cag tcc gaa cag aag aaa tcc cgg agg gga gga agg gag gag    48
   Met Ser Gln Ser Glu Gln Lys Lys Ser Arg Arg Gly Gly Arg Glu Glu
     1               5                  10                  15
   ayc ctc gag aaa tgg rtc caa gca aga aaa gay gcg gas gac ctc gag    96
   Xaa Leu Glu Lys Trp Xaa Gln Ala Arg Lys Asp Ala Xaa Asp Leu Glu
               20                  25                  30
   ara agg ctc cgg aag acc aag aga acc atc aag aga ctc gag gac gaa   144
   Xaa Arg Leu Arg Lys Thr Lys Arg Thr Ile Lys Arg Leu Glu Asp Glu
           35                  40                  45
   aat ccc tgg ctg ggg aac gtc ctc gga ata ata aga aaa ggg aag gac   192
   Asn Pro Trp Leu Gly Asn Val Leu Gly Ile Ile Arg Lys Gly Lys Asp
       50                  55                  60
   ggg gag gga gct ccc ccg gcg aag agg gcc cgg acg gat cgg atg gag   240
   Gly Glu Gly Ala Pro Pro Ala Lys Arg Ala Arg Thr Asp Arg Met Glu
   65                  70                  75                  80
   gtc gat tcc gga cct agg aag aga tct cgc gcg gga gac ttc acc gac   288
   Val Asp Ser Gly Pro Arg Lys Arg Ser Arg Ala Gly Asp Phe Thr Asp
                   85                  90                  95
   aag gag aga cag gac cac cga aga agg aag gcc ctc gag aac aag aag   336
   Lys Glu Arg Gln Asp His Arg Arg Arg Lys Ala Leu Glu Asn Lys Lys
               100                 105                 110
   aaa cag ctg aac gcg ggr gga aag tca ctc agc aag gag gaa gaa gag   384
   Lys Gln Leu Asn Ala Xaa Gly Lys Ser Leu Ser Lys Glu Glu Glu Glu
           115                 120                 125
   gaa ctc ggg agg ttg acc gar gaa gac gag aga agg gaa aga aga gta   432
   Glu Leu Gly Arg Leu Thr Glu Glu Asp Glu Arg Arg Glu Arg Arg Val
       130                 135                 140
   gcc ggc ccg cgg gtt ggg gat gtg aac cca cca cag gga cct ccg agg   480
   Ala Gly Pro Arg Val Gly Asp Val Asn Pro Pro Gln Gly Pro Pro Arg
   145                 150                 155                 160
   gga gcg ccc ggg ggc ggc ttt gtc cct caa atg ctg aac gtt ccc gag   528
   Gly Ala Pro Gly Gly Gly Phe Val Pro Gln Met Leu Asn Val Pro Glu
                   165                 170                 175
   tct ycc ttc acc agg acg gga gag gga ctg gac ata agg ggg aac cag   576
   Ser Xaa Phe Thr Arg Thr Gly Glu Gly Leu Asp Ile Arg Gly Asn Gln
               180                 185                 190
   cag ttc ccg tgg gta agc ccg gga gcc ccg tct ccc cgc ctt cca ctc   624
   Gln Phe Pro Trp Val Ser Pro Gly Ala Pro Ser Pro Arg Leu Pro Leu
           195                 200                 205
   ctc gag tgt act ccc caa taa                                       645
   Leu Glu Cys Thr Pro Gln
       210                 215
<210> 13
   <211> 214
   <212> PRT
   <213> virus de l'hépatite D
<400> 13
   Met Ser Gln Ser Glu Gln Lys Lys Ser Arg Arg Gly Gly Arg Glu Glu
   1               5                   10                  15
   Xaa Leu Glu Lys Trp Xaa Gln Ala Arg Lys Asp Ala Xaa Asp Leu Glu
               20                  25                  30
   Xaa Arg Leu Arg Lys Thr Lys Arg Thr Ile Lys Arg Leu Glu Asp Glu
           35                  40                  45
   Asn Pro Trp Leu Gly Asn Val Leu Gly Ile Ile Arg Lys Gly Lys Asp
       50                  55                  60
   Gly Glu Gly Ala Pro Pro Ala Lys Arg Ala Arg Thr Asp Arg Met Glu
   65                  70                  75                  80
   Val Asp Ser Gly Pro Arg Lys Arg Ser Arg Ala Gly Asp Phe Thr Asp
                   85                  90                  95
   Lys Glu Arg Gln Asp His Arg Arg Arg Lys Ala Leu Glu Asn Lys Lys
               100                 105                 110
   Lys Gln Leu Asn Ala Xaa Gly Lys Ser Leu Ser Lys Glu Glu Glu Glu
           115                 120                 125
   Glu Leu Gly Arg Leu Thr Glu Glu Asp Glu Arg Arg Glu Arg Arg Val
       130                 135                 140
   Ala Gly Pro Arg Val Gly Asp Val Asn Pro Pro Gln Gly Pro Pro Arg
   145                 150                 155                 160
   Gly Ala Pro Gly Gly Gly Phe Val Pro Gln Met Leu Asn Val Pro Glu
                   165                 170                 175
   Ser Xaa Phe Thr Arg Thr Gly Glu Gly Leu Asp Ile Arg Gly Asn Gln
               180                 185                 190
   Gln Phe Pro Trp Val Ser Pro Gly Ala Pro Ser Pro Arg Leu Pro Leu
           195                 200                 205
   Leu Glu Cys Thr Pro Gln
       210
<210> 14
   <211> 588
   <212> ADN
   <213> virus de l'hépatite D
<220>
   <221> CDS
   <222> (1)..(588)
   <223> ADNc de sHD
<400> 14
   atg agc cag tcc gaa cag aag aaa tcc cgg agg gga gga agg gag gag      48
   Met Ser Gln Ser Glu Gln Lys Lys Ser Arg Arg Gly Gly Arg Glu Glu
     1               5                  10                  15
   ayc ctc gag aaa tgg rtc caa gca aga aaa gay gcg gas gac ctc gag      96
   Xaa Leu Glu Lys Trp Xaa Gln Ala Arg Lys Asp Ala Xaa Asp Leu Glu
               20                  25                  30
   ara agg ctc cgg aag acc aag aga acc atc aag aga ctc gag gac gaa     144
   Xaa Arg Leu Arg Lys Thr Lys Arg Thr Ile Lys Arg Leu Glu Asp Glu
           35                  40                  45
   aat ccc tgg ctg ggg aac gtc ctc gga ata ata aga aaa ggg aag gac     192
   Asn Pro Trp Leu Gly Asn Val Leu Gly Ile Ile Arg Lys Gly Lys Asp
       50                  55                  60
   ggg gag gga gct ccc ccg gcg aag agg gcc cgg acg gat cgg atg gag     240
   Gly Glu Gly Ala Pro Pro Ala Lys Arg Ala Arg Thr Asp Arg Met Glu
    65                  70                  75                  80
   gtc gat tcc gga cct agg aag aga tct cgc gcg gga gac ttc acc gac     288
   Val Asp Ser Gly Pro Arg Lys Arg Ser Arg Ala Gly Asp Phe Thr Asp
                    85                  90                  95
   aag gag aga cag gac cac cga aga agg aag gcc ctc gag aac aag aag     336
   Lys Glu Arg Gln Asp His Arg Arg Arg Lys Ala Leu Glu Asn Lys Lys
               100                 105                 110
   aaa cag ctg aac gcg ggr gga aag tca ctc agc aag gag gaa gaa gag     384
   Lys Gln Leu Asn Ala Xaa Gly Lys Ser Leu Ser Lys Glu Glu Glu Glu
           115                 120                 125
   gaa ctc ggg agg ttg acc gar gaa gac gag aga agg gaa aga aga gta     432
   Glu Leu Gly Arg Leu Thr Glu Glu Asp Glu Arg Arg Glu Arg Arg Val
       130                 135                 140
   gcc ggc ccg cgg gtt ggg gat gtg aac cca cca cag gga cct ccg agg     480
   Ala Gly Pro Arg Val Gly Asp Val Asn Pro Pro Gln Gly Pro Pro Arg
   145                 150                 155                 160
   gga gcg ccc ggg ggc ggc ttt gtc cct caa atg ctg aac gtt ccc gag     528
   Gly Ala Pro Gly Gly Gly Phe Val Pro Gln Met Leu Asn Val Pro Glu
                   165                 170                 175
   tct ycc ttc acc agg acg gga gag gga ctg gac ata agg ggg aac cag     576
   Ser Xaa Phe Thr Arg Thr Gly Glu Gly Leu Asp Ile Arg Gly Asn Gln
               180                 185                 190
   cag ttc ccg tag                                                     588
   Gln Phe Pro
           195
<210> 15
   <211> 195
   <212> PRT
   <213> virus de l'hépatite D
<400> 15
   Met Ser Gln Ser Glu Gln Lys Lys Ser Arg Arg Gly Gly Arg Glu Glu
   1               5                   10                  15
   Xaa Leu Glu Lys Trp Xaa Gln Ala Arg Lys Asp Ala Xaa Asp Leu Glu
               20                  25                  30
   Xaa Arg Leu Arg Lys Thr Lys Arg Thr Ile Lys Arg Leu Glu Asp Glu
           35                  40                  45
   Asn Pro Trp Leu Gly Asn Val Leu Gly Ile Ile Arg Lys Gly Lys Asp
       50                  55                  60
   Gly Glu Gly Ala Pro Pro Ala Lys Arg Ala Arg Thr Asp Arg Met Glu
   65                  70                  75                  80
   Val Asp Ser Gly Pro Arg Lys Arg Ser Arg Ala Gly Asp Phe Thr Asp
                   85                  90                  95
   Lys Glu Arg Gln Asp His Arg Arg Arg Lys Ala Leu Glu Asn Lys Lys
               100                 105                 110
   Lys Gln Leu Asn Ala Xaa Gly Lys Ser Leu Ser Lys Glu Glu Glu Glu
           115                 120                 125
   Glu Leu Gly Arg Leu Thr Glu Glu Asp Glu Arg Arg Glu Arg Arg Val
       130                 135                 140
   Ala Gly Pro Arg Val Gly Asp Val Asn Pro Pro Gln Gly Pro Pro Arg
   145                 150                 155                 160
   Gly Ala Pro Gly Gly Gly Phe Val Pro Gln Met Leu Asn Val Pro Glu
                   165                 170                 175
   Ser Xaa Phe Thr Arg Thr Gly Glu Gly Leu Asp Ile Arg Gly Asn Gln
               180                 185                 190
   Gln Phe Pro
           195
<210> 16
   <211> 1697
   <212> ADN
   <213> virus de l'hépatite D
<220>
   <221> source
   <222> (1) .. (1697)
   <223> séquence génomique complète de dFr910
<400> 16
   atgggcctcc gtgccgacga agaatcggat cgataggaag tgggaggccc gagaggggct 60
   ccaagcaaag agaggaggaa tcccggggaa aaattctccc aagaagagtc gagagtaatc 120
   tcaagaagag gagatttccc agacgccggt ggagcaccag ggaacaagga aacaggagag 180
   gaacggtaga aaagagcgag cctcccgatc cgagggggcc ctggacccct cagctctgga 240
   gtcctccgac ccgtgaggtc gaggatcccc gcgcagggag acagccaccc ggagaagaca 300
   gagaaatcac ctccagagaa accctctcag cgaacaaaga ggcgctctga ggcgttgggg 360
   cgagtccata gcgatgggag gggatgctag gagtcggcag cgaccgaagc gaggagaaaa 420
   gtaaagaaag caacggggct agcaggtgga tgttccgcct ccccggaggt cccgagtgag 480
   gcttatcccg gggaactcgg ctaagccccg aaatgtctag ttccactgtc tccacttcca 540
   aagggaggag cgggtggacc tggaacatga ggggaccagt caatccgtgg gattgccccg 600
   gacctccggy tctccacact ccttcccccc tgcgggcttc ccagtaaaat gaaagaaacc 660
   cacccactgg ggtccgacgy cctccccctt tcttacctgt ggccggcatg gtcccagcct 720
   ccycgctggc gccggctggg caacattccg aggggaccgt ccctcggtaa tggcgaatgg 780
   gacccagaac tctctccagg ttcccagaga gaaccgagag aaaactggct ctcccttagc 840
   catccgagtg gacgttyygt cctccttcgg atgcccaggt cggaccgcgg ggaggtggag 900
   aygccatgcc gacccgaaga aggaaagaag gactcggacg cgaacccgtg agtggaactc 960
   tttatctttt actggggagt acactcgagg agtggaaggc ggggaggccg gggtcccggg 1020
   tctacccacg ggaattgctg gtttcccctt acgtccagtc cacttcccgt cctggagaag 1080
   ggagactcgg gaacatctag catttgaggg acaaagccgc ccccgggcgc tcccctcgga 1140
   ggtccctcgg gtgggttcac atccccaacc cgcgggccgg ctactcttct ttcccttctc 1200
   tcgtcttcct cggtcaactt ccggagttcc tcttcctcct ccctgctgag tgactttccc 1260
   cccgcgctca gctgattctt cttgttctcg agggctttcc ttcttcggtg atcctttctc 1320
   tcctggtcgg tgaagtctcc cgctcgggtc ctcttcctag gtccggagtc gacctccatc 1380
   tgatccgtcc gggccctttt cgccggggga gctccctccc cgtccttccc ttttcttatt 1440
   attccaagga tgttccccag ccagggattt tcatcctcga gtttcttgat ggttctcttg 1500
   gtcttccgga gcyttttctc gagatcctcc gagtcttttc ttgcttgcac ccacttctcg 1560
   aggatctcct cccttcctcc tctccgagct ttcttcgatt cggactggct catcctcgga 1620
   tagggggaga cggcttctgt tctcttattc ttccttttcg aagaggagac agcttgccgc 1680
   ccctccccca ccccggg                                                1697
<210> 17
   <211> 645
   <212> ADN
   <213> virus de l'hépatite D
<220>
   <221> CDS
   <222> (1)..(645)
   <223> Adnc de LHD
<400> 17
   atg agc cag tcc gaa tcg aag aaa gct cgg aga gga gga agg gag gag   48
   Met Ser Gln Ser Glu Ser Lys Lys Ala Arg Arg Gly Gly Arg Glu Glu
    1               5                  10                  15
   atc ctc gag aag tgg gtg caa gca aga aaa gac tcg gag gat ctc gag   96
   Ile Leu Glu Lys Trp Val Gln Ala Arg Lys Asp Ser Glu Asp Leu Glu
               20                  25                  30
   aaa arg ctc cgg aag acc aag aga acc atc aag aaa ctc gag gat gaa   144
   Lys Xaa Leu Arg Lys Thr Lys Arg Thr Ile Lys Lys Leu Glu Asp Glu
           35                  40                  45
   aat ccc tgg ctg ggg aac atc ctt gga ata ata aga aaa ggg aag gac   192
   Asn Pro Trp Leu Gly Asn Ile Leu Gly Ile Ile Arg Lys Gly Lys Asp
       50                  55                  60
   ggg gag gga gct ccc ccg gcg aaa agg gcc cgg acg gat cag atg gag   240
   Gly Glu Gly Ala Pro Pro Ala Lys Arg Ala Arg Thr Asp Gln Met Glu
   65                  70                  75                  80
   gtc gac tcc gga cct agg aag agg acc cga gcg gga gac ttc acc gac   288
   Val Asp Ser Gly Pro Arg Lys Arg Thr Arg Ala Gly Asp Phe Thr Asp
                   85                  90                  95
   cag gag aga aag gat cac cga aga agg aaa gcc ctc gag aac aag aag   336
   Gln Glu Arg Lys Asp His Arg Arg Arg Lys Ala Leu Glu Asn Lys Lys
               100                 105                 110
   aat cag ctg agc gcg ggg gga aag tca ctc agc agg gag gag gaa gag   384
   Asn Gln Leu Ser Ala Gly Gly Lys Ser Leu Ser Arg Glu Glu Glu Glu
           115                 120                 125
   gaa ctc cgg aag ttg acc gag gaa gac gag aga agg gaa aga aga gta   432
   Glu Leu Arg Lys Leu Thr Glu Glu Asp Glu Arg Arg Glu Arg Arg Val
       130                 135                 140
   gcc ggc ccg cgg gtt ggg gat gtg aac cca ccc gag gga cct ccg agg   480
   Ala Gly Pro Arg Val Gly Asp Val Asn Pro Pro Glu Gly Pro Pro Arg
   145                 150                 155                 160
   gga gcg ccc ggg ggc ggc ttt gtc cct caa atg cta gat gtt ccc gag   528
   Gly Ala Pro Gly Gly Gly Phe Val Pro Gln Met Leu Asp Val Pro Glu
                   165                 170                 175
   tct ccc ttc tcc agg acg gga agt gga ctg gac gta agg gga aac cag   576
   Ser Pro Phe Ser Arg Thr Gly Ser Gly Leu Asp Val Arg Gly Asn Gln
               180                 185                 190
   caa ttc ccg tgg gtg gac ccg gga ccc cgg cct ccc cgc ctt cca ctc   624
   Gln Phe Pro Trp Val Asp Pro Gly Pro Arg Pro Pro Arg Leu Pro Leu
           195                 200                 205
   ctc gag tgt act ccc cag taa                                       645
   Leu Glu Cys Thr Pro Gln
       210                 215
<210> 18
   <211> 214
   <212> PRT
   <213> virus de l'hépatite D
<400> 18
   Met Ser Gln Ser Glu Ser Lys Lys Ala Arg Arg Gly Gly Arg Glu Glu
   1               5                   10                  15
   Ile Leu Glu Lys Trp Val Gln Ala Arg Lys Asp Ser Glu Asp Leu Glu
               20                  25                  30
   Lys Xaa Leu Arg Lys Thr Lys Arg Thr Ile Lys Lys Leu Glu Asp Glu
           35                  40                  45
   Asn Pro Trp Leu Gly Asn Ile Leu Gly Ile Ile Arg Lys Gly Lys Asp
       50                  55                  60
   Gly Glu Gly Ala Pro Pro Ala Lys Arg Ala Arg Thr Asp Gln Met Glu
   65                  70                  75                  80
   Val Asp Ser Gly Pro Arg Lys Arg Thr Arg Ala Gly Asp Phe Thr Asp
                   85                  90                  95
   Gln Glu Arg Lys Asp His Arg Arg Arg Lys Ala Leu Glu Asn Lys Lys
               100                 105                 110
   Asn Gln Leu Ser Ala Gly Gly Lys Ser Leu Ser Arg Glu Glu Glu Glu
           115                 120                 125
   Glu Leu Arg Lys Leu Thr Glu Glu Asp Glu Arg Arg Glu Arg Arg Val
       130                 135                 140
   Ala Gly Pro Arg Val Gly Asp Val Asn Pro Pro Glu Gly Pro Pro Arg
   145                 150                 155                 160
   Gly Ala Pro Gly Gly Gly Phe Val Pro Gln Met Leu Asp Val Pro Glu
                   165                 170                 175
   Ser Pro Phe Ser Arg Thr Gly Ser Gly Leu Asp Val Arg Gly Asn Gln
               180                 185                 190
   Gln Phe Pro Trp Val Asp Pro Gly Pro Arg Pro Pro Arg Leu Pro Leu
           195                 200                 205
   Leu Glu Cys Thr Pro Gln
       210
<210> 19
   <211> 588
   <212> ADN
   <213> virus de l'hépatite D
<220>
   <221> CDS
   <222> (1)..(588)
   <223> ADNc de sHD
<400> 19
   atg agc cag tcc gaa tcg aag aaa gct cgg aga gga gga agg gag gag      48
   Met Ser Gln Ser Glu Ser Lys Lys Ala Arg Arg Gly Gly Arg Glu Glu
   1               5                   10                  15
   atc ctc gag aag tgg gtg caa gca aga aaa gac tcg gag gat ctc gag      96
   Ile Leu Glu Lys Trp Val Gln Ala Arg Lys Asp Ser Glu Asp Leu Glu
               20                  25                  30
   aaa arg ctc cgg aag acc aag aga acc atc aag aaa ctc gag gat gaa      144
   Lys Xaa Leu Arg Lys Thr Lys Arg Thr Ile Lys Lys Leu Glu Asp Glu
           35                  40                  45
   aat ccc tgg ctg ggg aac atc ctt gga ata ata aga aaa ggg aag gac      192
   Asn Pro Trp Leu Gly Asn Ile Leu Gly Ile Ile Arg Lys Gly Lys Asp
       50                  55                  60
   ggg gag gga gct ccc ccg gcg aaa agg gcc cgg acg gat cag atg gag      240
   Gly Glu Gly Ala Pro Pro Ala Lys Arg Ala Arg Thr Asp Gln Met Glu
   65                  70                  75                  80
   gtc gac tcc gga cct agg aag agg acc cga gcg gga gac ttc acc gac      288
   Val Asp Ser Gly Pro Arg Lys Arg Thr Arg Ala Gly Asp Phe Thr Asp
                   85                  90                  95
   cag gag aga aag gat cac cga aga agg aaa gcc ctc gag aac aag aag      336
   Gln Glu Arg Lys Asp His Arg Arg Arg Lys Ala Leu Glu Asn Lys Lys
               100                 105                 110
   aat cag ctg agc gcg ggg gga aag tca ctc agc agg gag gag gaa gag      384
   Asn Gln Leu Ser Ala Gly Gly Lys Ser Leu Ser Arg Glu Glu Glu Glu
           115                 120                 125
   gaa ctc cgg aag ttg acc gag gaa gac gag aga agg gaa aga aga gta      432
   Glu Leu Arg Lys Leu Thr Glu Glu Asp Glu Arg Arg Glu Arg Arg Val
       130                 135                 140
   gcc ggc ccg cgg gtt ggg gat gtg aac cca ccc gag gga cct ccg agg      480
   Ala Gly Pro Arg Val Gly Asp Val Asn Pro Pro Glu Gly Pro Pro Arg
   145                 150                 155                 160
   gga gcg ccc ggg ggc ggc ttt gtc cct caa atg cta gat gtt ccc gag      528
   Gly Ala Pro Gly Gly Gly Phe Val Pro Gln Met Leu Asp Val Pro Glu
                   165                 170                 175
   tct ccc ttc tcc agg acg gga agt gga ctg gac gta agg gga aac cag      576
   Ser Pro Phe Ser Arg Thr Gly Ser Gly Leu Asp Val Arg Gly Asn Gln
               180                 185                 190
   caa ttc ccg tga                                                      588
   Gln Phe Pro
           195
<210> 20
   <211> 195
   <212> PRT
   <213> virus de l'hépatite D
<400> 20
   Met Ser Gln Ser Glu Ser Lys Lys Ala Arg Arg Gly Gly Arg Glu Glu
   1               5                   10                  15
   Ile Leu Glu Lys Trp Val Gln Ala Arg Lys Asp Ser Glu Asp Leu Glu
               20                  25                  30
   Lys Xaa Leu Arg Lys Thr Lys Arg Thr Ile Lys Lys Leu Glu Asp Glu
           35                  40                  45
   Asn Pro Trp Leu Gly Asn Ile Leu Gly Ile Ile Arg Lys Gly Lys Asp
       50                  55                  60
   Gly Glu Gly Ala Pro Pro Ala Lys Arg Ala Arg Thr Asp Gln Met Glu
   65                  70                  75                  80
   Val Asp Ser Gly Pro Arg Lys Arg Thr Arg Ala Gly Asp Phe Thr Asp
                   85                  90                  95
   Gln Glu Arg Lys Asp His Arg Arg Arg Lys Ala Leu Glu Asn Lys Lys
               100                 105                 110
   Asn Gln Leu Ser Ala Gly Gly Lys Ser Leu Ser Arg Glu Glu Glu Glu
           115                 120                 125
   Glu Leu Arg Lys Leu Thr Glu Glu Asp Glu Arg Arg Glu Arg Arg Val
       130                 135                 140
   Ala Gly Pro Arg Val Gly Asp Val Asn Pro Pro Glu Gly Pro Pro Arg
   145                 150                 155                 160
   Gly Ala Pro Gly Gly Gly Phe Val Pro Gln Met Leu Asp Val Pro Glu
                   165                 170                 175
   Ser Pro Phe Ser Arg Thr Gly Ser Gly Leu Asp Val Arg Gly Asn Gln
               180                 185                 190
   Gln Phe Pro
           195
<210> 21
   <211> 1687
   <212> ADN
   <213> virus de l'hépatite D
<220>
   <221> source
   <222> (1)..(1687)
   <223> séquence génomique complète de dFr48
<400> 21
   agtgggccac aagccggcgg agagatcgag gatttgggga ggagagggga accgaggggg 60
   gttcgaactc aagaagagaa gattttcgag gaaatactcc caagaagttc ccggagggat 120
   ccaagagacg gaygactttc cccyattggt tgggaaaagt cccggaacca ggaaatggga 180
   aagagacggt agaaaagagc gagcctccca gttttcgagt agtcccggac ctatctaaat 240
   tggagtcatc cgaccgtatg gagaaaattt ccgacaagag gtgatccact gagggtttgc 300
   ggaaaaatca cctccagagg accccttcag cgaacggaag agactggaag ccccagagga 360
   agaccatagc catggggaga gatgctagga gtggggggcg accggaacga ggagaaaagt 420
   aaagagagca acggggctag cgagtggatg ttccgcctcc ccggggagcc gagtgaggct 480
   tatcccggag tgccgggcaa gtccccccat gcccgttcca cggctccctt ccagaagggc 540
   gggggtggcc tggagcgtcg ggccccagca gtccgtggaa ttcaccctct taccgcttct 600
   ccacactcct tcccccctgc gggctccccc cataagatga cgaggacccc actcatcggg 660
   gtccgcagtc tcatcctttc ttacctgatg gccggcatgg tcccagcctc ctcgctggcg 720
   ccggctgggc aacattccga ggggaccgtc cctcggyaat ggcgaatggg acccagaact 780
   ctctcagaat ctagggagat ctcccagatt cgagagaaaa ctggctctcc cttagccatc 840
   cgagtggacg tctgtcctcc tacggatgcc caggtcggac cgcggggagg tggagatgcc 900
   atgccgaccc gaagaggaaa gaaggactcg gacgcgaacc cgtgagtgga aacctcgttc 960
   tttattgggg agtacactcg aggagtggaa ggcggggagg gcggggagga gtgttacccy 1020
   acgggaactg ctgagtgccc ctgacgtcca gaccctcccc cgtccgggag aatggagatt 1080
   cgggaacgtg aagcatggtg gggacgaagc cccctccggg cgctcccctc ggatctccgc 1140
   cgggtgggtt cacatcccca acccgcgggc cggctgttct tcttttcctt tgctcgtctt 1200
   ccccggtcag cctcccgagt tcctcttctt cttccctgct gaggttcttc cctccggcgg 1260
   ccagttgctt cttcttgttc tcgagggcct tccttcgtcg gtgatcccgc ctctcctcgt 1320
   cggtgaaacc cgacttgtgc ggctttcccc taggtccgga atcgacctcc atttgatccg 1380
   tccgggcctt cttcgttggg ggtgctccct ccccgtcctt tccctttctt atgattccga 1440
   tgatattccc caaccaggga ttgtcatcct cgagtttctt gaggcccttc tgggtcttcc 1500
   ggagcttcct ttcgagatcc tctcgatttt ttcttagttc cacccacttc tcgaggatct 1560
   cttccctccc acctcgtttt ctcttctgct cggcggggcc catctcgact ggggggcggc 1620
   gtcctcagta ctctcttact ttctcaagaa aagaggagac tgctggtcgc ccgcccccgt 1680
   gttcgag                                                           1687
<210> 22
   <211> 642
   <212> ADN
   <213> virus de l'hépatite D
<220>
   <221> CDS
   <222> (1)..(692)
   <223> ADnc de LHD
<400> 22
   atg ggc ccc gcc gag cag aag aga aaa cga ggt ggg agg gaa gag atc    48
   Met Gly Pro Ala Glu Gln Lys Arg Lys Arg Gly Gly Arg Glu Glu Ile
     1               5                  10                  15
   ctc gag aag tgg gtg gaa cta aga aaa aat cga gag gat ctc gaa agg    96
   Leu Glu Lys Trp Val Glu Leu Arg Lys Asn Arg Glu Asp Leu Glu Arg
                20                  25                  30
   aag ctc cgg aag acc cag aag ggc ctc aag aaa ctc gag gat gac aat   144
   Lys Leu Arg Lys Thr Gln Lys Gly Leu Lys Lys Leu Glu Asp Asp Asn
            35                  40                  45
   ccc tgg ttg ggg aat atc atc gga atc ata aga aag gga aag gac ggg   192
   Pro Trp Leu Gly Asn Ile Ile Gly Ile Ile Arg Lys Gly Lys Asp Gly
        50                  55                  60
   gag gga gca ccc cca acg aag aag gcc cgg acg gat caa atg gag gtc   240
   Glu Gly Ala Pro Pro Thr Lys Lys Ala Arg Thr Asp Gln Met Glu Val
    65                  70                  75                  80
   gat tcc gga cct agg gga aag ccg cac aag tcg ggt ttc acc gac gag   288
   Asp Ser Gly Pro Arg Gly Lys Pro His Lys Ser Gly Phe Thr Asp Glu
                    85                  90                  95
   gag agg cgg gat cac cga cga agg aag gcc ctc gag aac aag aag aag   336
   Glu Arg Arg Asp His Arg Arg Arg Lys Ala Leu Glu Asn Lys Lys Lys
               100                 105                 110
   caa ctg gcc gcc gga ggg aag aac ctc agc agg gaa gaa gaa gag gaa   384
   Gln Leu Ala Ala Gly Gly Lys Asn Leu Ser Arg Glu Glu Glu Glu Glu
           115                 120                 125
   ctc ggg agg ctg acc ggg gaa gac gag caa agg aaa aga aga aca gcc   432
   Leu Gly Arg Leu Thr Gly Glu Asp Glu Gln Arg Lys Arg Arg Thr Ala
       130                 135                 140
   ggc ccg cgg gtt ggg gat gtg aac cca ccc ggc gga gat ccg agg gga   480
   Gly Pro Arg Val Gly Asp Val Asn Pro Pro Gly Gly Asp Pro Arg Gly
   145                 150                 155                 160
   gcg ccc gga ggg ggc ttc gtc ccc acc atg ctt cac gtt ccc gaa tct   528
   Ala Pro Gly Gly Gly Phe Val Pro Thr Met Leu His Val Pro Glu Ser
                   165                 170                 175
   cca ttc tcc cgg acg ggg gag ggt ctg gac gtc agg ggc act cag cag   576
   Pro Phe Ser Arg Thr Gly Glu Gly Leu Asp Val Arg Gly Thr Gln Gln
               180                 185                 190
   ttc ccg tgg ggt aac act cct ccc cgc cct ccc cgc ctt cca ctc ctc   624
   Phe Pro Trp Gly Asn Thr Pro Pro Arg Pro Pro Arg Leu Pro Leu Leu
           195                 200                 205
   gag tgt act ccc caa taa                                            642
   Glu Cys Thr Pro Gln
       210
<210> 23
   <211> 213
   <212> PRT
   <213> virus de l'hépatite D
<400> 23
   Met Gly Pro Ala Glu Gln Lys Arg Lys Arg Gly Gly Arg Glu Glu Ile
   1               5                   10                  15
   Leu Glu Lys Trp Val Glu Leu Arg Lys Asn Arg Glu Asp Leu Glu Arg
               20                  25                  30
   Lys Leu Arg Lys Thr Gln Lys Gly Leu Lys Lys Leu Glu Asp Asp Asn
           35                  40                  45
   Pro Trp Leu Gly Asn Ile Ile Gly Ile Ile Arg Lys Gly Lys Asp Gly
       50                  55                  60
   Glu Gly Ala Pro Pro Thr Lys Lys Ala Arg Thr Asp Gln Met Glu Val
   65                  70                  75                  80
   Asp Ser Gly Pro Arg Gly Lys Pro His Lys Ser Gly Phe Thr Asp Glu
                   85                  90                  95
   Glu Arg Arg Asp His Arg Arg Arg Lys Ala Leu Glu Asn Lys Lys Lys
               100                 105                 110
   Gln Leu Ala Ala Gly Gly Lys Asn Leu Ser Arg Glu Glu Glu Glu Glu
           115                 120                 125
   Leu Gly Arg Leu Thr Gly Glu Asp Glu Gln Arg Lys Arg Arg Thr Ala
       130                 135                 140
   Gly Pro Arg Val Gly Asp Val Asn Pro Pro Gly Gly Asp Pro Arg Gly
   145                 150                 155                 160
   Ala Pro Gly Gly Gly Phe Val Pro Thr Met Leu His Val Pro Glu Ser
                   165                 170                 175
   Pro Phe Ser Arg Thr Gly Glu Gly Leu Asp Val Arg Gly Thr Gln Gln
               180                 185                 190
   Phe Pro Trp Gly Asn Thr Pro Pro Arg Pro Pro Arg Leu Pro Leu Leu
           195                 200                 205
   Glu Cys Thr Pro Gln
       210
<210> 24
   <211> 585
   <212> ADN
   <213> virus de l'hépatite D
<220>
   <221> CDS
   <222> (1)..(585)
   <223> ADNc de sHD
<400> 24
   atg ggc ccc gcc gag cag aag aga aaa cga ggt ggg agg gaa gag atc      48
   Met Gly Pro Ala Glu Gln Lys Arg Lys Arg Gly Gly Arg Glu Glu Ile
    1               5                  10                  15
   ctc gag aag tgg gtg gaa cta aga aaa aat cga gag gat ctc gaa agg      96
   Leu Glu Lys Trp Val Glu Leu Arg Lys Asn Arg Glu Asp Leu Glu Arg
               20                  25                  30
   aag ctc cgg aag acc cag aag ggc ctc aag aaa ctc gag gat gac aat     144
   Lys Leu Arg Lys Thr Gln Lys Gly Leu Lys Lys Leu Glu Asp Asp Asn
           35                  40                  45
   ccc tgg ttg ggg aat atc atc gga atc ata aga aag gga aag gac ggg     192
   Pro Trp Leu Gly Asn Ile Ile Gly Ile Ile Arg Lys Gly Lys Asp Gly
       50                  55                  60
   gag gga gca ccc cca acg aag aag gcc cgg acg gat caa atg gag gtc     240
   Glu Gly Ala Pro Pro Thr Lys Lys Ala Arg Thr Asp Gln Met Glu Val
   65                  70                  75                  80
   gat tcc gga cct agg gga aag ccg cac aag tcg ggt ttc acc gac gag     288
   Asp Ser Gly Pro Arg Gly Lys Pro His Lys Ser Gly Phe Thr Asp Glu
                   85                  90                  95
   gag agg cgg gat cac cga cga agg aag gcc ctc gag aac aag aag aag     336
   Glu Arg Arg Asp His Arg Arg Arg Lys Ala Leu Glu Asn Lys Lys Lys
               100                 105                 110
   caa ctg gcc gcc gga ggg aag aac ctc agc agg gaa gaa gaa gag gaa     384
   Gln Leu Ala Ala Gly Gly Lys Asn Leu Ser Arg Glu Glu Glu Glu Glu
           115                 120                 125
   ctc ggg agg ctg acc ggg gaa gac gag caa agg aaa aga aga aca gcc     432
   Leu Gly Arg Leu Thr Gly Glu Asp Glu Gln Arg Lys Arg Arg Thr Ala
       130                 135                 140
   ggc ccg cgg gtt ggg gat gtg aac cca ccc ggc gga gat ccg agg gga     480
   Gly Pro Arg Val Gly Asp Val Asn Pro Pro Gly Gly Asp Pro Arg Gly
   145                 150                 155                 160
   gcg ccc gga ggg ggc ttc gtc ccc acc atg ctt cac gtt ccc gaa tct     528
   Ala Pro Gly Gly Gly Phe Val Pro Thr Met Leu His Val Pro Glu Ser
                   165                 170                 175
   cca ttc tcc cgg acg ggg gag ggt ctg gac gtc agg ggc act cag cag     576
   Pro Phe Ser Arg Thr Gly Glu Gly Leu Asp Val Arg Gly Thr Gln Gln
               180                 185                 190
   ttc ccg tag                                                         585
   Phe Pro
           195
<210> 25
   <211> 194
   <212> PRT
   <213> virus de l'hépatite D
<400> 25
   Met Gly Pro Ala Glu Gln Lys Arg Lys Arg Gly Gly Arg Glu Glu Ile
   1               5                   10                  15
   Leu Glu Lys Trp Val Glu Leu Arg Lys Asn Arg Glu Asp Leu Glu Arg
               20                  25                  30
   Lys Leu Arg Lys Thr Gln Lys Gly Leu Lys Lys Leu Glu Asp Asp Asn
           35                  40                  45
   Pro Trp Leu Gly Asn Ile Ile Gly Ile Ile Arg Lys Gly Lys Asp Gly
       50                  55                  60
   Glu Gly Ala Pro Pro Thr Lys Lys Ala Arg Thr Asp Gln Met Glu Val
   65                  70                  75                  80
   Asp Ser Gly Pro Arg Gly Lys Pro His Lys Ser Gly Phe Thr Asp Glu
                   85                  90                  95
   Glu Arg Arg Asp His Arg Arg Arg Lys Ala Leu Glu Asn Lys Lys Lys
               100                 105                 110
   Gln Leu Ala Ala Gly Gly Lys Asn Leu Ser Arg Glu Glu Glu Glu Glu
           115                 120                 125
   Leu Gly Arg Leu Thr Gly Glu Asp Glu Gln Arg Lys Arg Arg Thr Ala
       130                 135                 140
   Gly Pro Arg Val Gly Asp Val Asn Pro Pro Gly Gly Asp Pro Arg Gly
   145                 150                 155                 160
   Ala Pro Gly Gly Gly Phe Val Pro Thr Met Leu His Val Pro Glu Ser
                   165                 170                 175
   Pro Phe Ser Arg Thr Gly Glu Gly Leu Asp Val Arg Gly Thr Gln Gln
               180                 185                 190
   Phe Pro
<210> 26
   <211> 1680
   <212> ADN
   <213> virus de l'hépatite D
<220>
   <221> source
   <222> (1)..(1680)
   <223> séquence génomique complète de dFr644
<400> 26
   atgggccaag agccgacgaa gaggtcgaag gaggagaagg agtgagtccc caaggggttc 60
   gaagccaaag agtggaaaca ctcgaggaag agggagtccc aagagggcga ggggagcttt 120
   caggagggag ggtgaatccc cgagrcgctg gagactcccg gaatgaggaa agtggggata 180
   ggctaggtag aaaagagcga gcctcccaaa tcgagattgc cctgaaccta tcragtttgg 240
   agtcatccga gcttagggtt gaatgtagcg cagagggagg aagccaccag gagccggaga 300
   caagacacct ccagaggacc ccttcagcga acagaagagt ctgtccccag atgagaagag 360
   cgtagctaga gggggagatg ctgggagtag ggggagatcg aagcgaggag gaaagtaaag 420
   atggcaacgg ggctagcagg agggtgttcc gccccccggc ggggccgagt gaggcttatc 480
   ccggggaact cggactggtc ccggactgcc ggctccaggg gcccactcca aaggaccgag 540
   ggcaggactt ggagcaccgg gaattccgag caactccatg gtagactccg tcccccttct 600
   ccacactcct tccccccgcg ggcccccccg taaagtggag aaccccactc cgcagggtcc 660
   gcgccctcct ccctttctta cctgtggccg gcatggtccc agcctcctcg cggcgccggc 720
   cgggcaacat tccgagggga ccgtcccccg gtaatggcag atgggaccca ggcttccccc 780
   ggattccctc atggggatcg agggggagag ctggcactcc cttagccatc cgagtggacg 840
   tctgtcctcc ttcggatgcc caggtcggac cacggggagg tggagatgcc atgccgaccc 900
   gaagaggaaa gaaggagacc ggacgcaaac ctgtgagtgg aactctcdtc ctttattggg 960
   gggtacactc gaggagtgga aggcggggag gggggggcgg tctttgtccc tatggaaatt 1020
   gctggtctcc cctgatgtcc agtccatccc cgtgtctggt gaatggagac tccggaacac 1080
   ctagcatcct agggacaaat ccgcccccgg gcgctcccct cggacttcct ccgggagggt 1140
   tcacaccccc atcctgcggg ccggccgctc ttctttctct tttctcgtct tcaacgatca 1200
   acctcctgag ttccccttct tcctcctcgc tgagcttctt ccctccggag gacagttgct 1260
   tcttcttgtt ctcgagggcc ttccttcttc ggtgatccct cctctcctcg tcggtgaatc 1320
   ctcccttgcg actcttcttc cccggaccgg agtcgacctc catctgatct gttcgggctc 1380
   tcttcgctgg gggagctccc tcccctgcct tcccctttct tattattccg aggatgttcc 1440
   ccagccaggg attttcatcc tcgagtctct tgatgttcct cctcgtcttc cggatcctct 1500
   tttcgaggtc ttccagatct tttcttgctt cgacccactt tgagaggaca tcctcccgtc 1560
   ctccccttcg ttctctcttc gcatcggact ggctcatctc ggcgagggcg gcgatcctca 1620
   gtgctcttac tctttacagt agaaagagga gactgctgga tgccccgccc gggcycgagc 1680
<210> 27
   <211> 645
   <212> ADN
   <213> virus de l'hépatite D
<220>
   <221> CDS
   <222> (1)..(645)
   <223> ADNc de LHD
<400> 27
   atg agc cag tcc gat gcg aag aga gaa cga agg gga gga cgg gag gat      48
   Met Ser Gln Ser Asp Ala Lys Arg Glu Arg Arg Gly Gly Arg Glu Asp
    1               5                  10                  15
   gtc ctc tca aag tgg gtc gaa gca aga aaa gat ctg gaa gac ctc gaa      96
   Val Leu Ser Lys Trp Val Glu Ala Arg Lys Asp Leu Glu Asp Leu Glu
               20                  25                  30
   aag agg atc cgg aag acg agg agg aac atc aag aga ctc gag gat gaa     144
   Lys Arg Ile Arg Lys Thr Arg Arg Asn Ile Lys Arg Leu Glu Asp Glu
           35                  40                  45
   aat ccc tgg ctg ggg aac atc ctc gga ata ata aga aag ggg aag gca     192
   Asn Pro Trp Leu Gly Asn Ile Leu Gly Ile Ile Arg Lys Gly Lys Ala
       50                  55                  60
   ggg gag gga gct ccc cca gcg aag aga gcc cga aca gat cag atg gag     240
   Gly Glu Gly Ala Pro Pro Ala Lys Arg Ala Arg Thr Asp Gln Met Glu
   65                  70                  75                  80
   gtc gac tcc ggt ccg ggg aag aag agt cgc aag gga gga ttc acc gac     288
   Val Asp Ser Gly Pro Gly Lys Lys Ser Arg Lys Gly Gly Phe Thr Asp
                   85                  90                  95
   gag gag agg agg gat cac cga aga agg aag gcc ctc gag aac aag aag     336
   Glu Glu Arg Arg Asp His Arg Arg Arg Lys Ala Leu Glu Asn Lys Lys
               100                 105                 110
   aag caa ctg tcc tcc gga ggg aag aag ctc agc gag gag gaa gaa ggg     384
   Lys Gln Leu Ser Ser Gly Gly Lys Lys Leu Ser Glu Glu Glu Glu Gly
           115                 120                 125
   gaa ctc agg agg ttg atc gtt gaa gac gag aaa aga gaa aga aga gcg     432
   Glu Leu Arg Arg Leu Ile Val Glu Asp Glu Lys Arg Glu Arg Arg Ala
       130                 135                 140
   gcc ggc ccg cag gat ggg ggt gtg aac cct ccc gga gga agt ccg agg     480
   Ala Gly Pro Gln Asp Gly Gly Val Asn Pro Pro Gly Gly Ser Pro Arg
   145                 150                 155                 160
   gga gcg ccc ggg ggc gga ttt gtc cct agg atg cta ggt gtt ccg gag     528
   Gly Ala Pro Gly Gly Gly Phe Val Pro Arg Met Leu Gly Val Pro Glu
                   165                 170                 175
   tct cca ttc acc aga cac ggg gat gga ctg gac atc agg gga gac cag     576
   Ser Pro Phe Thr Arg His Gly Asp Gly Leu Asp Ile Arg Gly Asp Gln
               180                 185                 190
   caa ttt cca tgg gga caa aga ccg ccc ccc cct ccc cgc ctt cca ctc     624
   Gln Phe Pro Trp Gly Gln Arg Pro Pro Pro Pro Pro Arg Leu Pro Leu
           195                 200                 205
   ctc gag tgt acc ccc caa taa                                         645
   Leu Glu Cys Thr Pro Gln
       210                 215
<210> 28
   <211> 214
   <212> PRT
   <213> virus de l'hépatite D
<400> 28
   Met Ser Gln Ser Asp Ala Lys Arg Glu Arg Arg Gly Gly Arg Glu Asp
   1               5                   10                  15
   Val Leu Ser Lys Trp Val Glu Ala Arg Lys Asp Leu Glu Asp Leu Glu
               20                  25                  30
   Lys Arg Ile Arg Lys Thr Arg Arg Asn Ile Lys Arg Leu Glu Asp Glu
           35                  40                  45
   Asn Pro Trp Leu Gly Asn Ile Leu Gly Ile Ile Arg Lys Gly Lys Ala
       50                  55                  60
   Gly Glu Gly Ala Pro Pro Ala Lys Arg Ala Arg Thr Asp Gln Met Glu
   65                  70                  75                  80
   Val Asp Ser Gly Pro Gly Lys Lys Ser Arg Lys Gly Gly Phe Thr Asp
                   85                  90                  95
   Glu Glu Arg Arg Asp His Arg Arg Arg Lys Ala Leu Glu Asn Lys Lys
               100                 105                 110
   Lys Gln Leu Ser Ser Gly Gly Lys Lys Leu Ser Glu Glu Glu Glu Gly
           115                 120                 125
   Glu Leu Arg Arg Leu Ile Val Glu Asp Glu Lys Arg Glu Arg Arg Ala
       130                 135                 140
   Ala Gly Pro Gln Asp Gly Gly Val Asn Pro Pro Gly Gly Ser Pro Arg
   145                 150                 155                 160
   Gly Ala Pro Gly Gly Gly Phe Val Pro Arg Met Leu Gly Val Pro Glu
                   165                 170                 175
   Ser Pro Phe Thr Arg His Gly Asp Gly Leu Asp Ile Arg Gly Asp Gln
               180                 185                 190
   Gln Phe Pro Trp Gly Gln Arg Pro Pro Pro Pro Pro Arg Leu Pro Leu
           195                 200                 205
   Leu Glu Cys Thr Pro Gln
       210
<210> 29
   <211> 588
   <212> ADN
   <213> virus de l'hépatite D
<220>
   <221> CDS
   <222> (1)..(588)
   <223> ADNc de sHD
<400> 29
   atg agc cag tcc gat gcg aag aga gaa cga agg gga gga cgg gag gat      48
   Met Ser Gln Ser Asp Ala Lys Arg Glu Arg Arg Gly Gly Arg Glu Asp
    1               5                  10                  15
   gtc ctc tca aag tgg gtc gaa gca aga aaa gat ctg gaa gac ctc gaa      96
   Val Leu Ser Lys Trp Val Glu Ala Arg Lys Asp Leu Glu Asp Leu Glu
               20                  25                  30
   aag agg atc cgg aag acg agg agg aac atc aag aga ctc gag gat gaa     144
   Lys Arg Ile Arg Lys Thr Arg Arg Asn Ile Lys Arg Leu Glu Asp Glu
           35                  40                  45
   aat ccc tgg ctg ggg aac atc ctc gga ata ata aga aag ggg aag gca     192
   Asn Pro Trp Leu Gly Asn Ile Leu Gly Ile Ile Arg Lys Gly Lys Ala
       50                  55                  60
   ggg gag gga gct ccc cca gcg aag aga gcc cga aca gat cag atg gag     240
   Gly Glu Gly Ala Pro Pro Ala Lys Arg Ala Arg Thr Asp Gln Met Glu
   65                  70                  75                  80
   gtc gac tcc ggt ccg ggg aag aag agt cgc aag gga gga ttc acc gac     288
   Val Asp Ser Gly Pro Gly Lys Lys Ser Arg Lys Gly Gly Phe Thr Asp
                   85                  90                  95
   gag gag agg agg gat cac cga aga agg aag gcc ctc gag aac aag aag     336
   Glu Glu Arg Arg Asp His Arg Arg Arg Lys Ala Leu Glu Asn Lys Lys
               100                 105                 110
   aag caa ctg tcc tcc gga ggg aag aag ctc agc gag gag gaa gaa ggg     384
   Lys Gln Leu Ser Ser Gly Gly Lys Lys Leu Ser Glu Glu Glu Glu Gly
           115                 120                 125
   gaa ctc agg agg ttg atc gtt gaa gac gag aaa aga gaa aga aga gcg     432
   Glu Leu Arg Arg Leu Ile Val Glu Asp Glu Lys Arg Glu Arg Arg Ala
       130                 135                 140
   gcc ggc ccg cag gat ggg ggt gtg aac cct ccc gga gga agt ccg agg     480
   Ala Gly Pro Gln Asp Gly Gly Val Asn Pro Pro Gly Gly Ser Pro Arg
   145                 150                 155                 160
   gga gcg ccc ggg ggc gga ttt gtc cct agg atg cta ggt gtt ccg gag     528
   Gly Ala Pro Gly Gly Gly Phe Val Pro Arg Met Leu Gly Val Pro Glu
                   165                 170                 175
   tct cca ttc acc aga cac ggg gat gga ctg gac atc agg gga gac cag     576
   Ser Pro Phe Thr Arg His Gly Asp Gly Leu Asp Ile Arg Gly Asp Gln
               180                 185                 190
   caa ttt cca tag                                                     588
   Gln Phe Pro
           195
<210> 30
   <211> 195
   <212> PRT
   <213> virus de l'hépatite D
<400> 30
   Met Ser Gln Ser Asp Ala Lys Arg Glu Arg Arg Gly Gly Arg Glu Asp
   1 5 10 15
   Val Leu Ser Lys Trp Val Glu Ala Arg Lys Asp Leu Glu Asp Leu Glu
   20 25 30
   Lys Arg Ile Arg Lys Thr Arg Arg Asn Ile Lys Arg Leu Glu Asp Glu
   35 40 45
   Asn Pro Trp Leu Gly Asn Ile Leu Gly Ile Ile Arg Lys Gly Lys Ala
   50 55 60
   Gly Glu Gly Ala Pro Pro Ala Lys Arg Ala Arg Thr Asp Gln Met Glu
   65 70 75 80
   Val Asp Ser Gly Pro Gly Lys Lys Ser Arg Lys Gly Gly Phe Thr Asp
   85 90 95
   Glu Glu Arg Arg Asp His Arg Arg Arg Lys Ala Leu Glu Asn Lys Lys
   100 105 110
   Lys Gln Leu Ser Ser Gly Gly Lys Lys Leu Ser Glu Glu Glu Glu Gly
   115 120 125
   Glu Leu Arg Arg Leu Ile Val Glu Asp Glu Lys Arg Glu Arg Arg Ala
   130 135 140
   Ala Gly Pro Gln Asp Gly Gly Val Asn Pro Pro Gly Gly Ser Pro Arg
   145 150 155 160
   Gly Ala Pro Gly Gly Gly Phe Val Pro Arg Met Leu Gly Val Pro Glu
   165 170 175
   Ser Pro Phe Thr Arg His Gly Asp Gly Leu Asp Ile Arg Gly Asp Gln
   180 185 190
   Gln Phe Pro
   195
<210> 31
   <211> 25
   <212> ADN
   <213> virus de l'hépatite D
<400> 31
   gaggaaagaa ggacgcgaga cgcaa 25
<210> 32
   <211> 21
   <212> ADN
   <213> virus de l'hépatite D
<400> 32
   accccctcga aggtggatcg a 21
<210> 33
   <211> 22
   <212> ADN
   <213> virus de l'hépatite D
<400> 33
   catgccgacc cgaagaggaa ag 22
<210> 34
   <211> 24
   <212> ADN
   <213> virus de l'hépatite D
<400> 34
   gaaggaaggc cctcgagaac aaga 24
<210> 35
   <211> 24
   <212> ADN
   <213> virus de l'hépatite D
<400> 35
   ctccagagga ccccttcagc gaac 24
<210> 36
   <211> 24
   <212> ADN
   <213> virus de l'hépatite D
<400> 36
   cccgcgggtt ggggatgtga accc 24
<210> 37
   <211> 23 .
   <212> ADN
   <213> virus de l'hépatite D
<400> 37
   gtacactcga ggagtggaag gcg 23
<210> 38
   <211> 21
   <212> ADN
   <213> virus de l'hépatite D
<400> 38
   tctgttcgct gaaggggtcc t 21
<210> 39
   <211> 22
   <212> ADN
   <213> virus de l'hépatite D
<400> 39
   ccagaggacc ccttcagcga ac 22
<210> 40
   <211> 20
   <212> ADN
   <213> virus de l'hépatite D
<400> 40
   aacaccctcc tgctagcccc 20
<210> 41
   <211> 22
   <212> ADN
   <213> virus de l'hépatite D
<400> 41
   ccggagttcc tcttcctcct cc 22
<210> 42
   <211> 23
   <212> ADN
   <213> virus de l'hépatite D
<400> 42
   gttcgcgtcc gagtccttct ttc 23
<210> 43
   <211> 20
   <212> ADN
   <213> virus de l'hépatite D
<400> 43
   gagctttctt cgattcggac 20
<210> 44
   <211> 20
   <212> ADN
   <213> virus de l'hépatite D
<400> 44
   gactggtccc ctcatgttcc 20
<210> 45
   <211> 22
   <212> ADN
   <213> virus de l'hépatite D
<400> 45
   gttcgctgaa ggggtcctct gg 22
<210> 46
   <211> 24
   <212> ADN
   <213> virus de l'hépatite D
<400> 46
   tcatcctcga gtctcttgat ggtc 24
<210> 47
   <211> 20
   <212> ADN
   <213> virus de l'hépatite D
<400> 47
   aacatccact cgctagcccc 20
<210> 48
   <211> 396
   <212> ADN
   <213> virus de l'hépatite D
<400> 48
   catgccgacc cgaagaggaa agaaggattc ggacgcaaac ctgtgagtgg aagttcgctc 60
   tttattgggg ggtacactcg aggagtggaa ggcggggagg gggggtcgga ctaggtccct 120
   atggaaactg tcggtctcct cggatgtcga gtccctctcc cgttctggag aagggggact 180
   ccgggacacc tagcagttga ggaacgaagc cgcccccggg cgctcccctc ggtggtccct 240
   cgggagggtt cacatcccca acccgcgggc cggctattct tcttgctctt tgctcgtcat 300
   cggcggtcaa cttcctgagt tcctcttctt cctccttgct gagggacttt cctcctgcgg 360
   acagctgctt cttcttgttc tcgagggcct tccttc 396
<210> 49
   <211> 397
   <212> ADN
   <213> virus de l'hépatite D
<400> 49
   catgccgacc cgaagaggaa agaaggactc ggacgcgaac tcgtgagtgg aaacttattc 60
   ctttactggg gagtacactc gaggagtgga aggcggggag gccggggtcc cgggyytacc 120
   cacgggtact gctggttccc cctcacgtcc agtccctccc ccgtcctgga gaagggagac 180
   tcrggaacac ctagcatttg agggacaaag ccgcccccgg gcgctcccct cggagatgtt 240
   ccgggtgggt tcacatcccc aacccgcggg ccggctactc ttctttctct tctctcgtct 300
   tcctcggtca acttccggag ttcctcttcc tcctccttgc tragtgactt tcctcccgcg 360
   ctcagttgtt tcttcttgtt ctcgagggcc ttccttc 397
<210> 50
   <211> 397
   <212> ADN
   <213> virus de l'hépatite D
<400> 50
   catgccgacc cgaagaggaa agaaggactc ggacgcgaac ccgtgagtgg aaacctcgtt 60
   ctttattggg gagtacactc gaggagtgga aggcggggag ggcggggagg agtgttaccc 120
   yacgggaact gctgagtgcc cctgacgtcc agaccctccc ccgtccggga gaatggagat 180
   tcgggaacgt gaagcatggt ggggacgaag ccccctccgg gcgctcccct cggatctccg 240
   ccgggtgggt tcacatcccc aacccgcggg ccggctgttc ttcttttcct ttgctcgtct 300
   tccccggtca gcctcccgag ttcctcttct tcttccctgc tgaggttctt ccctccggcg 360
   gccagttgct tcttcttgtt ctcgagggcc ttccttc 397
<210> 51
   <211> 410
   <212> ADN
   <213> virus de l'hépatite D
<400> 51
   aygccatgcc gacccgaaga aggaaagaag gactcggacg cgaacccgtg agtggaactc 60
   tttatctttt actggggagt acactcgagg agtggaaggc ggggaggccg gggtcccggg 120
   tctacccacg ggaattgctg gtttcccctt acgtccagtc cacttcccgt cctggagaag 180
   ggagactcgg gaacatctag catttgaggg acaaagccgc ccccgggcgc tcccctcgga 240
   ggtccctcgg gtgggttcac atccccaacc cgcgggccgg ctactcttct ttcccttctc 300
   tcgtcttcct cggtcaactt ccggagttcc tcttcctcct ccctgctgag tgactttccc 360
   cccgcgctca gctgattctt cttgttctcg agggctttcc ttcttcggtg 410
<210> 52
   <211> 397
   <212> ADN
   <213> virus de l'hépatite D
<400> 52
   catgccgacc cgaagaggaa agaaggactc ggacgcgaac ccgtgagtgg aaccttattc 60
   ctttattggg gagtacactc gaggagtgga aggcggggag acggggctcc cgggcttacc 120
   yacgggaact gctggttccc ccttatgtcc agtccctctc ccgtcctggt gaaggragac 180
   tcgggaacgt tcagcatttg agggacaaag ccgcccccgg gcgctcccct cggaggtccc 240
   tgtggtgggt tcacatcccc aacccgcggg ccggctactc ttctttccct tctctcgtct 300
   tcytcggtca acctcccgag ttcctcttct tcctccttgc tgagtgactt tccycccgcg 360
   ttcagctgtt tcttcttgtt ctcgagggcc ttccttc 397
<210> 53
   <211> 399
   <212> ADN
   <213> virus de l'hépatite D
<400> 53
   catgccgacc cgaagaagga aagaaggact cggacgcgaa cccgtgagtg gaactcttta 60
   tcttttactg gggagtacac tcgaggagtg gaaggcgggg aggccggggt cccgggtcta 120
   cccacgggaa ttgctggttt ccccttacgt ccagtccact tcccgtcctg gagaagggag 180
   actcgggaac atctagcatt tgagggacaa agccgccccc gggcgctccc ctcggaggtc 240
   cctcgggtgg gttcacatcc ccaacccgcg ggccggctac tcttctttcc cttctctcgt 300
   cttcctcggt caacttccgg agttcctctt cctcctccct gctgagtgac tttccccccg 360
   cgctcagctg attcttcttg ttctcgaggg ctttccttc                       399
<210> 54
   <211> 397
   <212> ADN
   <213> virus de l'hépatite D
<400> 54
   catgccgacc cgaagaggaa agaaggattc ggacgcaaac ctgtgagtgg aaattctctt 60
   ctttattggg gagtacactc gaggagtgga aggcggggag ggggggtcgg actaggaacc 120
   tatgggaact gctggtttcc tcggatgtcc agtccatccc ccgtcctgga gaatggtgac 180
   tccgggactc ctagcagttg aggaacgaag ccgcccccgg gcgctcccct cgaaggcgcg 240
   gggggagggt tcacatcccc aacccgcggg ccggctgttc ttcttgctct tttctcgtcc 300
   tcgacggtca gcctcgcgag ttcctcttct tcttccttgc cgaggttctt tcctcccgcg 360
   gagagttgct tcctcttgtt ctcgagggcc ttccttc                         397
<210> 55
   <211> 397
   <212> ADN
   <213> virus de l'hépatite D
<400> 55
   catgccgacc cgaagaggaa agaaggactc ggacgcgaac tcgtgagtgg aaccttaatc 60
   ctttattggg gagtacactc gaggagtgga aggcggggag gccggggtcc cgagcttacc 120
   tacgggaact gctggtttcc tcttatgtcc agtccctctc ccgtcctaga gaagggagac 180
   tcgggaacgc ccagcatttg agggacaaag ccgcccccgg gcgctcccct cggaggttcc 240
   tcgggwgggt tcacatcccc aacccgcggg ccggctactc ttctttccct tctctcgtct 300
   tcctcggtca acttccggag ttcctcttcc tcctccctgc tgaggttctt tcctcccgcg 360
   gagagttgct tcctcttgtt ctcgagggcc ttccttc                         397
<210> 56
   <211> 397
   <212> ADN
   <213> virus de l'hépatite D
<400> 56
   catgccgacc cgaagaggaa agaaggactc ggacgcgaac tcgtgagtgg acacctcgtt 60
   ctttattggg gagtacactc gaggagtgga aggcggggag ggcggggagg agtgttaccc 120
   tatggaaact gctggttwcc cctcacgtcc agaccttctc ccgtccggga gaatggagat 180
   tcgggaacga gatgcatggt tgggacgaag ccccctccgg gcgctcccct cggacttccc 240
   tcgggtgggt tcacatcccc aacccgcggg ccggctattc ttcttttcct ttgctcgtca 300
   tccctggtca gcctcaggag ttcctcttct tcttccttgc tgaggttctt tcctcccgcg 360
   gagagttgct tcctcttgtt ctcgagggcc ttccttc                         397
<210> 57
   <211> 396
   <212> ADN
   <213> virus de l'hépatite D
<400> 57
   catgccgacc cgaagaggaa agaaggattc ggacgcaaac ctgtgagtgg aagctttctc 60
   tttattgggg ggtacactcg aggagtggaa ggcggggagg gggggtcgga ctaggtcccy 120
   atggaaactg ccggtttcct cggacgtcga gtccctctcc cgttctggag aagggggact 180
   ccgggactcc tagcagttga ggaacgaagc cgcccccggg cgctcccctc ggtggtccct 240
   cgggagggtt cacatcccca acctgcgggc cggctactct tcttgctctt tgctcgtctt 300
   cgacggtcaa cctctcgagt tcctcttcct cctccttgct gaggttcttt cctcccgcgg 360
   agagttgctt cctcttgttc tcgagggcct tccttc                           396
<210> 58
   <211> 398
   <212> ADN
   <213> virus de l'hépatite D
<400> 58
   catgccgacc cgaagaggaa agaaggagac cggacgcaaa cctgtgagtg gaactctcdt 60
   cctttattgg ggggtacact cgaggagtgg aaggcgggga ggggggggcg gtctttgtcc 120
   ctatggaaat tgctggtctc ccctgatgtc cagtccatcc ccgtgtctgg tgaatggaga 180
   ctccggaaca cctagcatcc tagggacaaa tccgcccccg ggcgctcccc tcggacttcc 240
   tccgggaggg ttcacacccc catcctgcgg gccggccgct cttctttctc ttttctcgtc 300
   ttcaacgatc aacctcctga gttccccttc ttcctcctcg ctgagcttct tccctccgga 360
   ggacagttgc ttcttcttgt tctcgagggc cttccttc                         398
<210> 59
   <211> 10
   <212> PRT
   <213> virus de l'hépatite D
<400> 59
   Arg Leu Pro Leu Leu Glu Cys Thr Pro Gln
   1               5                   10
<210> 60
   <211> 9
   <212> PRT
   <213> virus de l'hépatite D
<400> 60
   Trp Gly Pro Ser Pro Thr Pro Pro Pro
   1               5
<210> 61
   <211> 9
   <212> PRT
   <213> virus de l'hépatite D
<400> 61
   Trp Val Xaa Pro Gly Pro Arg Pro Pro
   1 5
<210> 62
   <211> 9
   <212> PRT
   <213> virus de l'hépatite D
<400> 62
   Trp Val Ser Pro Gly Ala Pro Ser Pro
   1 5
<210> 63
   <211> 9
   <212> PRT
   <213> virus de l'hépatite D
<400> 63
   Trp Val Asp Pro Gly Pro Arg Pro Pro
   1 5
<210> 64
   <211> 9
   <212> PRT
   <213> virus de l'hépatite D
<400> 64
   Trp Gly Asn Thr Pro Pro Arg Pro Pro
   1 5
<210> 65
   <211> 9
   <212> PRT
   <213> virus de l'hépatite D
<400> 65
   Trp Gly Gln Arg Pro Pro Pro Pro Pro
   1 5
<210> 66
   <211> 1685
   <212> ADN
   <213> virus de l'hépatite D
<220>
   <221> source
   <222> (1)..(1685)
   <223> séquence génomique complète de Pt26
<400> 66
   atgggccgca taccgagcga aggataccgg taaggggaga ggaggaatcc cgaggggatc 60
   gcccactaaa gagtagagga attctcggaa agctctccca agaagaacca gaatccctca 120
   agagagagga gaatccccat gacgctggaa gagacttccg ggtaaccaag tcaaggagaa 180
   ggaacggtag aaaagagcga gcctctcgat acgaaagggc cgcgagccta tcgagtttgg 240
   agtcatccgg gccgaagggt tgagacgcac ccacagacgg gagcctccag gagggtttga 300
   ggagaactca cctccagagg accccttcag cgaacagagg agtctctacc tcggaggaga 360
   aagaccgtag cgatggggga gatgctagga gtaggcggcg accgaagcga ggaggaaagc 420
   aaagaaagcc acggggctag cgagtggatg ttccgcccca aggggagccg agtgaggctt 480
   atcccgggga actcggtaat tcgccccgaa atgaggactc cggggtcccc ttccgaagga 540
   acggaggggg gtgactagga atcaggatcc agtggatccg tgggaattat cccgttccac 600
   ccccgcggca cactccttcc cccctgcggg cccccccata agatggcagg aacccactca 660
   ttggggtccg ctgttccatt ctttcttacc ttgtggccgg catggtccca gcctcctcgc 720
   tggcgccggc tgggcaacat tccgagggga ccgtccctcg gtaatggcga atgggaccca 780
   gaactctctc tagattccca gagagaatcg agagaaaact ggctctccct tagccatccg 840
   agtaggacgt ctgtcctcct acggatgccc aggtcggacc gcggggaggt ggagatgcca 900
   tgccgacccg aagaggaaag aagaacacgg acgcgaaccc gtaagtggaa ccctgatcct 960
   ttattggggg gtacactcga ggagtggaag gcgctgcccg gggggaaccg gatttaccta 1020
   cgggaatccc tgggttcctc tgatgtccag tccctctccc gtccgagtga agggagactc 1080
   cggaactcca gtcatttgag ggacgaatcc gcccccgggc gctcccctcg gacttcctcc 1140
   aggagggttc acatccccaa cccgcgggcc ggctactctt ctttcccttc tctcgtcttc 1200
   ctcggtcaac ctcctgagtt cctcttcttc ctccctgctg aggttctttc ctcccgcgga 1260
   gagttgcttc ctcttgttct cgagggcctt ccttctgcgg tgatcctccc tctccttgtc 1320
   ggtgaacccg ctcttgtgag gcctcttcat aggtccggag tcgacctcca tctgatctgt 1380
   tcgggccctc ttcgccgggg gagctccctc cccgtccttc ccttttctta tgattcccag 1440
   gatgttcccc agccagggat tttcatcctc gagtctcttg atggtctttc tggccttccg 1500
   gaggtctctc tcgagctcct ctgcctttct tcttgtggag atccactttt cgaggatttc 1560
   ttcccttcct cccctccggc ccttcctcga ctcgggttgg ctcatcctcg gcgagggcga 1620
   cggtcctcag ttctctctat tctttcttga aagaggagac tgctggacca aacgcccgag 1680
   ccggg                                                             1685
<210> 67
   <211> 1688
   <212> ADN
   <213> virus de l'hépatite D
<220>
   <221> source
   <222> (1)..(1688)
   <223> séquence génomique complète de Pt62
<400> 67
   atgggccgca agccgaacga aggattccgg taaggggaga ggaagaatcc cgaaggggtt 60
   ccccactaaa gagtggaaga attctcggga agcttctccc aagaagaacc agaatccccc 120
   aagagagaga atggatcccc atgacgctgg aagagactcc gggtaaccaa gtcaaggaga 180
   aggaacggta gaaaagagcg agcctctcga tacgaaaggg ccgcgaccta tcaagtttgg 240
   agtcatccgg gccaaagggt tgaaaaatcc cacagacggg agccaccagg agggatctag 300
   gagaatccac ctccagagga cccccctcaa tgaacagaag actctctacc tcggaggaaa 360
   aagaccatag cgataggaag agatgctagg agtaggcggc gaccaaagcg aggaagaaag 420
   taaagaaagc aacggggcta gcgagtggat gttccgcccc aaggggagcc gagtgaggct 480
   tatcccgggg aactcggcgt atcgtcccga aatgaggagc ccggatcccc ttccaaaaag 540
   acggagaggg ggtgactagg aatcgggctc cggtggatcc gtgggaccag cccgctccac 600
   ctccgcggca cactccttcc cccctgcggg cccccccata agatggcagg aacccactca 660
   ttggggtccg ctgttccatt ctttcttacc ttgtggccgg catggtccca gcctcctcgc 720
   tggcgccggc tgggcaacat tccgagggga ccgtccctcg gtaatggcga atgggaccca 780
   gaactctctc tagattccca gagagaatcg agagaaaact ggctctccct tagccatccg 840
   agtaggacgt ctgtcctcct acggatgccc aggtcggacc gcgaggaggt ggagatgcca 900
   tgccgacccg aagaggaaag aagaacacgg acgcgaaccc gtaagtggaa ccctgatcct 960
   ttattggggg gtacactcga ggagtggaag gcgctgcccg gggggagccg gattgaccta 1020
   cgggaatccc cggtcgcctc tgatgtccag tccctccccc gtccgagaga agggagattc 1080
   cggaactcca gtcatttgag ggacgaagcc gcccccgggc gctcccctcg gacttcctcc 1140
   aggagggttc acatccccaa cccgcgggcc ggctactctt ctttgtcttt cgtcgtcttc 1200
   aatggtcaac ctcctgagtt cctcttcttc ttccttgctg aggctctttc cccccgcgga 1260
   gagttggttc ttcttgttct ggagggcctt ccttctgcgg tggtcctgcc tctccttgtc 1320
   ggtgaacccg ctcttgtgag gtttcttcct aggtccggag tcgacctcca tctgatctgt 1380
   tcgggccctc ttcgccgggg gagctccctc cccgtccttc ccttttctta tgattcccag 1440
   gatgttcccc agccagggat tgtcatcctc gagtctcttg atggtctttc tggccttccg 1500
   gaggtctctc tcgagctctt ccgccttttt tcttgtggat acccactttt cgaggatatc 1560
   ttcccttcct cccctccggc ttttcctcga ttcggattgg ctcatcctcg acgagggcga 1620
   cggtcctcag ttctctctat tctttccttt tgaaagagga gactgctggt ccaaacgccc 1680
   gagtcggg                                                          1688

## Revendications

1. Molécules d'acide nucléique isolées, **caractérisées en ce qu'**elles sont sélectionnées dans le groupe constitué par :
- le génome complet du variant d'HDV dénommé dFr45 qui présente la séquence SEQ ID NO: 1, et
- le génome d'un variant d'HDV qui présente une divergence génétique ≤ 15 % avec la séquence SEQ ID NO: 1.

2. Molécules d'acide nucléique, **caractérisées en ce qu'**elles comprennent au moins l'un des fragments de la séquence d'un HDV variant selon la revendication 1, sélectionnés dans le groupe constitué par :
a) le fragment R0 qui présente la séquence SEQ ID NO:48,
b) le fragment R1 qui s'étend des positions 307 à 1289 du génome d'HDV,
c) le fragment R2 qui s'étend des positions 889 à 328 du génome d'HDV,
d) le fragment R3 qui s'étend des positions 1486 à 452 du génome d'HDV,
e) le fragment R'1 qui s'étend des positions 305 à 1161 du génome d'HDV,
f) le fragment R'2 qui s'étend des positions 984 à 331 du génome d'HDV,
g) l'ADNc codant pour la protéine sHD, de séquence SEQ ID NO:4,
h) l'ADNc codant pour la protéine LHD, de séquence SEQ ID NO:2,
et
i) les amorces de séquence SEQ ID NO:38 et SEQ ID NO: 47.

3. Méthode de détection d'un HDV variant tel que défini à la revendication 1 ou la revendication 2, par hybridation et/ou amplification, réalisée à partir d'un échantillon biologique, laquelle méthode est **caractérisée en ce qu'**elle comprend :
(1) une étape d'extraction de l'acide nucléique à détecter, appartenant au génome du virus, éventuellement présent dans l'échantillon biologique,
(2) la réalisation d'au moins une amplification génique à l'aide d'une paire d'amorces sélectionnée dans le groupe constitué par les amorces aptes à amplifier l'une des régions suivantes de l'ARN génomique d'HDV : R0, R1, R2, R3, R'1 et R'2, et
(3) l'analyse du produit amplifié par comparaison avec la molécule de séquence SEQ ID NO:1.

4. Méthode de détection selon la revendication 3, **caractérisée en ce que** l'étape (3) d'analyse peut être mise en oeuvre par restriction, séquençage ou hybridation.

5. Méthode selon la revendication 3 ou la revendication 4, **caractérisée en ce que** les amorces spécifiques pour l'amplification des régions R0, R1, R2, R3, R'1 et R'2, mises en oeuvre à l'étape (2) sont sélectionnées dans le groupe constitué par :
- les amorces 900S (SEQ ID NO:33) et 1280AS (SEQ ID NO:34), pour l'amplification de R0 (environ 400 pb),
- les amorces 320S (SEQ ID NO:39) et 1280AS (SEQ ID NO:34), pour l'amplification du fragment R1 (environ 960 pb),
- les amorces 900S (SEQ ID NO:33) et 320AS (SEQ ID NO:45), pour l'amplification de R2 (environ 1100 pb), qui contient le gène sHD correspondant aux positions 1598-950,
- les amorces 1480S (SEQ ID NO:46) et 440AS (SEQ ID NO:47), pour l'amplification de R3 (environ 650 pb),
- les amorces 3185 (SEQ ID NO:35) et 1150AS (SEQ ID NO:36), pour l'amplification de R'1 (environ 850 pb),
- les amorces 960S (SEQ ID NO:37) et 345AS (SEQ ID NO:38), pour l'amplification de R'2 (environ 1050 pb),

6. Méthode de détection et de génotypage d'HDV à partir d'un échantillon biologique, laquelle méthode est **caractérisée en ce qu'**elle comprend :
(a) une étape d'extraction de l'acide nucléique appartenant au génome du virus HDV,
(b) une étape d'amplification de la région R0 délimitée par les positions 889 à 1289 du génome HDV,
(c) un premier traitement des molécules d'acides nucléiques amplifiées par les enzymes de restriction *Sma*I et *Xho*I, pour produire un premier ensemble de fragments de restriction,
(d) un deuxième traitement des molécules d'acides nucléiques par l'enzyme de restriction *Sac*II, pour produire un deuxième ensemble de fragments de restriction
(e) l'analyse combinée des deux ensembles de fragments de restriction produits par RFLP (*Restriction Fragment Length Polymorphism*), de manière à détecter la présence et/ou déterminer le type d'HDV présent dans ledit échantillon biologique.

7. Méthode selon la revendication 6, **caractérisée en ce que** l'étape (b) d'amplification des molécules d'acide nucléique dudit échantillon par RT-PCR est avantageusement mise en oeuvre avec les amorces 900S (SEQ ID NO: 33) et 1280AS (SEQ ID NO:34).

8. Méthode selon la revendication 6 ou la revendication 7, **caractérisée en ce qu'**elle comprend en outre :
(a) l'amplification des molécules d'acide nucléique dudit échantillon par RT-PCR avec les amorces 900S (SEQ ID NO:33) et 320AS (SEQ ID NO: 45) de manière à amplifier la région R2, et
(b) le séquençage direct de la région R2 amplifiée et la comparaison avec la molécule d'ARN de séquence SEQ ID NO: 1.

9. Vecteur recombinant, notamment un plasmide comprenant un insert constitué par une molécule d'acide nucléique selon la revendication 1 ou la revendication 2.

10. Cellule transformée par une molécule d'acide nucléique selon la revendication 1 ou la revendication 2.

11. Produits de traduction codés par la molécule d'ARN de séquence SEQ ID NO:1 ou par ses séquences complémentaires sens ou anti-sens.

12. Protéines, **caractérisées en ce qu'**elles sont codées par l'une des molécules d'acide nucléique selon la revendication 1 ou la revendication 2.

13. Protéine selon la revendication 12, **caractérisée en ce qu'**elle est sélectionnée dans le groupe constitué par :
- la proteine LHD de dFr45, qui présente la séquence SEQ ID NO: 3,
et
- la proteine sHD de dFr45 qui présente la séquence SEQ ID NO: 5.

14. Peptide, **caractérisé en ce qu'**il est constitué par un fragment d'une protéine selon la revendication 12 ou la revendication 13, sélectionné dans le groupe constitué par :
- le peptide A constitué par les 19 acides aminés de l'extrémité carboxy-terminale de la séquence SEQ ID NO: 3,
- le peptide B de séquence (code à une lettre) RLPLLECTPQ (SEQ ID NO:59) constitué par les 10 acides aminés de l'extréminé carboxy-terminale de la séquence SEQ ID NO: 3, et
- le peptide C constitué par les 9 acides aminés précédents la séquence SEQ ID NO:59 (SEQ ID NO:60).

15. Utilisation d'une molécule d'acide nucléique selon la revendication 1 ou la revendication 2 ou d'une protéine selon l'une quelconque des revendications 11 à 14 pour la préparation d'un kit de détection et de génotypage d'un HDV.

## Claims

1. Isolated nucleic acid molecules, **characterised in that** they are selected from the group consisting of:
- the complete genome of the HDV variant referred to as dFr45, which has the sequence SEQ ID NO:1, and
- the genome of an HDV variant which has a genetic divergence of ≤ 15% from sequence SEQ ID NO:1.

2. Nucleic acid molecules, **characterised in that** they include at least one of the fragments of the sequence of a variant HDV according to claim 1, selected from the group consisting of:
a) the R0 fragment which has the sequence SEQ ID NO:48,
b) the R1 fragment which extends from positions 307 to 1289 of the HDV genome
c) the R2 fragment which extends from positions 889 to 328 of the HDV genome,
d) the R3 fragment which extends from positions 1486 to 452 of the HDV genome,
e) the R'1 fragment which extends from positions 305 to 1161 of the HDV genome,
f) the R'2 fragment which extends from positions 984 to 331 of the HDV genome,
g) the cDNA encoding the sHD protein, of sequence SEQ ID NO:4,
h) the cDNA encoding the LHD protein, of sequence SEQ ID NO:2,
and
i) the primers of sequence SEQ ID NO:38 and SEQ ID NO:47.

3. Method of detection of a variant HDV as defined in claim 1 or claim 2, by hybridisation and/or amplification, carried out from a biological sample, which method is **characterised in that** it comprises:
(1) a step of extracting the nucleic acid to be detected, belonging to the genome of the virus possibly present in the biological sample,
(2) performing at least one gene amplification using a pair of primers selected from the group consisting of the primers capable of amplifying one of the following regions of the HDV genomic RNA: R0, R1, R2, R3, R'1 and R'2,
and
(3) analysing the amplified product by comparison with the molecule of sequence SEQ ID NO:1.

4. Method of detection according to claim 3, **characterised in that** the analysis step (3) can be carried out by restriction, sequencing or hybridisation.

5. Method according to claim 3 or claim 4, **characterised in that** the specific primers for amplification of the R0, R1, R2, R3, R'1 and R'2 regions used in step (2) are selected from the group consisting of:
- the primers 900S (SEQ ID NO:33) and 1280AS (SEQ ID NO:34), for the amplification of R0 (approximately 400 pb),
- the primers 320S (SEQ ID NO:39) and 1280AS (SEQ ID NO:34), for the amplification of the R1 fragment (approximately 960 pb),
- the primers 900S (SEQ ID NO:33) and 320AS (SEQ ID NO:45), for the amplification of R2 (approximately 1100 pb), which contains the sHD gene corresponding to positions 1598 to 950,
- the primers 1480S (SEQ ID NO:46) and 440AS (SEQ ID NO:47), for the amplification of R3 (approximately 650 pb),
- the primers 318S (SEQ ID NO:35) and 1150AS (SEQ ID NO:36), for the amplification of R'1 (approximately 850 pb),
- the primers 960S (SEQ ID NO:37) and 345AS (SEQ ID NO:38), for the amplification of R'2 (approximately 1050 pb).

6. Method of detection and of genotyping of HDV from a biological sample, which method is **characterised in that** it comprises:
(a) a step of extracting the nucleic acid belonging to the genome of the HDV virus,
(b) a step of amplifying the R0 region delimited by positions 889 to 1289 of the HDV genome,
(c) a first treatment of the amplified nucleic acid molecules with the *Sma*I and *Xho*I restriction enzymes to produce a first set of restriction fragments,
(d) a second treatment of the nucleic acid molecules with the *Sac*II restriction enzyme to produce a second set of restriction fragments,
(e) the combined analysis by RFLP (*restriction fragment length polymorphism*) of the two sets of restriction fragments produced, so as to detect the presence and/or to determine the type of HDV present in said biological sample.

7. Method according to claim 6, **characterised in that** step (b) comprising amplification of the nucleic acid molecules of said sample by RT-PCR is advantageously performed with the primers 900S (SEQ ID NO:33) and 1280AS (SEQ ID NO:34).

8. Method according to claim 6 or claim 7, **characterised in that** it also comprises:
(a) amplification of the nucleic acid molecules of said sample by RT-PCR with the primers 900S (SEQ ID NO:33) and 320AS (SEQ ID NO:45), so as to amplify the R2 region, and
(b) direct sequencing of the amplified R2 region and comparison with the RNA molecule of sequence SEQ ID NO:1.

9. Recombinant vector, in particular a plasmid comprising an insert consisting of a nucleic acid molecule according to claim 1 or claim 2.

10. Cell transformed by a nucleic acid molecule according to claim 1 or claim 2.

11. Translation products encoded by the RNA molecule of sequence SEQ ID NO:1 or by its complementary sense or antisense sequences.

12. Proteins **characterised in that** they are encoded by one of the nucleic acid molecules according to claim 1 or claim 2.

13. Protein according to claim 12, **characterised in that** it is selected from the group consisting of:
- the LHD protein of dFr45, which has the sequence SEQ ID NO:3,
and
- the sHD protein of dFr45 which has the sequence SEQ ID NO:5.

14. Peptide **characterised in that** it consists of a fragment of a protein according to claim 12 or claim 13, selected from the group consisting of:
- peptide A consisting of the 19 amino acids of the carboxy-terminal end of the sequence SEQ ID NO:3,
- peptide B of sequence (one-letter code) RLPLLECTPQ (SEQ ID NO:59) consisting of the 10 amino acids of the carboxy-terminal end of the sequence SEQ ID NO:3, and
- peptide C consisting of the 9 amino acids preceding the sequence SEQ ID NO:59 (SEQ ID NO:60).

15. Use of a nucleic acid molecule according to claim 1 or claim 2 or of a protein according to one of claims 11 to 14 to prepare an HDV detection and genotyping kit.

## Patentansprüche

1. Isolierte Nukleinsäuremoleküle, **dadurch gekennzeichnet, dass** sie ausgewählt sind, aus der Gruppe, bestehend aus:
- dem gesamten Genom einer HDV-Variante, dFr45 genannt, die die Sequenz SEQ ID Nr. 1 aufweist, und
- dem Genom einer HDV-Variante, die eine genetische Divergenz ≤ 15 % zu der Sequenz SEQ ID Nr. 1 aufweist.

2. Nukleinsäuremoleküle, **dadurch gekennzeichnet, dass** sie wenigstens eines der Fragmente der Sequenz der HDV-Variante nach Anspruch 1 umfassen, ausgewählt aus der Gruppe, bestehend aus:
a) dem Fragment R0, das die SEQ ID Nr. 48 aufweist,
b) dem Fragment R1, das sich über die Positionen 307 bis 1289 des HDV-Genoms erstreckt,
c) dem Fragment R2, das sich über die Positionen 889 bis 328 des HDV-Genoms erstreckt,
d) dem Fragment R3, das sich über die Positionen 1486 bis 452 des HDV-Genoms erstreckt,
e) dem Fragment R'1, das sich über die Positionen 305 bis 1161 des HDV-Genoms erstreckt,
f) dem Fragment R'2, das sich über die Positionen 984 bis 331 des HDV-Genoms erstreckt,
g) der cDNA der Sequenz SEQ ID Nr. 4, die für das sHD-Protein codiert,
h) der cDNA der Sequenz SEQ ID Nr. 2, die für das LDH-Protein codiert,
i) den Primern der Sequenz SEQ ID Nr. 38 und SEQ ID Nr. 47.

3. Verfahren zum Detektieren einer HDV-Variante, wie sie nach Anspruch 1 oder Anspruch 2 definiert ist, durch Hybridisierung und/oder Amplifizierung, das ausgehend von einer biologischen Probe umgesetzt wird, wobei das Verfahren **dadurch gekennzeichnet ist, dass** es folgendes umfasst:
(1) einen Schritt der Extraktion der zu detektierenden Nukleinsäure, die zu einem Virus-Genom gehört, das möglicherweise in der biologischen Probe vorliegt,
(2) Umsetzung wenigstens einer genetischen Amplifizierung mit Hilfe eines Primerpaares, ausgewählt aus der Gruppe, bestehend aus den Primern, die geeignet sind eine der folgenden Regionen der genomischen RNA des HDV zu amplifizieren: R0, R1, R2, R3, R'1 und R'2, und
(3) Analyse des amplifizierten Produkts durch Vergleich mit dem Molekül der Sequenz SEQ ID Nr. 1.

4. Verfahren zum Detektieren nach Anspruch 3, **dadurch gekennzeichnet, dass** der Schritt (3) der Analyse durch Restriktion, Sequenzierung oder Hybridisierung durchgefiihrt werden kann.

5. Verfahren nach Anspruch 3 oder Anspruch 4, **dadurch gekennzeichnet, dass** die spezifischen Primern für die Amplifizierung der Regionen R0, R1, R2, R3, R'1 und R'2, die in Schritt (2) durchgeführt wird, ausgewählt sind, aus der Gruppe, bestehend aus:
- den Primern 900S (SEQ ID NR. 33) und 1280AS (SEQ ID Nr. 34), zum Amplifizieren von R0 (ungefähr 400 bp),
- den Primern 320S (SEQ ID NR. 39) und 1280AS (SEQ ID Nr. 34), zum Amplifizieren des Fragments R1 (ungefähr 960 bp),
- den Primern 900S (SEQ ID NR. 33) und 320AS (SEQ ID Nr. 45), zum Amplifizieren von R2 (ungefähr 1100 bp), das das sHD-Gen enthält, das den Positionen 1598 bis 950 entspricht,
- den Primern 1480S (SEQ ID NR. 46) und 440AS (SEQ ID Nr. 47), zum Amplifizieren von R3 (ungefähr 650 bp),
- den Primern 318S (SEQ ID NR. 35) und 1150AS (SEQ ID Nr. 36), zum Amplifizieren von R'1 (ungefähr 850 bp),
- den Primern 960S (SEQ ID NR. 37) und 345AS (SEQ ID Nr. 38), zum Amplifizieren von R'2 (ungefähr 1050 bp).

6. Verfahren zum Detektieren und Genotypisieren von HDV ausgehend von einer biologischen Probe, wobei das Verfahren **dadurch gekennzeichnet ist, dass** es folgendes umfasst:
(a) einen Schritt der Extraktion der Nukleinsäure, die zu dem Genom des HDV-Virus gehört,
(b) einen Schritt der Amplifizierung der Region R0, die von den Positionen 889 bis 1289 des HDV-Genoms begrenzt wird,
(c) eine erste Behandlung der Nukleinsäuremoleküle, die mit den Restriktionsenzymen *Sma*I und *Xho*I amplifiziert wurden, um eine erste Gesamtheit von Restriktionsfragmenten herzustellen,
(d) eine zweite Behandlung der Nukleinsäuremoleküle mit dem Restriktionsenzym *Sac*II, um eine zweite Gesamtheit von Restriktionsfragmenten herzustellen,
(e) eine Kombinationsanalyse der beiden Gesamtheiten der Restriktionsfragmente, die durch RFLP (*Restriction Fragment Length Polymorphism*) hergestellt wurden, derart, dass die Anwesenheit und/oder der Typ des in der biologischen Probe vorliegenden HDV detektiert wird.

7. Verfahren nach Anspruch 6, **dadurch gekennzeichnet, dass** der Schritt (b) des Amplifizierens der Nukleinsäuremoleküle der Probe durch RT-PCR vorteilhafterweise mit den Primern 900S (SEQ ID NR. 33) und 1280AS (SEQ ID Nr. 34) durchgeführt wird.

8. Verfahren nach Anspruch 6 oder Anspruch 7, **dadurch gekennzeichnet, dass** es ferner folgendes umfasst:
(a) Amplifizieren der Nukleinsäuremoleküle der Probe durch RT-PCR mit den Primern 900S (SEQ ID NR. 33) und 320AS (SEQ ID Nr. 45), derart, dass die Region R2 amplifiziert wird, und
(b) direktes Sequenzieren der amplifizierten Region R2 und Vergleich mit dem RNA-Molekül der Sequenz SEQ ID Nr. 1.

9. Rekombinanter Vektor, insbesondere Plasmid, umfassend ein aus einem Nukleinsäure-Molekül nach Anspruch 1 oder Anspruch 2 gebildetes Insert.

10. Zelle, die mit einem Nukleinsäure-Molekül nach Anspruch 1 oder Anspruch 2 transformiert ist.

11. Translationsprodukte, die von dem RNA-Molekül der Sequenz SEQ ID Nr. 1 codiert werden oder von dessen komplementären Sens- oder Antisens-Sequenzen.

12. Proteine, **dadurch gekennzeichnet, dass** sie von einem der Nukleinsäuremoleküle nach Anspruch 1 oder Anspruch 2 codiert werden.

13. Protein nach Anspruch 12, **dadurch gekennzeichnet, dass** es ausgewählt ist aus der Gruppe, bestehend aus:
- dem LDH-Protein von dFr45, das die Sequenz SEQ ID Nr. 3 aufweist,
- dem sHD-Protein von dFr45, das die Sequenz SEQ ID Nr. 5 aufweist.

14. Peptid, **dadurch gekennzeichnet, dass** es von einem Fragment des Proteins nach Anspruch 12 oder Anspruch 13 gebildet wird, ausgewählt aus der Gruppe, bestehend aus:
- dem Peptid A, das aus 19 Aminosäuren des Carboxy-terminalen Endes der Sequenz SEQ ID Nr. 3 gebildet wird,
- dem Peptid B der Sequenz (Einbuchstabencode) RLPLLECTPQ (SEQ ID Nr. 59), das aus 10 Aminosäuren des Carboxy-terminalen Endes der Sequenz SEQ ID Nr. 3 gebildet wird, und
- dem Peptid C, das aus 9 Aminosäuren gebildet wird, die der Sequenz SEQ ID Nr. 59 vorangehen (SEQ ID Nr. 60).

15. Verwendung eines Nukleinsäure-Moleküls nach Anspruch 1 oder Anspruch 2 oder eines Proteins nach einem der Ansprüche 11 bis 14 zum Herstellen eines Detektions-Kits und eines Kits zur Genotypisierung eines HDV.
